(19) Europäisches Patentamt
European Patent Office
Office européen des brevets

(11) EP 4 019 607 B1

(12) EUROPEAN PATENT SPECIFICATION

(45) Date of publication and mention of the grant of the patent:
**15.05.2024 Bulletin 2024/20**

(21) Application number: **20306663.4**

(22) Date of filing: **22.12.2020**

(51) International Patent Classification (IPC):
*C09K 9/02* (2006.01) *C07D 401/04* (2006.01)
*C07D 401/14* (2006.01) *C07D 413/04* (2006.01)
*C07D 417/04* (2006.01) *C07D 421/04* (2006.01)
*C07D 471/04* (2006.01)

(52) Cooperative Patent Classification (CPC):
**C09K 9/02; C07D 401/04; C07D 401/14; C07D 413/04; C07D 417/04; C07D 421/04; C07D 471/04**

(54) **ELECTROCHROMIC COMPOUNDS AND OPTICAL ARTICLES CONTAINING THEM**

ELEKTROCHROME VERBINDUNGEN UND OPTISCHE ARTIKEL DAMIT

COMPOSÉS ÉLECTROCHROMIQUES ET ARTICLES OPTIQUES LES CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.06.2022 Bulletin 2022/26**

(73) Proprietor: **ESSILOR INTERNATIONAL**
**94220 Charenton-Le-Pont (FR)**

(72) Inventors:
- **BIVER, Claudine**
  **94220 CHARENTON LE PONT (FR)**
- **BERIT-DEBAT, Fabien**
  **94220 CHARENTON-LE-PONT (FR)**
- **ARCHAMBEAU, Samuel**
  **94220 CHARENTON-LE-PONT (FR)**
- **AIKEN, Stuart**
  **LEEDS, WEST YORKSHIRE LS2 9JT (GB)**
- **ARMITAGE, Georgina K.**
  **LEEDS, WEST YORKSHIRE LS2 9JT (GB)**
- **BROADBENT, Thomas D.**
  **LEEDS, WEST YORKSHIRE LS2 9JT (GB)**
- **CROSSLEY, Daniel L.**
  **LEEDS, WEST YORKSHIRE LS2 9JT (GB)**
- **GABBUTT, Christopher D.**
  **LEEDS, WEST YORKSHIRE LS2 9JT (GB)**
- **HERON, Bernard Mark**
  **LEEDS, WEST YORKSHIRE LS2 9JT (GB)**

(74) Representative: **Plasseraud IP**
**66, rue de la Chaussée d'Antin**
**75440 Paris Cedex 09 (FR)**

(56) References cited:
**GB-A- 1 317 442 JP-A- 2013 245 176**
**US-A- 3 678 062**

- **SALEHI NAEIMEH ET AL: "Synthesis and biological evaluation of newN-benzylpyridinium-based benzoheterocycles as potential anti-Alzheimer's agents", BIOORGANIC CHEMISTRY, vol. 83, 13 November 2018 (2018-11-13), pages 559-568, XP085602017, ISSN: 0045-2068, DOI: 10.1016/J.BIOORG.2018.11.010**
- **YU H ET AL: "Preparation and photophysical properties of benzimidazole-based gels", JOURNAL OF PHOTOCHEMISTRY AND PHOTOBIOLOGY, A: CHEMISTRY, ELSEVIER SEQUOIA, LAUSANNE, CH, vol. 178, no. 1, 20 February 2006 (2006-02-20), pages 62-69, XP028008576, ISSN: 1010-6030, DOI: 10.1016/J.JPHOTOCHEM.2005.06.022 [retrieved on 2006-02-20]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a group of novel electrochromic compounds disclosed in the claims. More specifically, it relates to benzazoles and condensed azole compounds defined in the claims, and the use of these compounds as a variable transmittance medium for the manufacture of an optical article, such as an ophthalmic lens as defined in the claims.

Electrochromism is a well-known physical phenomenon which is observed with certain classes of chemical compounds that reversibly change colour when a voltage is applied to them. The material undergoes reversible changes in optical properties by oxidation and reduction. Advantageously, the electrochromic material is colourless when an electric field is not applied and becomes coloured when an electric field is applied.

An electrochromic device, i.e. a device containing electrochromic compounds, the absorbance of which depends only on the presence of an electric field, can thus have two states, i.e. a coloured state (when electrically activated) and a bleached state (in the inactive state). The optical transmission properties of the device depend on the nature of the electrochromic compounds.

There remains a need for improving electrochromic materials in order to use them as transparent media for forming high quality articles, in particular high quality ophthalmic lenses, while keeping electrochromic properties and having a wide range of colours.

Surprisingly, the inventors have found that introducing an azole ring or an azolium ring in the structure of the electrochromic molecules lowers their absorption wavelength in the visible range. Indeed, different azole rings have been introduced and were found to efficiently widen the wavelength coverage at various activation potentials. This was the case for instance with substituted imidazoliums and fused-ring derivatives, substituted benzimidazoliums, substituted benz(iso)thiazolium compounds. In addition, viologens molecules or benzazoles or condensed azole containing molecules of the invention present one or two reduction potentials.

For instance, viologens molecules usually present two reduction potentials from a bication ($bipm^{2+}$) to the monocation ($bipm^{+}$) and from the monocation to the neutral species ($bipm^{0}$).

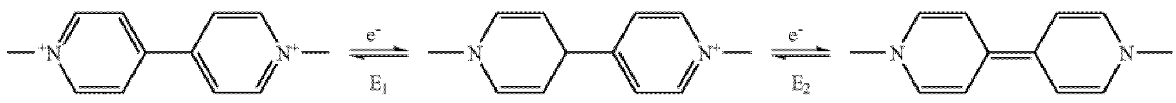

bipm2+ bipm+ bipm0

The reaction from $bipm^{2+}$ to $bipm^{+}$ occurs at a first potential $E_1$ and is a reversible reaction, while the reaction from $bipm^{+}$ to $bipm^{0}$ occurs at a lower potential $E_2$ and is often irreversible. $Bipm^{0}$ is consider as an instable compound which can react with the oxygen present in the device or with another molecule leading to a different chemical structure and else losing its electrochromic properties.

Here the inventors have tried by modifying the chemical structures to increase the distance between the two reduction peaks in order to improve the stability of the device and hence its lifetime. Chemical groups added are such that the distance in potential between these two reduction peaks is below 0.1V, preferably below 0.3V, even preferably below 0.5V.

JP2013245176A discloses an electrochromic display comprising a central non-charged benzimidazole group in which the benzene ring is substituted with two pyridinium moieties. US3678062A discloses compounds featuring a benzisoxazolyl or a benzofuranyl core substituted with a N-alkyl pyridinium group, for use in lowering the blood sugar levels of warm blooded animals. GB1317442A discloses compounds that are used as diquaternary ammonium salts to be used for developing a photographic image recorded by exposing to actinic-radiation a photosensitive-inorganic lead compound, among which said document discloses a compound featuring a benzothiazolyl group substituted with a N-methyl pyridinium group. Salehi Naeimeh et al. (Bioorganic Chemistry, 83(2019), 559-568) disclose salts comprising a benzoimidazolyl or a benzothiazolyl core substituted with a N-benzylpyridinium moiety, as potential anti-Alzheimer's agents. Yu et al. (Journal of Photochemistry and Photobiology A: Chemistry 178 (2006) 62-69) disclose alkylpyrididinylium benzimidazole derivatives which are described as fluorescent gelators.

After conducting extensive research, the present inventors provide novel electrochromic compounds that exhibit excellent electrochromic properties and that can be easily incorporated in a cell to form, for instance, an electrochromic lens.

As such, the compounds of the present invention are advantageously:

- reversibly oxidized or reduced;
- easily activated, i.e. they have an electrochemical potential from -1.5 to -0.5 V. - stable, i.e. no generation of degradation products.

More particularly the compounds of the present invention exhibit either one low reversible reduction peak or two reversible reduction peaks separated by at least 0.1V, preferably at least 0.3 V, more preferably at least 0.4 V, even more preferably at least 0.5 V the first reversible reduction peak being low).

The present invention is set out in the appended set of claims. In particular, the present invention relates to electrochromic compounds of formula (I) as defined in the appended claims 1 to 6.

The present invention also relates to an electrochromic composition, as defined in appended claims 7 or 8, comprising at least one compound of formula (I) as defined in appended claims 1 to 6.

Finally, the present invention relates to an electrochromic device as defined in appended claims 9 or 10, such as an ophthalmic lens, comprising an electrochromic compound of formula (I) as defined in appended claims 1 to 6, or an electrochromic composition as defined in appended claims 9 or 10.

## Definitions

The term "aromatic compound" means unsaturated chemical compounds characterized by one or more planar rings of atoms joined by covalent bonds.

The term "cyclic compound or ring compound" corresponds to a compound in which one or more series of atoms in the compound is connected to form a ring. Rings may vary in size from three to many atoms, for example 5 or 6 atoms and include examples where all the atoms are carbon (i.e., are carbocycles), or where both carbon and non-carbon atoms are present (heterocyclic compounds). More precisely, a "heterocyclic compound or ring structure" is a cyclic compound that has atoms of at least two different elements as members of its ring(s)

The term "azole" represents any five-membered heterocyclic radical containing a nitrogen atom and at least one other non-carbon atom as part of the ring. Examples of other non-carbon atoms include nitrogen, oxygen, sulfur and selenium. The five-membered heterocyclic ring (Cycle C5) in the present invention is typically an azole. Examples of azole groups include imidazole, pyrazole, oxazole, isoxazole, thiazole, isothiazole and selenazole.

The term "a conjugated system" refers to a system of connected p orbitals with delocalized electrons in a molecule, which in general lowers the overall energy of the molecule and increases stability. It is conventionally represented as having alternating single and multiple bonds. Lone pairs, radicals or carbenium ions may be part of the system, which may be cyclic, acyclic, linear or mixed. The conjugated system according to the present invention formed by the five-membered heterocyclic ring (Cycle C5) and the six-membered (hetero)cyclic ring (Cycle C6) is for example a benzazole (or benzoxazole), a benzisoxazole, a benzothiazole, a benzimidazole, an indazole, an imidazo[1,2-a]pyridine.

The term "pyridinium" refers to the cation of pyridine in which the nitrogen atom is positively charged and is represented by the following formula:

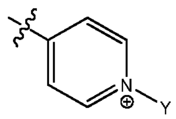

wherein Y include $C_1$-$C_{18}$ alkyl, (hetero)aryl or (hetero)arylalkyl.

The expression "alkyl" or "$C_1$-$C_{18}$ alkyl" represents any monovalent radical of a linear or branched hydrocarbon chain comprising 1 to 18 carbon atoms. The expression "$C_3$-$C_6$ alkyl" represents an alkyl group having 3 to 6 carbon atoms. Examples of $C_1$-$C_{18}$ alkyl groups include $C_1$-$C_4$ alkyl groups such as methyl, ethyl, *n*-propyl, *i*-propyl, *n*-butyl, *i*-butyl, *s*-butyl or *t*-butyl, $C_6$-$C_8$ alkyl groups such as *n*-hexyl, *n*-heptyl or *n*-octyl, as well as *n*-pentyl, 2-ethylhexyl, 3,5,5-trimethylhexyl, *n*-nonyl, *n*-decyl, *n*-undecyl, *n*-dodecyl or *n*-octadecyl.

The expression "aryl" represents any monovalent radical of an aromatic hydrocarbon comprising 6 to 18 carbon atoms, either monocyclic or polycyclic. The expression "Polycyclic aryls or polycyclic aromatic compounds" refers to either compounds in which at least one carbon-carbon bond is common to two aromatic rings (fused aromatic rings) or polycyclic aromatic hydrocarbons which contain two or more benzenoid rings joined by a carbon-carbon single bond. Examples of $C_6$-$C_{18}$ aryl groups include phenyl, naphthyl, anthracenyl and phenanthrenyl.

The expression "arylalkyl" represents any aryl derivative of an alkyl group. The expression "arylalkyl" represents an aryl group as defined above combined to an alkyl group as defined above. Examples of arylalkyl groups include benzyl, phenylethyl, phenylpropyl, phenylbutyl, phenylpentyl, phenylhexyl, naphthylmethyl, naphthylethyl, naphthylpropyl, naphthylbutyl, naphthylpentyl, naphthylhexyl, anthracenylmethyl, anthracenylethyl, anthracenylpropyl, anthracenylbutyl, anthracenylpentyl, anthracenylhexyl, phenanthrenylmethyl, phenanthrenylethyl, phenanthrenylpropyl, phenanthrenylbutyl, phenanthrenylpentyl and phenanthrenylhexyl.

The expression "heteroaryl" represents any monovalent radical of a monocyclic or polycyclic 5 to 10 membered aromatic group comprising from 1 to 3 heteroatoms independently selected from oxygen, nitrogen and sulfur. Examples of $C_5$-$C_{10}$ heteroaryl groups include furyl, thienyl, pyrrolyl, pyrazoyl, imidazolyl, isoxazolyl, isothiazoyl, thiazolyl, oxazolyl,

1,2,3-triazolyl, 1,2,4-triazolyl, 1-benzofuryl, 1-benzothienyl, indolyl, benzimidazolyl, indazolyl, 1,2-benzisoxazolyl, 2,1-benzisoxazolyl, 1,2-benzisothiazolyl, 2,1-benzisothiazolyl, benzothiazolyl, benzoxazolyl, benzotriazolyl, pyridyl, pyridinium, quinolinyl, quinolinium, isoquinolinyl, isoquinolinium, pyridazinyl, cinnolinyl, phthalazinyl, pyrimidinyl, quinazolinyl, pyrazinyl and quinoxalinyl.

Unless mentioned otherwise, the groups and radicals defined hereinabove may be unsubstituted or substituted by one or more substituents such as, for example, halogen, alkyl, alkoxy, aryl, heteroaryl, haloalkyl, haloalkoxy, alkoxycarbonyl, alkanoyl, aroyl, formyl, nitrile, nitro, amido, alkylthio, alkylsulfinyl, alkylsulfonyl, arylthio, arylsulfinyl, arylsulfonyl, amino, alkylamino, arylamino, dialkylamino and diarylamino.

These substituents are not a phosphonate group (P=O(OR)(OR') with R and R' either alkyl or aryl), a phosphate group, a carboxyl group (C=O(OH)), a trihalosilyl group such as trichlorosilyl group, a trialkoxysilyl group (such as a triethoxysilyl group or a trimethoxysilyl group), a monohalosilyl group (such as monochlorosilyl group) or a monoalkoxysilyl group.

## Electrochromic compounds

The electrochromic compounds of the present invention defined in the appended claims have a central core comprising a five-membered heterocyclic ring, typically an azole ring, fused to a six-membered (hetero)cyclic ring, onto which are branched one or several lateral pyridinium groups, in particular one, two or three lateral pyridinium groups.

As such, the electrochromic compounds of the present invention are represented by formula (I):

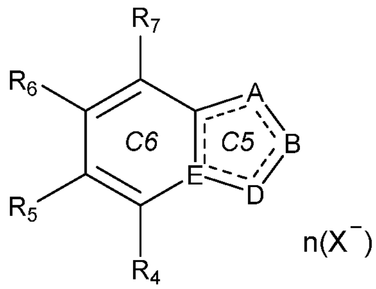

(I)

wherein:

**A** is N, $^+N$, N-$R_1$, $^+N$-$R_1$ or C-$R_1$;
**B** is C-$R_2$, S, Se, O, N, N-$R_2$ or $^+N$-$R_2$;
**D** is C-$R_3$, N, S, O, Se, N-$R_3$ or $^+N$-$R_3$;
**E** is C, N or $^+N$;
**$R_1$** is H, $C_1$-$C_{18}$ alkyl, aryl or **Z;**
**$R_2$** is H, $C_1$-$C_{18}$ alkyl, aryl, **Z** or aryl substituted by **Z;**
**$R_3$** is H or $C_1$-$C_{18}$ alkyl, aryl or **Z;**
**$R_4$** is H, $C_1$-$C_{18}$ alkyl, aryl or **Z;**
**$R_5$** is H, $C_1$-$C_{18}$ alkyl, aryl or **Z;**
**$R_6$** is H, $C_1$-$C_{18}$ alkyl, aryl or **Z;**
**$R_7$** is H, $C_1$-$C_{18}$ alkyl, aryl or **Z;**
**$R_7$** and **$R_6$** and/or **$R_6$** and **$R_5$** and/or **$R_5$** and **$R_4$** may form together an aromatic ring or heteroaromatic ring fused to the six- membered (hetero)cyclic core (Cycle *C6)* they are attached to, optionally substituted by **Z,**
wherein **Z** is

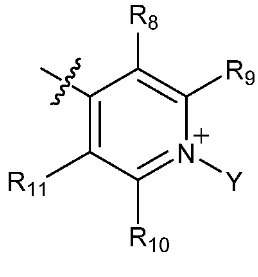

;

with **Y** being Ci-C alkyl or aryl;
$\mathbf{R_8}$, $\mathbf{R_9}$, $\mathbf{R_{10}}$ and $\mathbf{R_{11}}$ are independently selected from H and $C_1$-$C_{18}$ alkyl;
$\mathbf{R_8}$ and $\mathbf{R_9}$ or $\mathbf{R_{10}}$ and $\mathbf{R_{11}}$ may form an aromatic ring fused to the pyridium group they are attached to,
when **B** = C-Z, and when **A**=$^+$N, $\mathbf{R_8}$ or $\mathbf{R_{11}}$ may form with **A** a ring, aromatic or not, fused with the five-membered heterocyclic ring (Cycle *C5)* **A** is attached to,
**n** is selected to counterbalance the number of positive charges;
**X** is a counterion;
\- - - - - -is a single bond or a double bond;

**with the 3 following provisos:**

1) cycle *C5* is a five membered heterocyclic ring with 2 of **A, B, D** and **E** being independently selected from: N, N-$R_1$, $^+$N-$R_1$, N-$R_2$,$^+$N-$R_2$, N-$R_3$, $^+$N-$R_3$, S, Se and O;
2) cycle *C5* and Cycle *C6* form a conjugated system; and
3) at least one of $\mathbf{R_1}$, $\mathbf{R_2}$, $\mathbf{R_3}$, $\mathbf{R_4}$, $\mathbf{R_5}$, $\mathbf{R_6}$ or $\mathbf{R_7}$ is **Z** or at least $\mathbf{R_7}$ and $\mathbf{R_6}$ form together an aromatic ring substituted by **Z** or at least $\mathbf{R_5}$ and $\mathbf{R_6}$ form together an aromatic ring substituted by **Z** or at least $\mathbf{R_5}$ and $\mathbf{R_4}$ form together an aromatic ring substituted by **Z;**

and wherein said compound of formula (I) is selected from those wherein:

- **A** is $^+$N-$R_1$; **B** is C-$R_2$; **D** is S or N-$R_3$; **E** is C; or
- **A** is $^+$N-$R_1$; **B** is C-$R_2$; **D** is C-$R_3$; **E** is N; or
- **A** is N; **B** is C-$R_2$; **D** is Se or O; **E** is C; or
- **A** is C-$R_1$; **B** is N or $^+$N-$R_2$; **D** is S; **E** is C; or
- **A** is C-$R_1$; **B** is N, **D** is O; **E** is C; or
- **A** is C-$R_1$; **D** is N, $^+$N-$R_3$, S, Se or O; **E** is C; or
- **B** is N or $^+$N-$R_2$; **A** is C-$R_1$; **D** is C-$R_3$; **E** is N; or
- **D** is $^+$N-$R_3$ or N; **A** is C-$R_1$; **B** is C-$R_2$; **E** is N; or
- **E** is $^+$N; **A** is O, S, or Se; **B** is C-$R_2$; **D** is C-$R_3$;

and wherein said electrochromic compound of formula (I) is not one of the following compounds:

a compound of formula $D_1$:

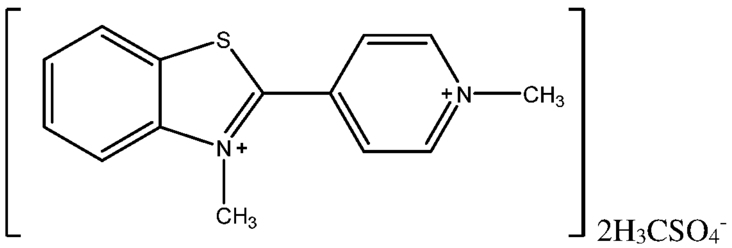

($D_1$)

or a compound of formula D2:

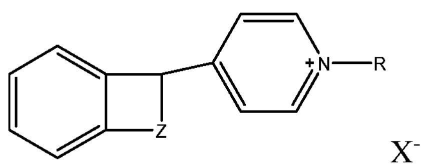

($D_2$)

wherein R is lower alkyl having one to four carbon atoms selected from the group consisting of methyl, ethyl, propyl, isopropyl, butyl and isobutyl; Z is a trivalent radical selected from the group consisting of:

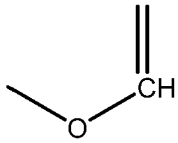, and 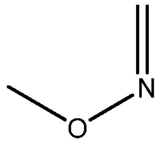;

and X is a monovalent pharmaceutically acceptable anion selected from the group consisting of chloride, bromide, iodide and fluoride;

or one compound selected from the group consisting of:

1-methyl-4-(1,2-benzisoxazol-3-yl)pyridinium iodide,
1-ethyl-4-(1,2-benzisoxazol-3-yl)pyridinium chloride,
1-methyl-4-(3-benzofuranyl)-pyridinium iodide,
1-methyl-4-(3-benzofuranyl)-pyridinium chloride, and
1-butyl-4-(3-benzofuranyl)pyridinium bromide

In all the present invention, **Y** is $C_1$-$C_6$ alkyl or aryl; for example, **Y** is methyl, *n*-hexyl or phenyl.

In all the present invention, the counterion $\mathbf{X}^-$ may be selected from halide, preferably fluoride and chloride, tetrafluoroborate, tetraphenylborate, hexafluorophosphate, nitrate, methanesulfonate, trifluoromethanesulfonate, *p*-toluenesulfonate, hexachloroantimonate, bis(trifluoromethanesulfonyl)imide, perchlorate, acetate and sulfate, preferably, $\mathbf{X}^-$ is tetrafluoroborate or hexafluorophosphate.

The electrochromic compounds of the present invention are typically represented by formulae (II), (III), (IV), (V), (VI), (VII), (VIII), (IX) or (X):

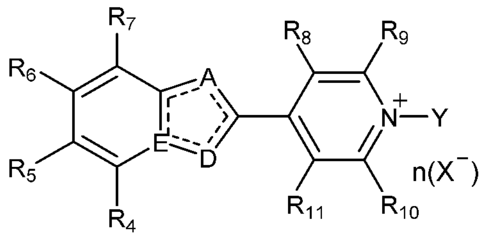

(II)

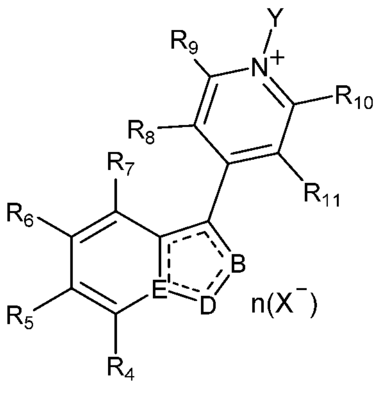

(III)

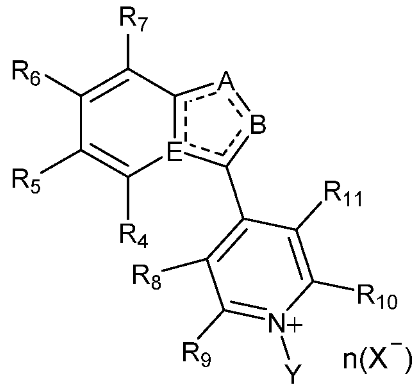

(IV)

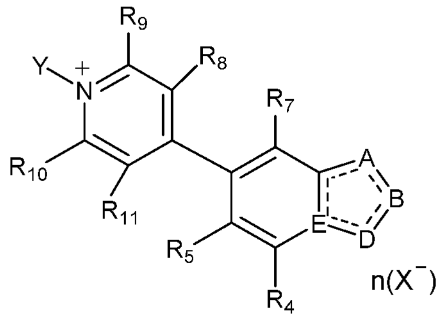

(V)

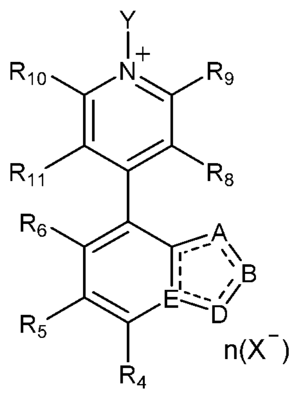

(VI)

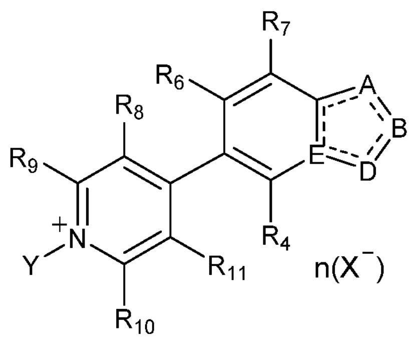

(VII)

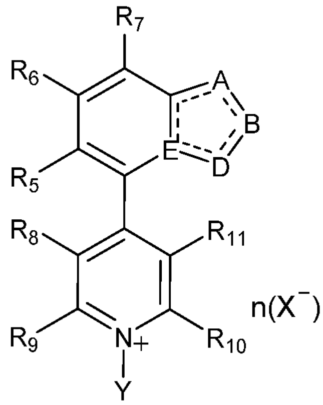

(VIII)

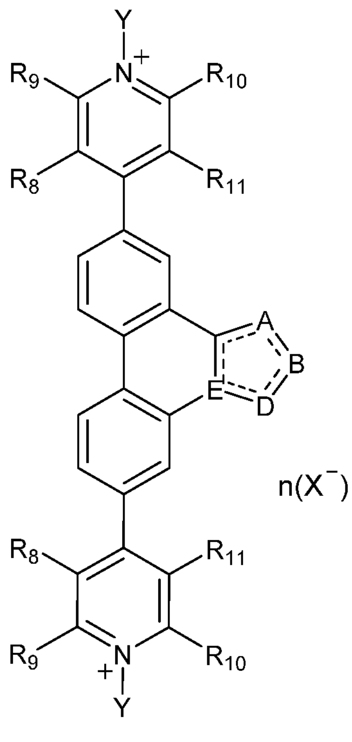

(IX)

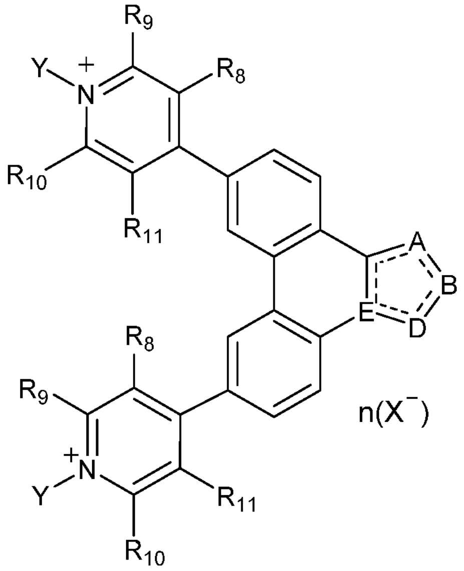

(X)

wherein **A, B, D, E, $R_1$**, **$R_2$**, **$R_3$**, **$R_4$**, **$R_5$**, **$R_6$**, **$R_7$**, **$R_8$**, **$R_9$**, **$R_{10}$** and **$R_{11}$**, when present, and **Z, Y, n** and **X** are as described above.

In a group of compounds disclosed herein, among which some compounds are not part of the present invention, the five-membered heterocyclic ring *C5* of the central core of the electrochromic compounds is an azole ring and the compounds of formulae (I), (II), (III), (IV), (V), (VI), (VII), (VIII), (IX) or (X) are those wherein:

- **A** is N, $^+$N, N-$R_1$ or $^+$N-$R_1$,
- and/ or **B** is N, N-$R_2$ or $^+$N-$R_2$,
- and/ or **D** is N, N-$R_3$ or $^+$N-$R_3$.

Another group of compounds of formulae (I), (II), (III), (IV), (V), (VI), (VII), (VIII), (IX) or (X), among which some compounds which are not part of the invention can also be those wherein **E** is N or $^+$N and/ or wherein **D** is S.

The compounds of formula (I) of the invention, with the provisos defined in the appended claims are, for example:

- those wherein A is $^+$N-$R_1$; **B** is C-$R_2$; **D** is S or N-$R_3$; **E** is C and **$R_1$**, **$R_2$**, **$R_3$**, **$R_4$**, **$R_5$**, **$R_7$**, **$R_8$**, **$R_9$**, **$R_{10}$**, **$R_{11}$**, **Z, Y, n** and **X** are as defined above;
- those wherein **A** is $^+$N-$R_1$; **B** is C-$R_2$; **D** is C-$R_3$; **E** is N and **$R_1$**, **$R_2$**, **$R_3$**, **$R_4$**, **$R_5$**, **$R_7$**, **$R_5$**, **$R_9$**, **$R_{10}$**, **$R_{11}$**, **Z, Y, n** and **X** are as defined above;
- those wherein **A** is N; **B** is C-$R_2$; **D** is Se or O; **E** is C and **$R_1$**, **$R_2$**, **$R_3$**, **$R_4$**, **$R_5$**, **$R_7$**, **$R_8$**, **$R_9$**, **$R_{10}$**, **$R_{11}$**, **Z, Y, n** and **X** are as defined above;
- those wherein A is C-$R_1$; **B** is N or $^+$N-$R_2$; **D** is S; **E** is C and **$R_1$**, **$R_2$**, **$R_3$**, **$R_4$**, **$R_5$**, **$R_7$**, **$R_8$**, **$R_9$**, **$R_{10}$**, **$R_{11}$**, **Z, Y, n** and **X** are as defined above;
- those wherein **A** is C-$R_1$; **B** is N, **D** is O; **E** is C and **$R_1$**, **$R_2$**, **$R_3$**, **$R_4$**, **$R_5$**, **$R_7$**, **$R_8$**, **$R_9$**, **$R_{10}$**, **$R_{11}$**, **Z, Y, n** and **X** are as defined above;
- those wherein **A** is C-$R_1$; **D** is N, $^+$N-$R_3$, S, Se or O; **E** is C; and **$R_1$**, **$R_2$**, **$R_3$**, **$R_4$**, **$R_5$**, **$R_7$**, **$R_8$**, **$R_9$**, **$R_{10}$**, **$R_{11}$**, **Z, Y, n** and **X** are as defined above;
- those wherein **B** is N or $^+$N-$R_2$; **A** is C-$R_1$; **D** is C-$R_3$; **E** is N; and **$R_1$**, **$R_2$**, **$R_3$**, **$R_4$**, **$R_5$**, **$R_7$**, **$R_8$**, **$R_9$**, **$R_{10}$**, **$R_{11}$**, **Z, Y, n** and **X** are as defined above;
- those wherein **D** is $^+$N-$R_3$ or N; **A** is C-$R_1$; **B** is C-$R_2$; **E** is N; and **$R_1$**, **$R_2$**, **$R_3$**, **$R_4$**, **$R_5$**, **$R_7$**, **$R_8$**, **$R_9$**, **$R_{10}$**, **$R_{11}$**, **Z, Y, n** and **X** are as defined above; or
- those wherein **E** is $^+$N; **A** is O, S, or Se; **B** is C-$R_2$; **D** is C-$R_3$. And **$R_1$**, **$R_2$**, **$R_3$**, **$R_4$**, **$R_5$**, **$R_7$**, **$R_8$**, **$R_9$**, **$R_{10}$**, **$R_{11}$**, **Z, Y, n** and **X** are as defined above.

Another group of compounds, among which several are not included in the claims defining the present invention, are those of **formula** (**II**) wherein **A** is N, $^+$N, N-$R_1$ or $^+$N-$R_1$; **D** is N-$R_3$, $^+$N-$R_3$, S, Se, or O; **E** is C; and **$R_1$**, **$R_3$**, **$R_4$**, **$R_5$**, **$R_6$**, **$R_7$**, **$R_8$**, **$R_9$**, **$R_{10}$**, **$R_{11}$**, **Z, Y, n** and **X** are as defined above.

Yet another group of compounds of **formula** (**II**), among which some are not included in the claims defining the present invention, are those wherein **A** is $N^+$ or $^+N-R_1$, **D** is S; and **E** is C and $\mathbf{R_1}$, $\mathbf{R_3}$, $\mathbf{R_4}$, $\mathbf{R_5}$, $\mathbf{R_6}$, $\mathbf{R_7}$, $\mathbf{R_8}$, $\mathbf{R_9}$, $\mathbf{R_{10}}$, $\mathbf{R_{11}}$, **Z, Y, n** and **X** are as defined above.

According to a first particular embodiment of the invention, compounds can be those of **formula** (**II**) wherein: **A** is $^+N-R_1$; **D** is S; **E** is C; $\mathbf{R_1}$ is $C_1$-$C_{18}$ alkyl; preferably $\mathbf{R_1}$ is $C_1$-$C_6$ alkyl; more preferably $\mathbf{R_1}$ is methyl; $\mathbf{R_4}$ is preferably H, $C_1$-$C_{18}$ alkyl or aryl; more preferably $\mathbf{R_4}$ is H; $\mathbf{R_5}$ is preferably H, $C_1$-$C_{18}$ alkyl or aryl; more preferably $\mathbf{R_5}$ is H; $\mathbf{R_6}$ is preferably H, $C_1$-$C_{18}$ alkyl or aryl; more preferably $\mathbf{R_6}$ is H; $\mathbf{R_7}$ is preferably H, $C_1$-$C_{18}$ alkyl or aryl; more preferably $\mathbf{R_7}$ is H; $\mathbf{R_8}$ is preferably H, $C_1$-$C_{18}$ alkyl or aryl; more preferably $\mathbf{R_8}$ is H; $\mathbf{R_9}$ is preferably H, $C_1$-$C_{18}$ alkyl or aryl; more preferably $\mathbf{R_9}$ is H; $\mathbf{R_{10}}$ is preferably **H,** $C_1$-$C_{18}$ alkyl or aryl; more preferably $\mathbf{R_{10}}$ is H; $\mathbf{R_{11}}$ is preferably H, $C_1$-$C_{18}$ alkyl or aryl; more preferably $\mathbf{R_{11}}$ is H; **Y** is $C_1$-$C_6$ alkyl or aryl; even more preferably **Y** is *n*-hexyl or phenyl; **n** is 2; and $\mathbf{X}^-$ is tetrafluoroborate or hexafluorophosphate, preferably tetrafluoroborate.

Other compounds which are not part of the present invention, are those of **formula** (**II**) wherein **A** is $^+N$, **D** is S; **E** is C and; $\mathbf{R_8}$ forms with **A,** a five or six-membered ring, unsaturated or not, fused with Cycle *C5* A is attached to and is represented by formula (XI), (XII) or (XIII).

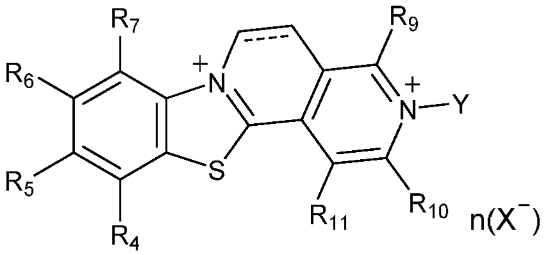

(XI)

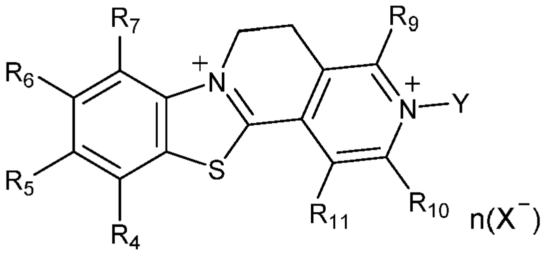

(XII)

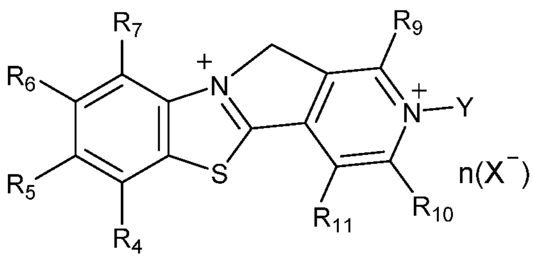

(XIII)

$\mathbf{R_4}$, $\mathbf{R_5}$, $\mathbf{R_6}$, $\mathbf{R_7}$, $\mathbf{R_9}$, $\mathbf{R_{10}}$, $\mathbf{R_{11}}$, **Z, Y, n** and **X** being as defined above.

Still according to this first embodiment of the present invention, preferred compounds can be those of **formula** (**II**) wherein **A** is $^+N-R_1$, **D** is S; **E** is C; and $\mathbf{R_1}$, $\mathbf{R_4}$, $\mathbf{R_5}$, $\mathbf{R_6}$, $\mathbf{R_7}$, $\mathbf{R_8}$, $\mathbf{R_9}$, $\mathbf{R_{10}}$, $\mathbf{R_{11}}$, **Z, Y, n** and **X** are as defined above. More specifically, these compounds can be the ones of **Formula** (**II**) wherein $\mathbf{R_7}$ and $\mathbf{R_6}$ and/or $\mathbf{R_6}$ and $\mathbf{R_5}$ and/or $\mathbf{R_5}$

and $\mathbf{R_4}$ form together an aromatic ring or heteroaromatic ring fused to the (hetero)cyclic core (Cycle *C6)* they are attached to, optionally substituted by **Z**, $\mathbf{R_1}$, $\mathbf{R_8}$, $\mathbf{R_9}$, $\mathbf{R_{10}}$, $\mathbf{R_{11}}$, **Z**, **Y**, **n** and **X** being as defined above. Specific examples are represented below:

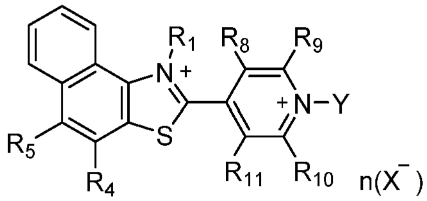

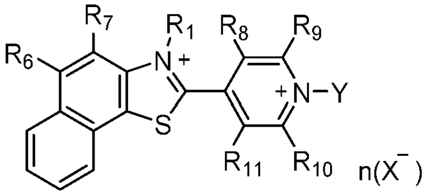

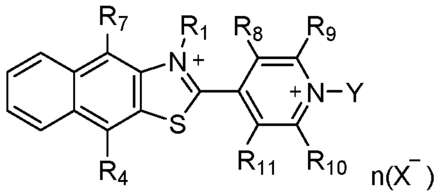

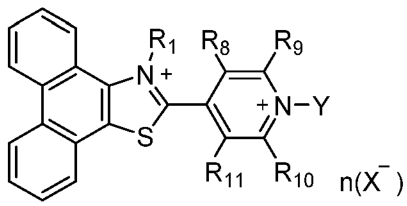

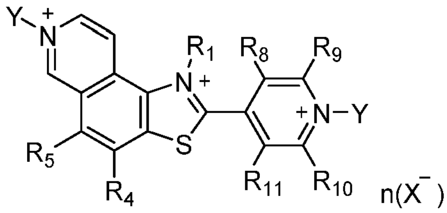

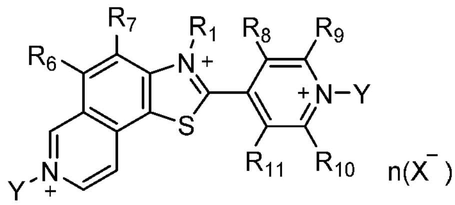

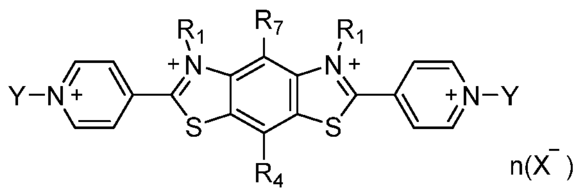

The compounds according to this first embodiment can also be the ones wherein $\mathbf{R_8}$ and $\mathbf{R_9}$ or $\mathbf{R_{10}}$ and $\mathbf{R_{11}}$ form an aromatic ring fused to the pyridium group they are attached to and $\mathbf{R_1}$, $\mathbf{R_4}$, $\mathbf{R_5}$, $\mathbf{R_6}$, $\mathbf{R_7}$, **Z**, **Y**, **n** and **X** are as defined above. An example of such a compound is the one shown below:

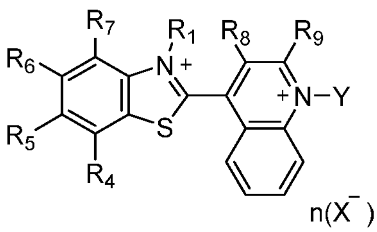

In this particular first embodiment, $\mathbf{R_1}$ is preferably $C_1$-$C_{18}$ alkyl; more preferably $\mathbf{R_1}$ is $C_1$-$C_6$ alkyl such as

methyl; $\mathbf{R_4}$ is preferably H, $C_1$-$C_{18}$ alkyl or aryl; more preferably $\mathbf{R_4}$ is H; $\mathbf{R_5}$ is preferably H, $C_1$-$C_{18}$ alkyl or aryl; more preferably $\mathbf{R_5}$ is H; $\mathbf{R_6}$ is preferably H, $C_1$-$C_{18}$ alkyl or aryl; more preferably $\mathbf{R_6}$ is H; $\mathbf{R_7}$ is preferably H; $C_1$-$C_{18}$ alkyl or aryl; more preferably $\mathbf{R_7}$ is H; $\mathbf{R_8}$ is preferably H, $C_1$-$C_{18}$ alkyl or aryl; more preferably $\mathbf{R_8}$ is H; $\mathbf{R_9}$ is preferably H, $C_1$-$C_{18}$ alkyl or aryl; more preferably $\mathbf{R_9}$ is H; **Y** is $C_1$-$C_6$ alkyl or aryl; even more preferably **Y** is phenyl; **n** is 2; and $\mathbf{X^-}$ is tetrafluoroborate or hexafluorophosphate.

In a second embodiment also part of the invention, the compounds of **Formula** (**II**) are those wherein **A** is $^+$N-$R_1$; **D** is N-$R_3$; **E** is C; and $\mathbf{R_1}$, $\mathbf{R_3}$, $\mathbf{R_4}$, $\mathbf{R_5}$, $\mathbf{R_6}$, $\mathbf{R_7}$, $\mathbf{R_8}$, $\mathbf{R_9}$, $\mathbf{R_{10}}$, $\mathbf{R_{11}}$, **Z, Y, n** and **X** are as defined above.

According to this second embodiment of the invention, compounds can be those of **formula** (**II**) wherein A is $^+$N-$R_1$; **D** is N-$R_3$; **E** is C; and $\mathbf{R_1}$ is $C_1$-$C_{18}$ alkyl; preferably $\mathbf{R_1}$ is $C_1$-$C_6$ alkyl such as methyl or *n*-hexyl; $\mathbf{R_4}$ is H, $C_1$-$C_{18}$ alkyl or aryl; preferably $\mathbf{R_4}$ is H; $\mathbf{R_5}$ is H, $C_1$-$C_{18}$ alkyl or aryl; preferably $\mathbf{R_5}$ is H; $\mathbf{R_6}$ is H, $C_1$-$C_{18}$ alkyl or aryl; preferably $\mathbf{R_6}$ is H; $\mathbf{R_7}$ is H; Ci-Cis alkyl or aryl; preferably $\mathbf{R_7}$ is H; $\mathbf{R_8}$ is H, $C_1$-$C_{18}$ alkyl or aryl; preferably $\mathbf{R_8}$ is H; $\mathbf{R_9}$ is H, $C_1$-$C_{18}$ alkyl or aryl; preferably $\mathbf{R_9}$ is H; $\mathbf{R_{10}}$ is H, $C_1$-$C_{18}$ alkyl or aryl; preferably $\mathbf{R_{10}}$ is H; $\mathbf{R_{11}}$ is H, $C_1$-$C_{18}$ alkyl or aryl; preferably $\mathbf{R_{11}}$ is H; **Y** is $C_1$-$C_6$ alkyl or aryl; even more preferably **Y** is methyl, *n*-hexyl or phenyl; **n** is 2; and $\mathbf{X^-}$ is tetrafluoroborate or hexafluorophosphate, preferably hexafluorophosphate.

Other preferred compounds according to this second embodiment of the invention are typically those of **formula** (**II**) wherein **A** is $^+$N-$R_1$, **D** is N-$R_3$; **E** is C; $\mathbf{R_7}$ and $\mathbf{R_6}$ and/or $\mathbf{R_6}$ and $\mathbf{R_5}$ and/or $\mathbf{R_5}$ and $\mathbf{R_4}$ form together an aromatic ring or heteroaromatic ring fused to the (hetero)cyclic core (Cycle *C6)* they are attached to, optionally substituted by **Z,** $\mathbf{R_1}$, $\mathbf{R_3}$, $\mathbf{R_8}$, $\mathbf{R_9}$, $\mathbf{R_{10}}$, $\mathbf{R_{11}}$, **Z, Y, n** and **X** being as defined above. An example of such a compound is the one shown below:

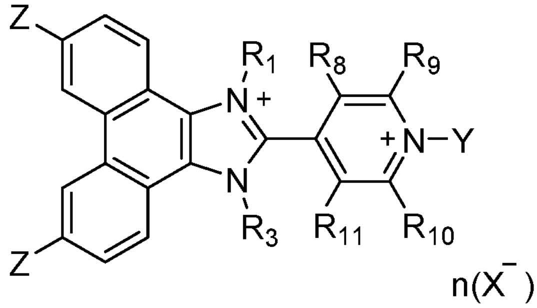

In this second embodiment of the invention, $\mathbf{R_1}$ is preferably $C_1$-$C_{18}$ alkyl; more preferably $\mathbf{R_1}$ is $C_1$-$C_6$ alkyl such as methyl; $\mathbf{R_3}$ is preferably $C_1$-$C_{18}$ alkyl or optionally substituted aryl, more preferably $\mathbf{R_3}$ is optionally substituted aryl such as 4-t-butylphenyl; $\mathbf{R_8}$ is preferably H, $C_1$-$C_{18}$ alkyl or aryl; more preferably $\mathbf{R_8}$ is H; $\mathbf{R_9}$ is preferably H, $C_1$-$C_{18}$ alkyl or aryl; more preferably $\mathbf{R_9}$ is H; **Y** is $C_1$-$C_6$ alkyl or aryl; even more preferably **Y** is methyl; **n** is 4; and $\mathbf{X^-}$ is tetrafluoroborate or hexafluorophosphate.

In a third embodiment of the present invention, the compounds of **Formula** (**II**) are those wherein **A** is N; **D** is Se; **E** is C; and $\mathbf{R_4}$, $\mathbf{R_5}$, $\mathbf{R_6}$, $\mathbf{R_7}$, $\mathbf{R_8}$, $\mathbf{R_9}$, $\mathbf{R_{10}}$, $\mathbf{R_{11}}$, **Z, Y, n** and **X** are as defined above.

According to this third embodiment, compounds can be those of **formula** (**II**) wherein: **A** is N; **D** is Se; **E** is C; $\mathbf{R_4}$ is preferably H, $C_1$-$C_{18}$ alkyl or aryl; more preferably $\mathbf{R_4}$ is H; $\mathbf{R_5}$ is preferably H, $C_1$-$C_{18}$ alkyl or aryl; more preferably $\mathbf{R_5}$ is H; $\mathbf{R_6}$ is preferably H, $C_1$-$C_{18}$ alkyl or aryl; more preferably $\mathbf{R_6}$ is H; $\mathbf{R_7}$ is preferably H, $C_1$-$C_{18}$ alkyl or aryl; more preferably $\mathbf{R_7}$ is H; $\mathbf{R_8}$ is preferably H, $C_1$-$C_{18}$ alkyl or aryl; more preferably $\mathbf{R_8}$ is H; $\mathbf{R_9}$ is preferably H, Ci-Cis alkyl or aryl; more preferably $\mathbf{R_9}$ is H; $\mathbf{R_{10}}$ is preferably H, Ci-Cis alkyl or aryl; more preferably $\mathbf{R_{10}}$ is H; $\mathbf{R_{11}}$ is preferably H, $C_1$-$C_{18}$ alkyl or aryl; more preferably $\mathbf{R_{11}}$ is H; **Y** is $C_1$-$C_6$ alkyl or aryl; even more preferably Y is n-hexyl or phenyl; **n** is 2; and $\mathbf{X^-}$ is tetrafluoroborate or hexafluorophosphate.

In a fourth embodiment of the present invention, the compounds of **Formula** (**II**) are those wherein **A** is N; **D** is O; **E** is C; and $\mathbf{R_4}$, $\mathbf{R_5}$, $\mathbf{R_6}$, $\mathbf{R_7}$, $\mathbf{R_8}$, $\mathbf{R_9}$, $\mathbf{R_{10}}$, $\mathbf{R_{11}}$, **Z, Y, n** and **X** are as defined above.

According to this fourth embodiment, compounds can be those of **formula** (**II**) wherein: **A** is N; **D** is O; **E** is C; $\mathbf{R_4}$ is preferably H, $C_1$-$C_{18}$ alkyl or aryl; more preferably $\mathbf{R_4}$ is H; $\mathbf{R_5}$ is preferably H, $C_1$-$C_{18}$ alkyl or aryl; more preferably $\mathbf{R_5}$ is H; $\mathbf{R_6}$ is preferably H, $C_1$-$C_{18}$ alkyl or aryl; more preferably $\mathbf{R_6}$ is H; $\mathbf{R_7}$ is preferably H, $C_1$-$C_{18}$ alkyl or aryl; more preferably $\mathbf{R_7}$ is H; $\mathbf{R_8}$ is preferably H, $C_1$-$C_{18}$ alkyl or aryl; more preferably $\mathbf{R_8}$ is H; $\mathbf{R_9}$ is preferably H, Ci-Cis alkyl or aryl; more preferably $\mathbf{R_9}$ is H; $\mathbf{R_{10}}$ is preferably H, Ci-Cis alkyl or aryl; more preferably $\mathbf{R_{10}}$ is H; $\mathbf{R_{11}}$ is preferably H, $C_1$-$C_{18}$ alkyl or aryl; more preferably $\mathbf{R_{11}}$ is H; **Y** is $C_1$-$C_6$ alkyl or aryl; even more preferably **Y** is *n*-hexyl or phenyl; **n** is 2; and $\mathbf{X^-}$ is tetrafluoroborate or hexafluorophosphate.

In a fifth embodiment of the present invention, the compounds of the invention are those of **Formula** (**II**) wherein **A** is $^+$N-$R_1$, **D** is C-$R_3$; **E** is N; and $\mathbf{R_1}$, $\mathbf{R_4}$, $\mathbf{R_5}$, $\mathbf{R_6}$, $\mathbf{R_7}$, $\mathbf{R_8}$, $\mathbf{R_9}$, $\mathbf{R_{10}}$, $\mathbf{R_{11}}$, **Z, Y, n** and **X** are as defined above.

According to this fifth embodiment, the compounds of the invention can be those wherein $\mathbf{R_1}$ is preferably $C_1$-$C_{18}$ alkyl; more preferably $\mathbf{R_1}$ is $C_1$-$C_6$ alkyl such as methyl; $\mathbf{R_3}$ is preferably H or $C_1$-$C_{18}$ alkyl, more preferably $\mathbf{R_3}$ is H; $\mathbf{R_4}$ is preferably H, $C_1$-$C_{18}$ alkyl or aryl; more preferably $\mathbf{R_4}$ is H; $\mathbf{R_5}$ is preferably H, $C_1$-$C_{18}$ alkyl or aryl; more

preferably **$R_5$** is H; **$R_6$** is preferably $C_1$-$C_{18}$ alkyl, aryl or Z; more preferably **$R_6$** is aryl or Z; **$R_7$** is preferably H, $C_1$-$C_{18}$ alkyl or aryl; more preferably **$R_7$** is H; **$R_8$** is preferably H, $C_1$-$C_{18}$ alkyl or aryl; more preferably **$R_8$** is H; **$R_9$** is preferably H, $C_1$-$C_{18}$ alkyl or aryl; more preferably **$R_9$** is H; **$R_{10}$** is preferably H, $C_1$-$C_{18}$ alkyl or aryl; more preferably **$R_{10}$** is H; **$R_{11}$** is preferably H, $C_1$-$C_{18}$ alkyl or aryl; more preferably **$R_{11}$** is H; **Y** is $C_1$-$C_6$ alkyl or aryl; even more preferably **Y** is *n*-hexyl or phenyl; **n** is preferably 2 or 3; and **$X^-$** is tetrafluoroborate or hexafluorophosphate.

In a sixth embodiment of the present invention, the compounds of the invention are those of **Formula (III)** wherein **B** is N or $^+$N-$R_2$; **D** is S; **E** is C; and **$R_2$**, **$R_4$**, **$R_5$**, **$R_6$**, **$R_7$**, **$R_8$**, **$R_9$**, **$R_{10}$**, **$R_{11}$**, **Z**, **Y**, **n** and **X** are as defined above.

According to this sixth embodiment of the present invention, the compounds of the invention can be the ones wherein **$R_2$**, when present, is preferably $C_1$-$C_{18}$ alkyl, more preferably **$R_2$** is $C_1$-$C_6$ alkyl such as methyl; **$R_4$** is preferably H, $C_1$-$C_{18}$ alkyl or aryl; more preferably **$R_4$** is H; **$R_5$** is preferably H, $C_1$-$C_{18}$ alkyl or aryl; more preferably **$R_5$** is H; **$R_6$** is preferably $C_1$-$C_{18}$ alkyl, aryl; more preferably **$R_6$** is H; **$R_7$** is preferably H, $C_1$-$C_{18}$ alkyl or aryl; more preferably **$R_7$** is H; **$R_8$** is preferably H, $C_1$-$C_{18}$ alkyl or aryl; more preferably **$R_8$** is H; **$R_9$** is preferably H, $C_1$-$C_{18}$ alkyl or aryl; more preferably **$R_9$** is H; **$R_{10}$** is preferably H, $C_1$-$C_{18}$ alkyl or aryl; more preferably **$R_{10}$** is H; **$R_{11}$** is preferably H, $C_1$-$C_{18}$ alkyl or aryl; more preferably **$R_{11}$** is H; **Y** is $C_1$-$C_6$ alkyl or aryl; even more preferably **Y** is *n*-hexyl or phenyl; **n** is 1 or 2; and **$X^-$** is tetrafluoroborate or hexafluorophosphate.

In a seventh embodiment of the present invention, the compounds of the invention are those of **Formula (III)** wherein **B** is N; **D** is O; **E** is C; and **$R_2$**, **$R_4$**, **$R_5$**, **$R_6$**, **$R_7$**, **$R_8$**, **$R_9$**, **$R_{10}$**, **$R_{11}$**, **Z**, **Y**, **n** and **X** are as defined above.

According to this seventh embodiment of the present invention, the compounds of the invention can be the ones wherein **$R_4$** is preferably H, $C_1$-$C_{18}$ alkyl or aryl; more preferably **$R_4$** is H; **$R_5$** is preferably H, $C_1$-$C_{18}$ alkyl or aryl; more preferably **$R_5$** is H; **$R_6$** is preferably $C_1$-$C_{18}$ alkyl, aryl; more preferably **$R_6$** is H; **$R_7$** is preferably H, $C_1$-$C_{18}$ alkyl or aryl; more preferably **$R_7$** is H; **$R_8$** is preferably H, $C_1$-$C_{18}$ alkyl or aryl; more preferably **$R_8$** is H; **$R_9$** is preferably H, $C_1$-$C_{18}$ alkyl or aryl; more preferably **$R_9$** is H; **$R_{10}$** is preferably H, $C_1$-$C_{18}$ alkyl or aryl; more preferably **$R_{10}$** is H; **$R_{11}$** is preferably H, $C_1$-$C_{18}$ alkyl or aryl; more preferably **$R_{11}$** is H; **Y** is $C_1$-$C_6$ alkyl or aryl; even more preferably **Y** is *n*-hexyl or phenyl; **n** is 1; and **$X^-$** is tetrafluoroborate or hexafluorophosphate.

In an eighth embodiment of the present invention, the compounds of the invention are those of **Formula (V)** wherein **A** is $^+$N-$R_1$; **B** is C-$R_2$; **D** is C-$R_3$; **E** is N; and **$R_1$**, **$R_2$**, **$R_3$**, **$R_4$**, **$R_5$**, **$R_7$**, **$R_8$**, **$R_9$**, **$R_{10}$**, **$R_{11}$**, **Z**, **Y**, **n** and **X** are as defined above.

According to this eighth embodiment of the present invention, the compounds of the invention can be the ones wherein **$R_1$** is preferably $C_1$-$C_{18}$ alkyl; more preferably **$R_1$** is $C_1$-$C_6$ alkyl such as methyl; **$R_2$** is preferably $C_1$-$C_{18}$ alkyl or aryl, more preferably **$R_2$** is aryl such as phenyl; **$R_3$** is preferably H, $C_1$-$C_{18}$ alkyl or aryl; more preferably **$R_3$** is H; **$R_4$** is preferably H, $C_1$-$C_{18}$ alkyl or aryl; more preferably **$R_4$** is H; **$R_5$** is preferably H, $C_1$-$C_{18}$ alkyl or aryl; more preferably **$R_5$** is H; **$R_7$** is preferably H, $C_1$-$C_{18}$ alkyl or aryl; more preferably **$R_7$** is H; **$R_8$** is preferably H, $C_1$-$C_{18}$ alkyl or aryl; more preferably **$R_8$** is H; **$R_9$** is preferably H, $C_1$-$C_{18}$ alkyl or aryl; more preferably **$R_9$** is H; **$R_{10}$** is preferably H, $C_1$-$C_{18}$ alkyl or aryl; more preferably **$R_{10}$** is H; **$R_{11}$** is preferably H, $C_1$-$C_{18}$ alkyl or aryl; more preferably **$R_{11}$** is H; **Y** is $C_1$-$C_6$ alkyl or aryl; even more preferably **Y** is *n*-hexyl or phenyl; **n** is 2; and **$X^-$** is tetrafluoroborate or hexafluorophosphate.

Some compounds not part of the present invention are those of **Formula (IX) or (X)** wherein **A** is N-$R_1$; **B** is C-$R_2$; **D** is C-$R_3$; E is N; and **$R_1$**, **$R_2$**, **$R_3$**, **$R_8$**, **$R_9$**, **$R_{10}$**, **$R_{11}$**, **Z**, **Y**, **n** and **X** are as defined in above.

Those compounds not belonging to the present invention of **Formula (IX) or (X)** wherein **A** is N-$R_1$; **B** is C-$R_2$; **D** is C-$R_3$; and **E** is N, can be the ones wherein **$R_1$** is preferably $C_1$-$C_{18}$ alkyl; more preferably **$R_1$** is $C_1$-$C_6$ alkyl such as methyl; **$R_2$** is preferably substituted aryl, more preferably **$R_2$** is 4-*t*-butylphenyl or phenyl substituted by **Z; $R_3$** is preferably $C_1$-$C_{18}$ alkyl or aryl; more preferably **$R_3$** is aryl, for example 4-*t*-butylphenyl; **$R_8$** is preferably H, $C_1$-$C_{18}$ alkyl or aryl; more preferably **$R_8$** is H; **$R_9$** is preferably H, $C_1$-$C_{18}$ alkyl or aryl; more preferably **$R_9$ is H; $R_{10}$** is preferably H, $C_1$-$C_{18}$ alkyl or aryl; more preferably **$R_{10}$** is H; **$R_{11}$** is preferably H, $C_1$-$C_{18}$ alkyl or aryl; more preferably **$R_{11}$** is H; **Y** is preferably $C_1$-$C_{18}$ alkyl or aryl; more preferably **Y** is $C_1$-$C_6$ alkyl such as methyl; **n** is 3 or 4; and **$X^-$** is tetrafluoroborate or hexafluorophosphate.

In a particularly preferred embodiment of the present invention defined in the appended claims, the compound of formula (I) is selected from the group consisting of:

| Compound | Structure |
|---|---|
| 1 | 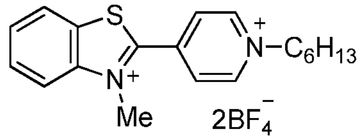 |

(continued)

| Compound | Structure |
|---|---|
| 2 | 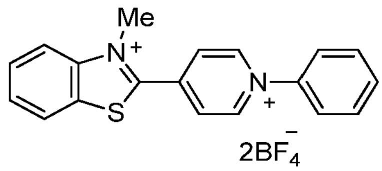 |
| 3 | 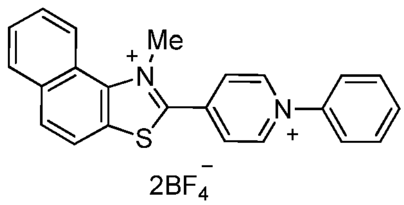 |
| 4 | 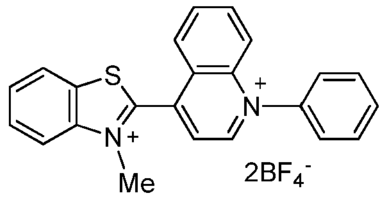 |
| 5 | 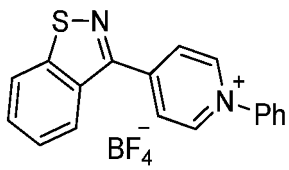 |
| 6 | 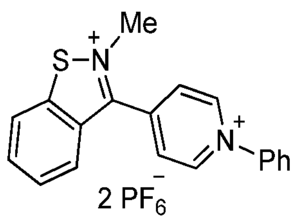 |
| 7 | 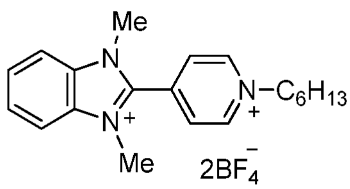 |
| 8 | 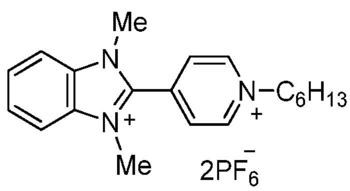 |
| 9 | 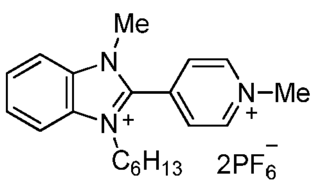 |

(continued)

| Compound | Structure |
|---|---|
| 10 | 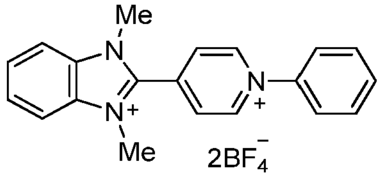 |
| 11 | 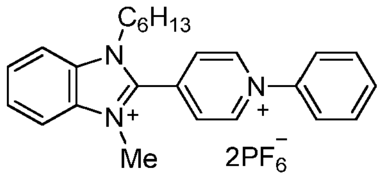 |
| 12 | 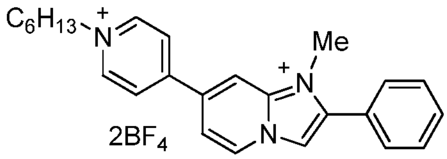 |
| 13 | 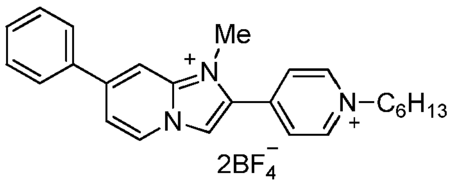 |
| 14 | 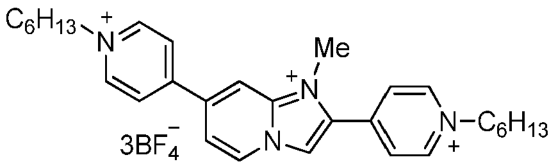 |
| 15 | 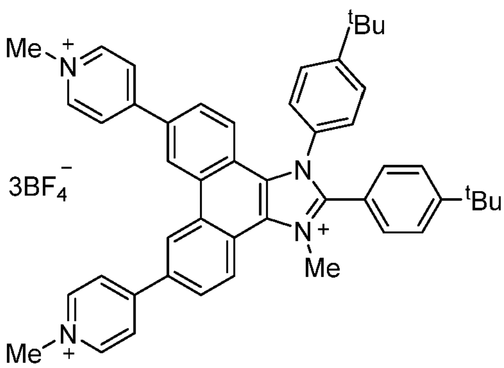 |

(continued)

| Compound | Structure |
|---|---|
| 16 | 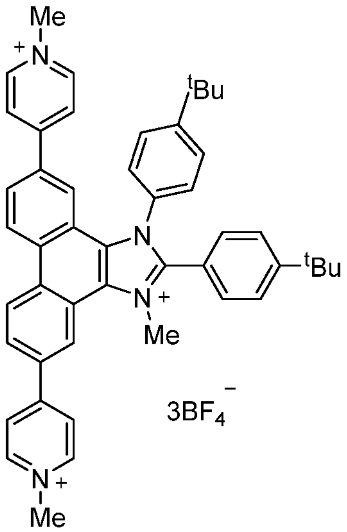 |
| 17 | 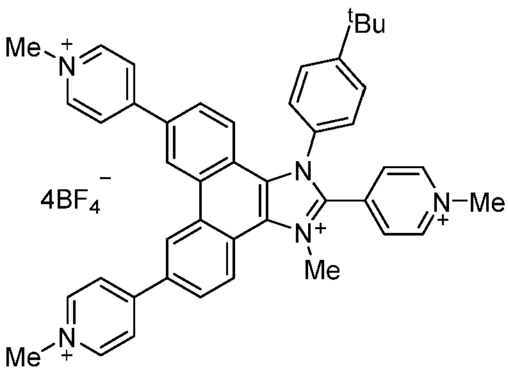 |
| 18 | 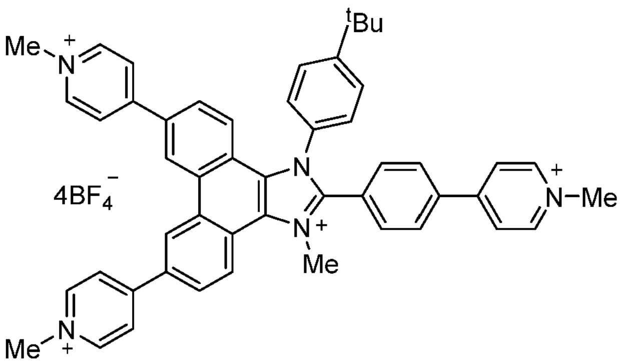 |
| 19 | 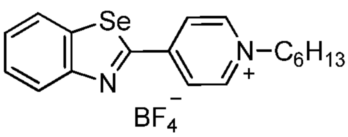 |
| 20 | 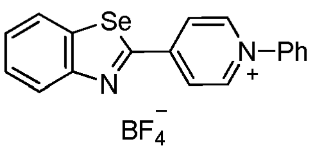 |

(continued)

| Compound | Structure |
|---|---|
| 21 | 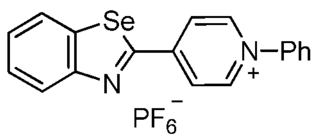 |
| 22 | 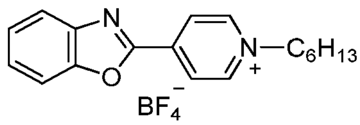 |
| 23 | 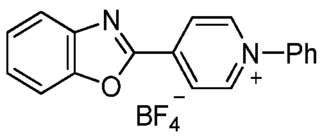 |
| 24 | 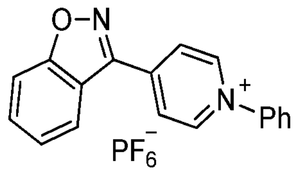 |

Compounds represented by formula (I) according to the appended claims may be prepared according to various methods well known in the art.

For example, compounds represented by formula (I) according to the appended claims may be obtained according to the synthetic routes detailed hereinafter.

A variety of routes to access benzimidazole and benzothiazole derivatives have been described; the simplest and most useful of which involve annulation of the heterocyclic ring onto a 1,2-phenylenediamine or a 2-aminothiophenol respectively. Synthetic routes involving these precursors have been reviewed. (J. Revuelta, F. Machetti and S. Cicchi in Modern Heterocyclic Chemistry eds. J. Alvarez-Builla, J. J. Vaquero and J. Barluenga, Wiley-WCH, Weinheim, 2011, vol. 2, pp. 809-923.)

Although 2-(hetero)arylbenzothiazoles are accessible via a tandem acylation-cyclodehydration sequence of a 2-aminothiophenol, other routes are known. For example, 2-(4-pyridyl)benzothiazole was first obtained from the high temperature reaction of 4-picoline with sulfur in the presence of aniline, or from isonicotinic acid and 2-aminothiophenol in the presence of thionyl chloride. Moreover the same compound could be obtained by cyclocondensation of 2-aminothiophenol with pyridine-4-carboxaldehyde and subsequent oxidation of the resulting benzothiazoline with iron(III) sulfate (P. E. Miller, G. L. Oliver, J. R. Dann and J. W. Gates Jr., J. Org. Chem., 1957, 22, 664). More recently it has been shown that benzothiazoles can also be obtained from aldehydes or alkanophenones and 2-aminothiophenol; the oxidation step is accomplished by aerial oxidation in DMSO (Y. Liao, H. Qi, S. Chen, P. Jiang, W. Zhou and G.-J. Deng, Org. Lett., 2012, 14, 6004). A combination of these approaches was used in the present work in which an ethanolic solution of 2-aminothiophenol and pyridine-4-carboxaldehyde was stirred in air. Subsequent N-alkyation with a haloalkane e.g. 1-iodohexane proceeded readily on the pyridine moiety as expected (K. Halman and O. H. Hankovszky, Acta Chim. Acad. Sci. Hung., 1965, 43, 263; P. Zavins, E. S. Lavinovitch and A. Arens, Khim. Geterotsikl. Soedin., 1973, 104). Thus 1-iodohexane provided a good yield of the iodide salt and ultimately the tetrafluoroborate following anion exchange. The latter could then be further alkylated on N-3 of the benzothiazole moiety with methyl tosylate as shown in scheme 1.

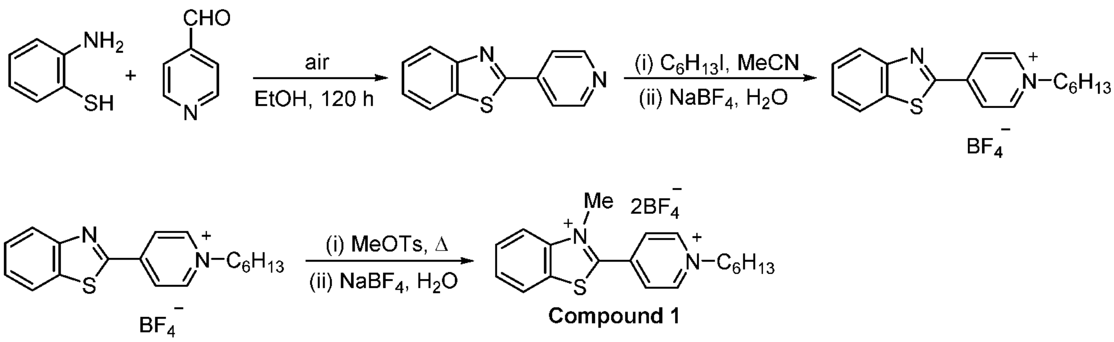


Compound 1

Scheme 1

Although 2-(1-phenylpyridin-4-yl-1-ium)benzothiazole has been synthesised by a convoluted sequence using pyrylium salt chemistry (G. N. Dorofeenko, A. V. Koblik, B. A. Tertov and T. I. Polyakova, Khim. Geterotsikl. Soedin., 1973, 1016) a simpler approach to this and derived salts is illustrated in scheme 6. The key step involves N-arylation of the pyridine moiety with readily available diphenyliodonium triflate (M. Bielawski and B. Olofsson, Chem. Commun., 2007, 2521) under copper catalysis. This approach has been employed for the N-phenylation of a range of other heterocyclic systems (T. Lv, Z. Wang, J. You, J. Lan and G. Gao, J. Org. Chem., 2013, 73, 5723; C. Reus, M. Stolar, J. Vanderkley, J. Neubauer and T. Baumgartner, J. Am. Chem. Soc., 2015, 137, 11710). Alkylation of the benzothiazole with methyl tosylate proceeds straightforwardly.

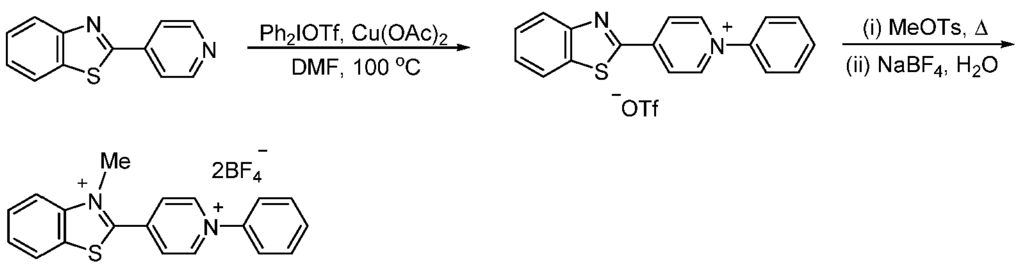


Compound 2

Scheme 2

Similar sequences to those depicted in Schemes 1 and 2 are applicable to readily available quinoline-4-carbaldehyde (W.-Z. Weng, J.-S. Guo, K.-X. Liu, T.-Q. Shao, L.-Q. Song, Y.-P. Zhu, Y.-Y. Sun and Q.-G. Meng, Can. J. Chem., 2020, 98, 179.) and the derived benzothiazole.

Although accessible from *o*-phenylenediamine (OPD) and carboxylic acid derivatives, both 2-(2-pyridyl)- and 2-(4-pyridyl)- benzimidazole were obtained efficiently via condensation-aerial oxidation of the appropriate pyridinecarboxaldehyde with OPD (cf. S. Haneda, Z. Gan, K. Eda and M. Hayashi, Organometallics, 2007, 26, 6551). 2-(4-Pyridyl)benzimidazoles could be selectively methylated in the imidazole ring by treatment with MeI under basic conditions. Sequential alkylations within the pyridine moiety and benzimidazole rings have been accomplished. In a similar manner to that employed previously, the Cu-mediated *N*-arylation of the pyridine moiety in 1-methyl-2-(4-pyridyl)benzimidazole with diphenyliodonium triflate followed by treatment with a haloalkane or with MeOTs has provided access to a novel series of compounds (Scheme 3).

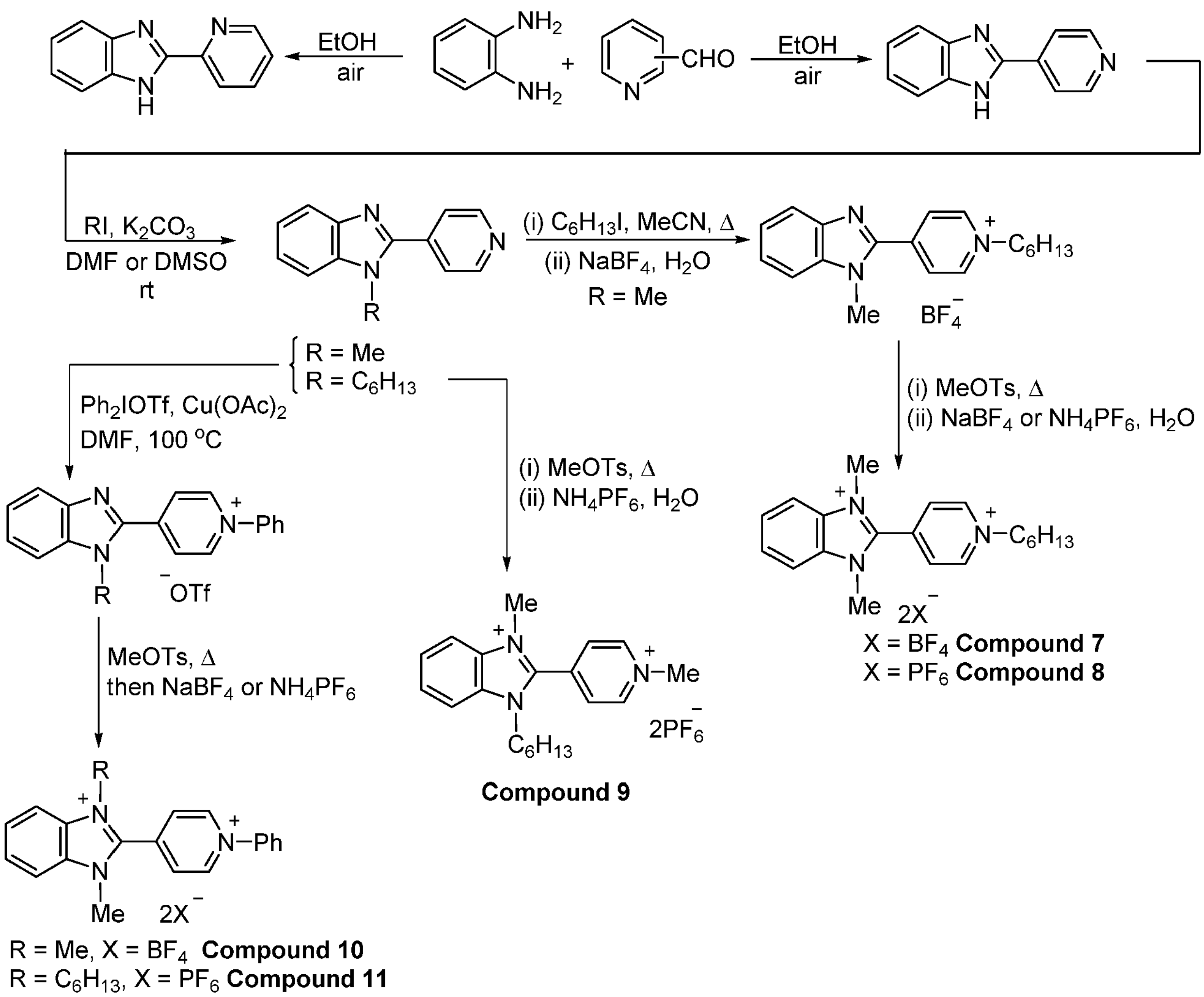


Scheme 3

Imidazo[1,2-*a*]pyridines are available by a variety of routes and a number of reviews of the synthesis and chemistry of these compounds are available (H. L. Blewitt in Special Topics in Heterocyclic Chemistry eds. A. Weissberger and E. C. Taylor, Wiley-Interscience, New York, 1977, pp. 117-178; F. Couty and G. Evano, Comprehensive Heterocyclic Chemistry III eds. A. R. Katritzky, C. A. Ramsden, E. F. V. Scriven and R. J. K. Taylor, Elsevier, Oxford, 2008, vol. 11, pp. 409-499; A. K. Bagdi, S. Santra, K. Monir and A. Hajra, Chem. Commun., 2015, 51, 1555; S. M. Roopan, S. M. Patil and J. Palaniraja, Res. Chem. Intermed., 2016, 42, 2740). By far the most useful entry to these bicyclic rings involves the N-alkylation-cyclocondensation of 2-aminopyridines with α-bromo ketones. Thus, reaction of commercially available 2-amino-4-bromopyridine with phenacyl bromide in methanol in the presence of $NaHCO_3$ provided 7-bromo-2-phenylimidazo[1,2-*a*]pyridine **A** [K. C. L. Lee and E. T. Sun, PCT WO2006/101455A1 (2006)]. An analogous reaction in which 4-(bromoacetyl)pyridine hydrobromide (M. P. Hay, S. Turcette, J. U. Flannagan, M. Bonnet, D. A. Chan, P. D. Sutphin, P. Nguyen, A. J. Graccia and W. A. Denny, J. Med. Chem., 2010, 53, 787) replaced phenacyl bromide furnished 7-bromo-2-(4-pyridyl)imidazo[1,2-*a*]pyridine **B** in good yield. Suzuki-Miyaura coupling of **A** with 4-(4,4,5,5-tetramethyl-1,3,2-dioxaborolan-2-yl)pyridine provided 2-phenyl-7-(4-pyridyl)imidazo[1,2-a]pyridine **C** in high yield. Application of this Pd-coupling reaction to **B** was employed to generate the 7-phenyl derivative **D** and the bis-pyridyl compound **E.** Alkylation of **C** and **D** with 1-iodohexane in MeCN proceeded on the pyridine nitrogen to give the salts **F** and **G** respectively. Further alkylation of **F** and **G** at N-1 was accomplished by heating with methyl tosylate. Bis-alkylation of both pyridine moieties in **E** could be accomplished by heating with excess alkyl halide to provide **H** that could also be methylated on N-1 with methyl tosylate. (Scheme 4)

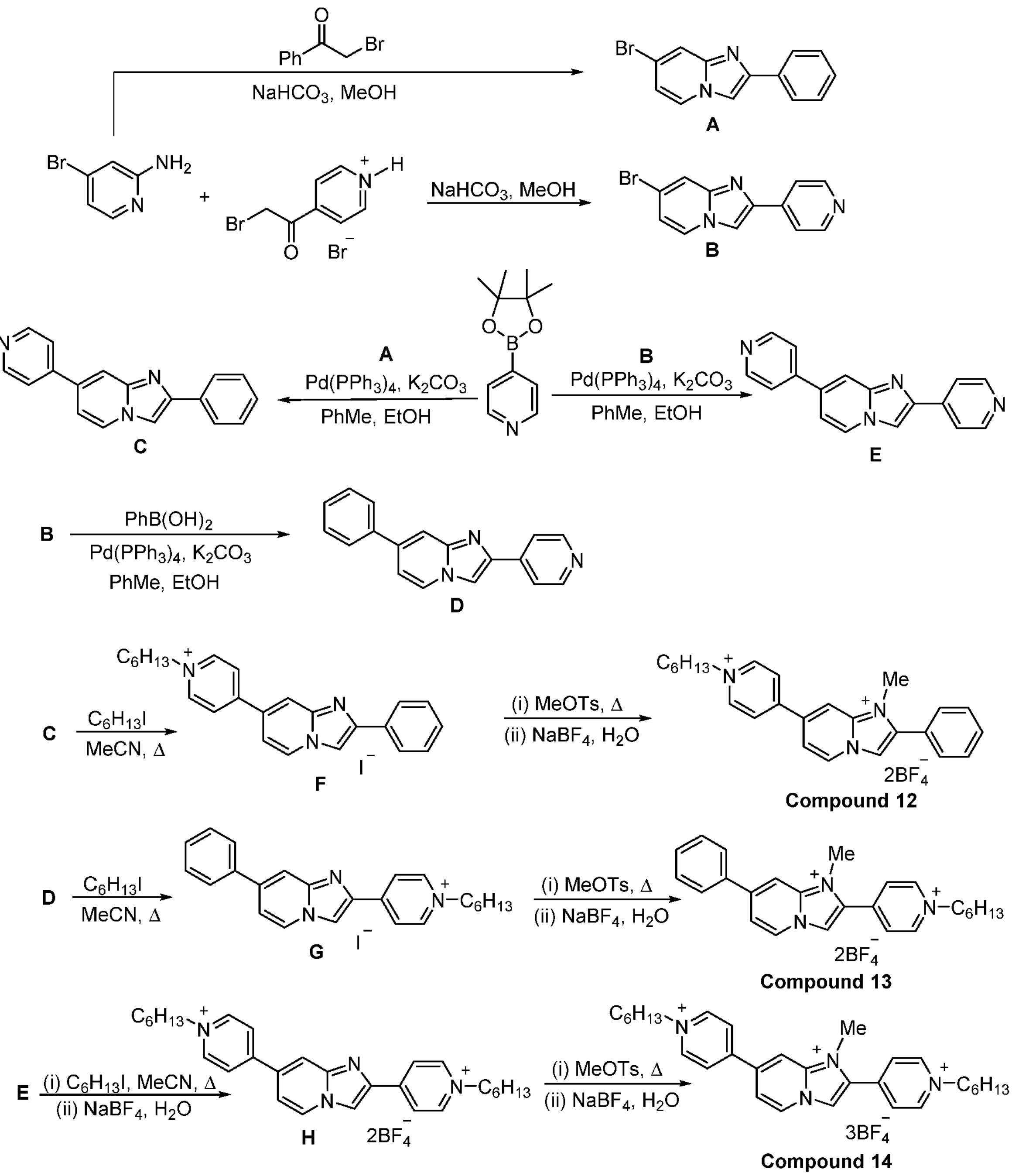


Scheme 4

4,4'-Dibromobenzil is widely commercially available and the isomeric dibromophenanthrene-9,10-diones, 3,6-dibromophenanthrene-9,10-dione (M. O. BaniKhaled, J. D. Mottishaw, H. Sun, Cryst. Growth Des., 2015, 15, 2235; A. Patel, S. Y. Sharp, K. Hall, W. Lewis, M. F. G. Stevens, P. Workman, C. J. Moody, Org. Biomol. Chem., 2016, 14, 3889) and 2,7-dibromophenanthrene-9,10-dione (T. H. Vo, M. Shekhirev, D. A. Kunkel, F. Orange, M. J.-F. Guinel, A. Endersbe, A. Sinitskii, Chem. Commun., 2014, 50, 4172), were prepared by selective bromination of phenanthrene-9,10-dione according to published literature procedures.

The multicomponent condensation reaction between a 1,2-dicarbonyl compound an aromatic aldehyde, an aniline and a small molecule capable of providing a nitrogen atom such as ammonium acetate in a suitable medium is a versatile route to highly substituted imidazoles and fused imidazoles (K. Skonieczny, D. T. Gryko, J. Org. Chem., 2015, 80, 5753; M. M. Heravil, M. Daraiel, V. Zadsirjan, Mol. Divers., 2015, 19, 577**;** D. Kumar, K.R. J. Thomas, J. Photochem. Photobiol. A: Chem., 2011, 218, 162). The synthesis of 6,9-dibromo-1,2-bis(4-(*tert*-butyl)phenyl)-1H-phenanthro[9,10-d]imidazole has been accomplished by such a multicomponent condensation between 3,6-dibromophenanthrene-9,10-dione, 4-tert-butylbenzaldehyde, 4-tert-butylaniline and ammonium acetate in acetic acid (W-C. Chen, Y. Yuan, Y. Xiong, A. L. Rogach, Q-X. Tong, C-S. Lee, ACS Appl. Mater. Interfaces, 2017, 9, 26268). Using identical reaction conditions and reagents 2,7-dibromophenanthrene-9,10-dione was transformed into 5,10-dibromo-1,2-bis(4-(tert-butyl)phenyl)-1*H*-phenanthro[9,10-*d*]imidazole (Scheme 5).

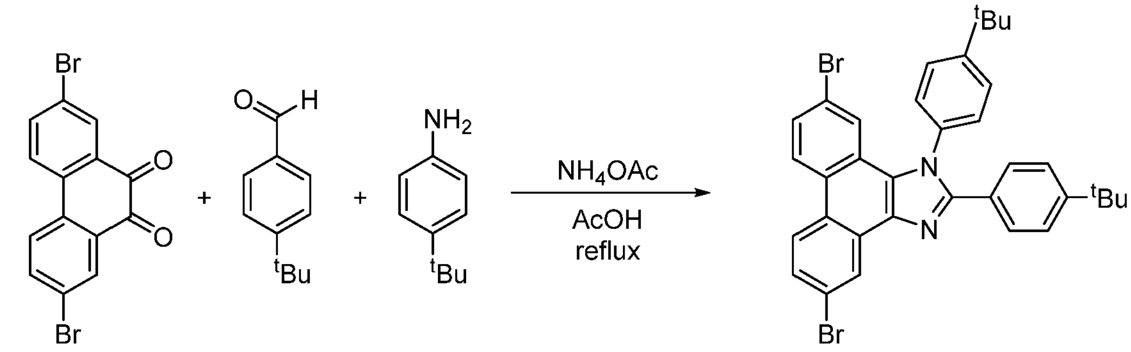


Scheme 5

Repeating the multicomponent condensation depicted in scheme 1 but starting from 3,6-dibromophenanthrene-9,10-dione and using either 4-bromobenzaldehyde or pyridine-4-carboxaldehyde as the aldehyde component gave 6,9-dibromo-2-(4-bromophenyl)-1-(4-(*tert*-butyl)phenyl)-1*H*-phenanthro[9,10-d]imidazole or 6,9-dibromo-1-(4-(*tert*-butyl)phenyl)-2-(pyridin-4-yl)-1*H*-phenanthro[9,10-d]imidazole, respectively (Scheme 6).

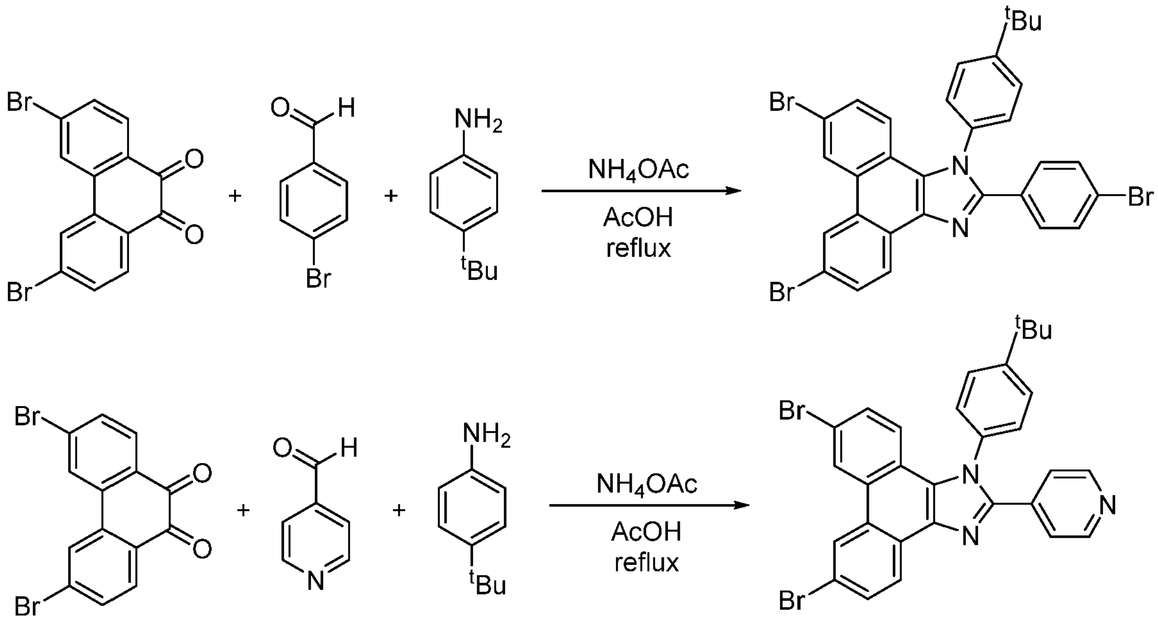


Scheme 6

The foregoing bromo substituted imidazoles and phenanthro[9,10-*d*]imidazoles were each subjected to a generic Suzuki cross-coupling protocol with 4-(4,4,5,5-tetramethyl-1,3,2-dioxaborolan-2-yl)pyridine employing a carbonate base with a palladium catalyst, typically tetrakis(triphenylphosphine)palladium(0), in a mixed solvent system to afford the poly (4-pyridyl)substituted imidazoles and phenanthro[9,10-*d*]imidazoles (Scheme 7). The Suzuki coupling of 4-(4,4,5,5-tetramethyl-1,3,2-dioxaborolan-2-yl)pyridine with aryl bromides has been widely reported (M. Juricek, J. C. Barnes, E. J. Dale, W-G. Liu, N. L. Strutt, C. J. Bruns, N. A. Vermeulen, K. C. Ghooray, A. A. Sarjeant, C. L. Stern, Y. Y. Botros, W. A. Goddard, J. F. Stoddart, J. Am. Chem. Soc., 2013, 135, 12736; Y. Nakamura, N. Aratani, A. Osuka,

Chem. Asian J., 2007, 2, 860; V. Gray, K. Börjesson, D. Dzebo, M. Abrahamsson, B. Albinsson, K. Moth-Poulsen, J. Phys. Chem. C, 2016, 120, 19018) (Scheme 7).

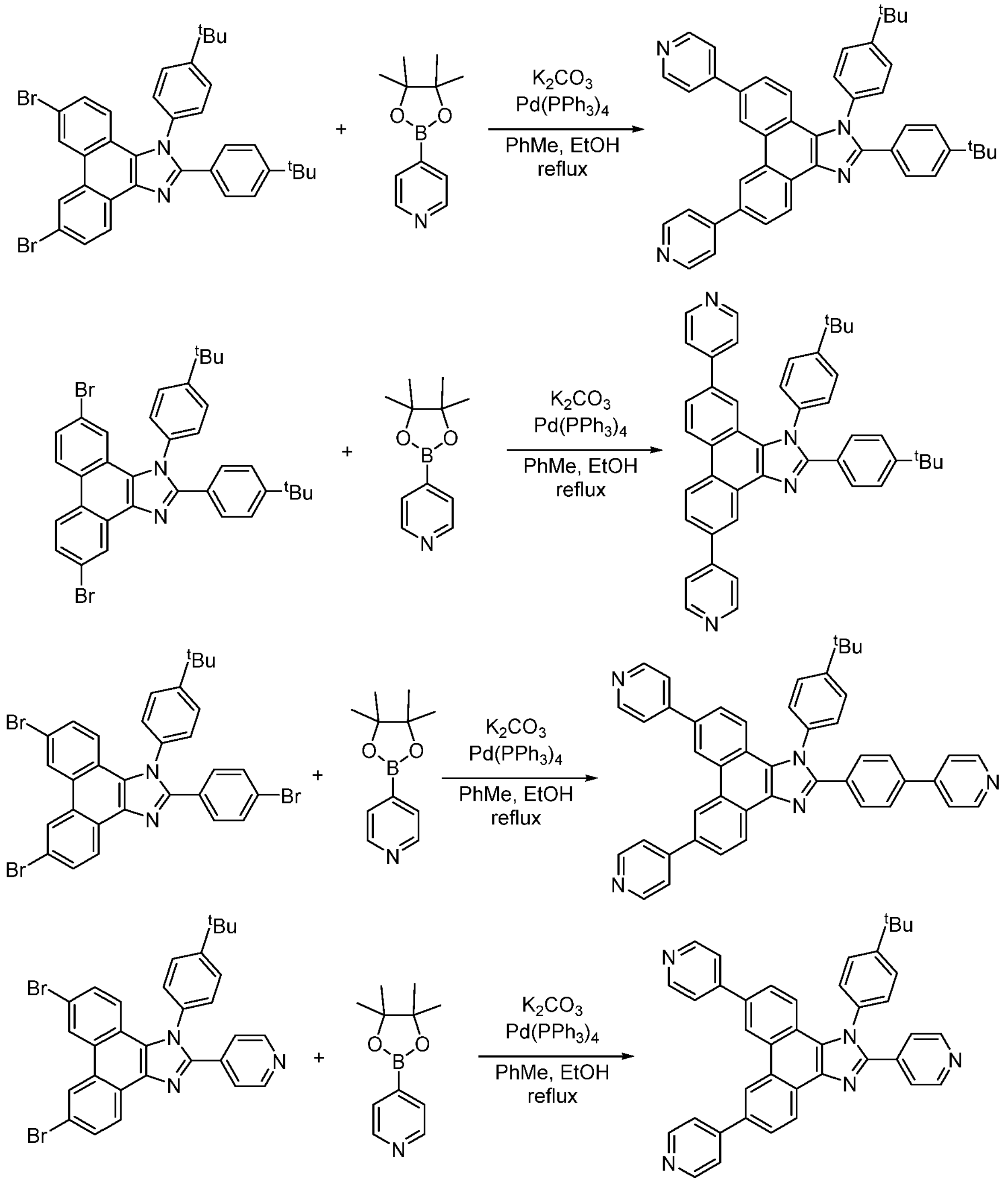


Scheme 7

Methyl tosylate has been used as an effective methylating agent either neat or with a co-solvent (J. F. S. Carvalho, J. Louvel, M. L. J. Doornbos, E. Klaase, Z. Yu, J. Brussee, A. P. Ijzerman, J. Med. Chem., 2013, 56, 2828; A. N. Wodward, J. M. Kolesar, S. R. Hall, N-A. Saleh, D. S. Jones, M. G. Walter, J. Am. Chem. Soc., 2017, 139, 8467; L. Pescatori, A. Arduini, A. Pochini, A. Secchi, C. Massera, F. Ugozzoli, Org. Biomol. Chem., 2009, 7, 3698; M. Kuroboshi, T. Yamamoto, H. Tanaka, Synlett, 2013, 24, 0197; J. A. Zoltewicz, M. P. Cruskie, Jr., Tetrahedron, 1995, 51, 3103). In this work methyl tosylate was employed to simultaneously *N*-methylate the pyridine ring N-atoms and N3-of the imidazole ring. The resulting poly (tosylate) salts were directly converted into their fluoroborate salts (Scheme 8).

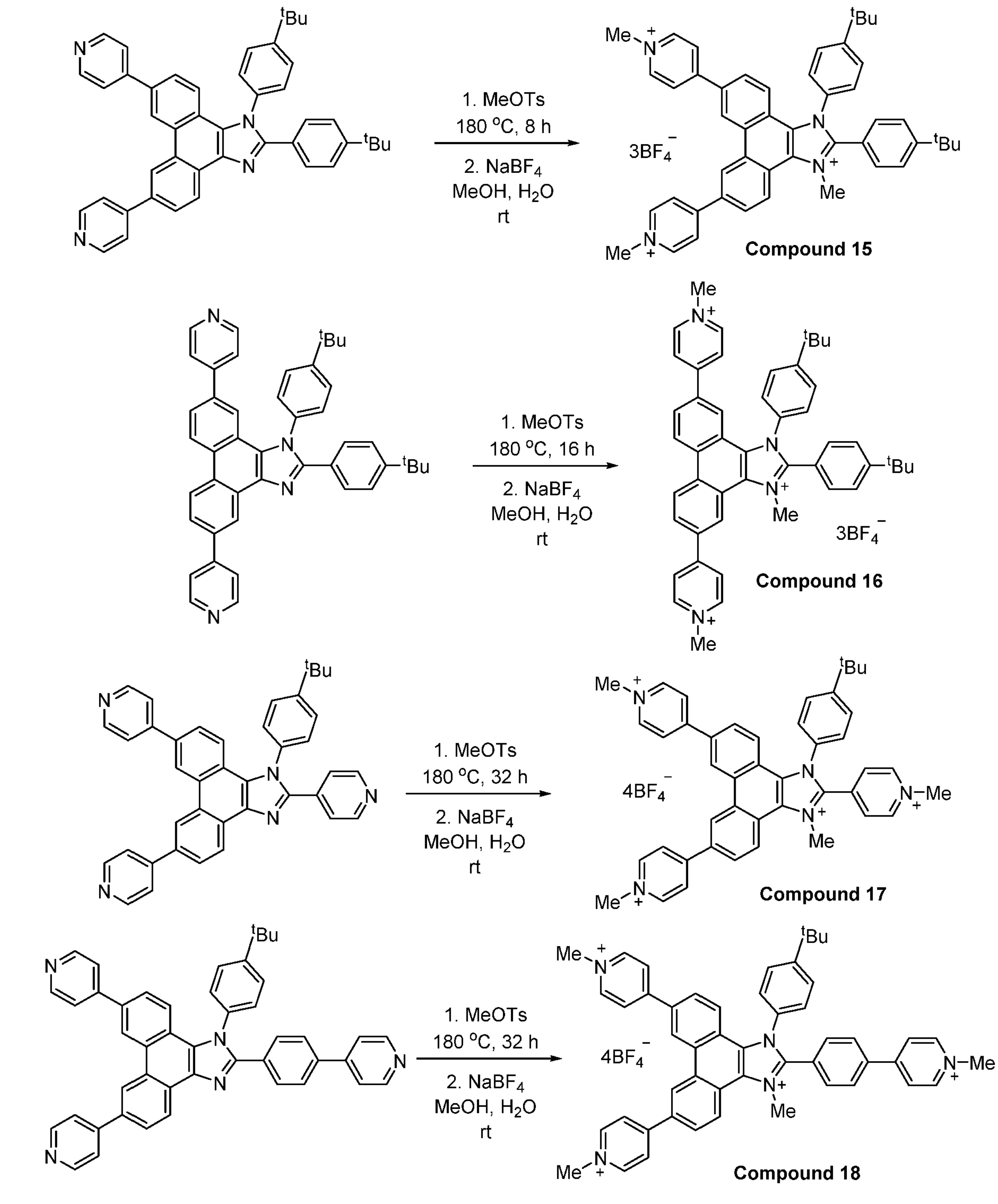


**Compound 15**

**Compound 16**

**Compound 17**

**Compound 18**

Scheme 8

2-(Pyridin-4-yl)benzoselenazole was synthesised according to a modified literature procedure (T. Su, S. Xie, B. Li, J. Yan, L. Huang, X. Li, Synlett 2015; 26, 215). Selective alkylation of the pyridine *N*-atom of 2-(pyridin-4-yl)benzoselenazole was accomplished upon reaction with an appropriate alkyl halide (Scheme 11). Alternatively, pyridine N-atom arylation of 2-(pyridin-4-yl)benzoselenazole was accomplished using an aryliodonium salt according to general literature protocols (T. Lv, Z. Wang, J. You, J. Lan and G. Gao, J. Org. Chem., 2013, 73, 5723; C. Reus, M. Stolar, J. Vanderkley, J. Neubauer and T. Baumgartner, J. Am. Chem. Soc., 2015, 137, 11710). A final counterion exchange with either $NaBF_4$ or $NH_4PF_6$ afforded the target electrochromic compounds (Scheme 9).

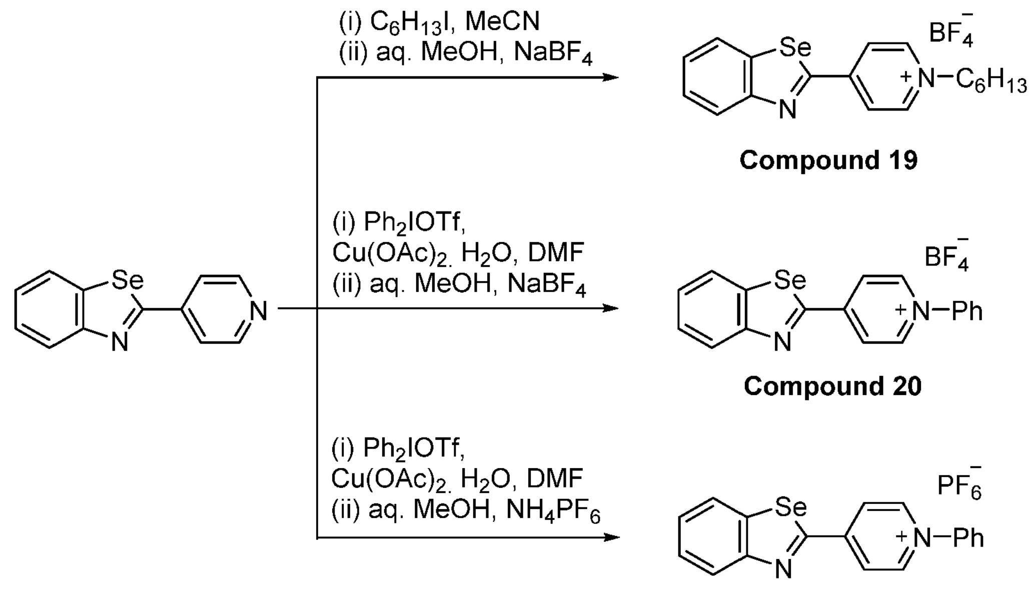


**Compound 19**

**Compound 20**

**Compound 21**

Scheme 9

Benzoxazoles are accessible by similar routes used to access the benzothiazoles. The condensation of 2-aminophenol with pyridine-4-carboxaldehyde in the presence of air affords exclusively the 2,3-dihydrobenzoxazole derivative. Subsequent dehydrogenation to 2-(4-pyridyl)benzoxazole is readily accomplished by treatment with 2,3-dichloro-5,6-dicyano-1,4-benzoquinone (DDQ) in dichloromethane. Both *N*-alkylation and *N*-arylation of the pyridine moiety can be accomplished by standard procedures (Scheme 10).

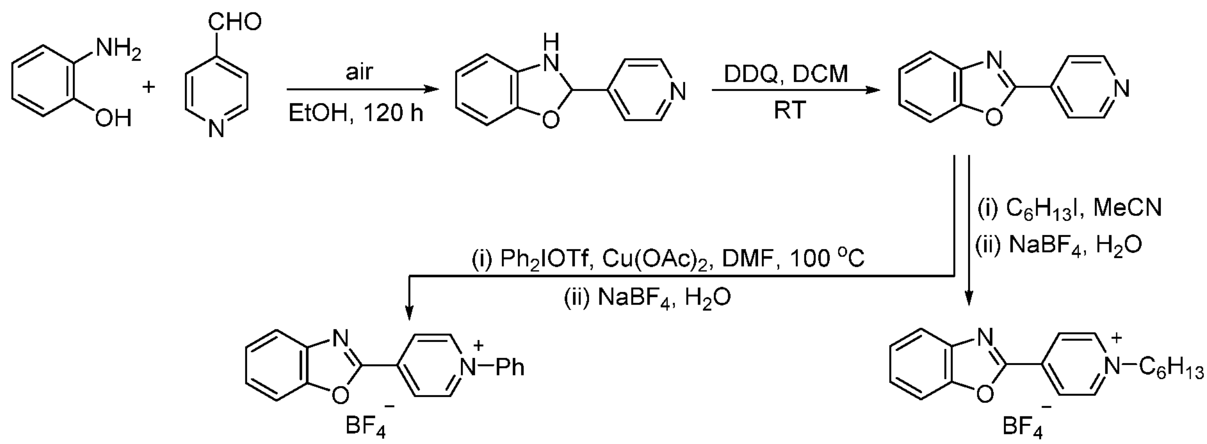


**Compound 23**

**Compound 22**

Scheme 10

Numerous methods are available to construct other fused-ring azoles. For example, 2-arylnaphtho[1,2-*d*]thiazoles are accessible from a one-pot dehydrogenation (Semmler-Wolff aromatisation)-sulfur transfer-cyclocondensation sequence between 1-tetralone oxime *O*-acetate and aryl aldehydes in the presence of elemental sulfur in DMSO (Z. Xu, H. Huang, H. Chen and G.-J. Deng, Org. Chem. Front., 2019, 6, 3060). In this way, 2-(pyridin-4-yl)benzothiazole has been obtained and the pyridine moiety selectively *N*-arylated by the usual means with diphenyliodonium triflate under Cu-catalysis. Subsequent *N*-methylation of the thiazole ring was accomplished by treatment with MeOTf. (Scheme 11) These quaternisation reactions are widely applicable to other bi-heterocyclic systems.

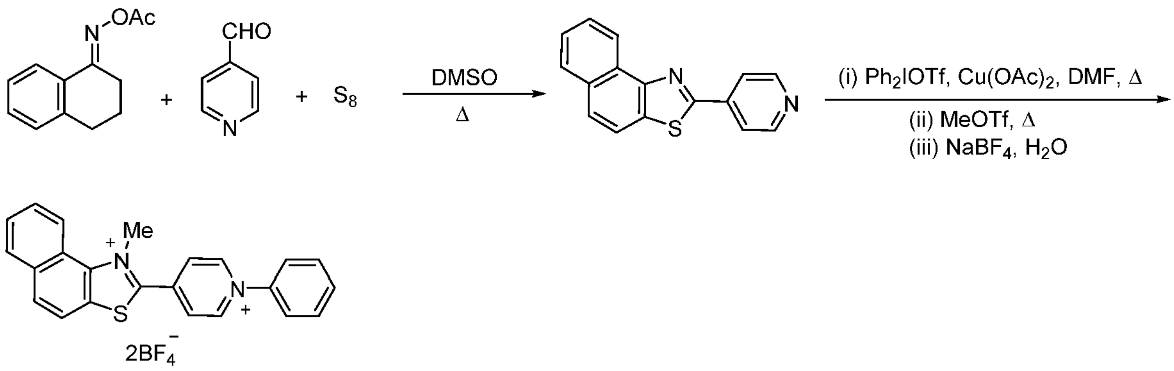


**Compound 3**

Scheme 11

3-Aryl-1,2-benzisothiazoles including the 3-(pyridin-4-yl)- derivative can be accessed *via* quenching the thioanisole dianion (obtained by lithiation with BuLi-TMEDA in TBME) with 4-pyridinecarbonitrile (R. Zhu, Z. Liu, J. Chen, X. Xiong, Y. Wang, L. Huang, J. Bai, Y. Deng and J. Huang, Org. Lett., 2018, 20, 3161) (Scheme 12)

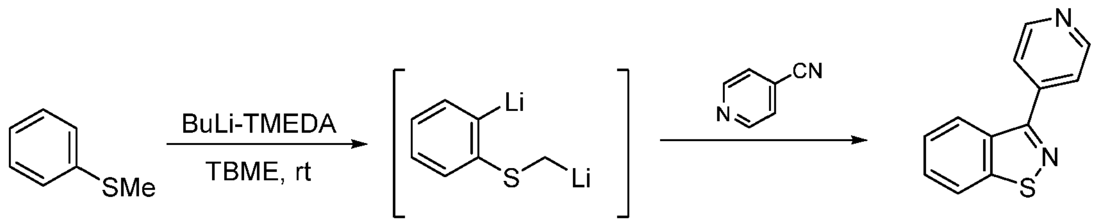


Scheme 12

3-(Hetero)aryl-1,2-benzisoxazoles are readily available by an intramolecular cyclisation of oximes or imines derived from 2-halogeno- or 2-hydroxy- benzophenones (for a review see F. Gualtieri and M. Giannella in Isoxazoles eds. P. Grünanger and P. Vita Finzi, Chemistry of Heterocyclic Compounds, John Wiley & Sons Inc., 1999, vol. 48, part 2, p. 1). 3-(Pyridin-4-yl)-1,2-benzisoxazole was prepared *via* oxidative cyclisation of the imine derived from 4-(2-hydroxybenzoyl)pyridine with N-chlorosuccinimide (Scheme 13) according to the literature procedure (H. Hepburn and T. J. Donohoe, Chem. Eur. J., 2020, 26, 1963).

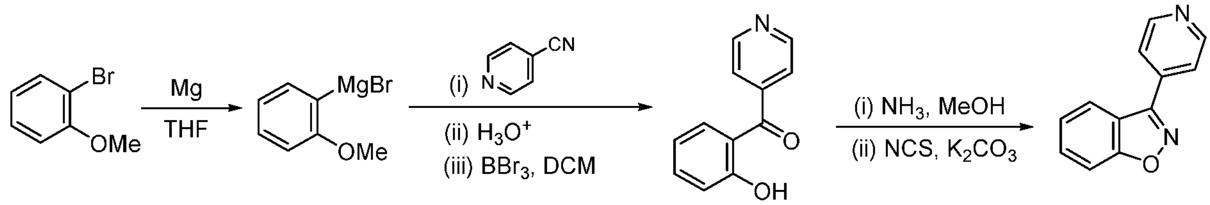


Scheme 13

## Electrochromic composition

The present invention also relates to electrochromic compositions, as defined in the claims.

Said electrochromic compositions comprise at least one compound of formula (I) as defined in the appended claims, as an oxidizing electrochromic compound. One or more additional oxidizing electrochromic compounds can be added to the composition of the invention so as to adapt the colour or the intensity of the coloured state of the composition. Said additional compound can be another compound of formula (I) according to the appended claims, or a different compound such as compatible dyes or pigments. For example, the additional oxidizing electrochromic compound can be selected from alkylviologens, arylviologens, alkylarylviologens or anthraquinone and derivatives. Preferably, the additional compound has a redox potential close to the compound of formula (I).

The composition may also comprise at least one reducing compound. The reducing compound may also be

an electrochromic compound. Example of reducing compounds include 5,10-dihydrophenazine, phenothiazine, phenoxazine, N,N,N',N'-tetramethyl-p-phenylenediamine, thioanthrene, tetrathiafulvalene, ferrocene and their derivatives.

The composition of the invention according to the appended claims may comprise a host medium that may be a fluid, a mesomorphous medium or a gel. The host medium is introduced in the composition of the invention to dissolve the electrochromic compounds. The host medium is preferably selected from the group consisting of organic solvents, liquid crystals, polymers, liquid crystal polymers and mixtures thereof.

Examples of suitable organic solvents that can be used as host medium are redox-compatible solvents which cannot react with the electrochromic compounds of the composition, such as ethylene carbonate, propylene carbonate, γ-butyrolactone, γ-valerolactone, acetronitrile, propionitrile, benzonitrile, glutaronitrile, methylglutaronitrile, dimethylformamide, N-methylpyrrolidone, sulfolane, 3-methyl sulfolane, benzene, toluene, methyl ethyl ketone, acetone, ethanol, tetrahydrofurfuryl alcohol, 2-methoxyethyl ether, xylene, cyclohexane, 3-methylcyclohexanone, ethyl acetate, ethyl phenylacetate, tetrahydrofuran, methanol, methyl propionate, ethylene glycol, ethylene carbonate, ionic liquids, and mixtures thereof. Preference is given to carbonates and particularly propylene carbonate.

Examples of suitable liquid crystals that can be used as host medium are nematic or chiral nematic media.

Examples of suitable polymers that can be used as host medium are polymers which are soluble with the solvent, in particular PMMA or other acrylate polymers, polyurethane, polyethylene oxide, polypropylene oxide, polyvinyl acetate, poly(N-vinyl pyrrolidone), and polyvinylidene fluoride.

Examples of suitable liquid crystal polymers that may be used as host medium are Merck RM257 (Merck), LC242 (BASF) or SLM 90519 (Wacker). These liquid crystal polymers are generally used in combination with an organic solvent, for example one of the organic solvents mentioned above.

## Electrochromic device

The present invention also relates to an electrochromic device as defined in the appended claims.

The electrochromic device according to the invention comprises a compound of formula (I) or a composition according to the appended claims. Said d electrochromic evice according to the present invention may be selected from an optical article, preferably an optical lens, or an optical filter, a window, preferably an aircraft window, a visor, a mirror and a display, in particular a segmented or matrix display. Preferably, the device of the invention is an optical article, more preferably an optical lens, and even more preferably an ophthalmic lens.

Examples of ophthalmic lens include corrective and non-corrective lenses, including single vision or multi-vision lenses, which may be either segmented or non-segmented, as well as other elements used to correct, protect, or enhance vision, including without limitation contact lenses, intra-ocular lenses, sunglasses, ski goggles, magnifying lenses, protective lenses and visors, for example motorcycle visors and helmets. Non-limiting examples of display elements and devices include screens and monitors. Non-limiting examples of windows include automotive, marine and aircraft windows, filters, shutters, and optical switches.

Preferably, the electrochromic device of the invention comprises a mechanism for holding the compound or composition of the invention in a mechanically stable environment. More preferably, said electrochromic device according to the invention may comprise a pair of opposed substrates having a gap there between for receiving the mixture of the host medium and said electrochromic compound or said electrochromic composition of the present invention, according to the appended claims, and a frame for holding said pair of substrates adjacent one another.

The electrochromic device of the present invention may thus comprise an optical component provided with at least one transparent cell arrangement juxtaposed in a parallel direction to the surface thereof, as disclosed in WO 2006/013250, each cell being tightly closed and containing at least one compound or composition of the present invention, according to the appended claims.

Other electrochromic devices according to the invention can be electrochromic devices as described in FR 2937154 or FR2950710 comprising at least one compound or composition of the invention, according to the appended claims.

## EXAMPLES

This invention will be further illustrated by the following examples which are given for illustrative purposes of the appended claims.

**Synthesis of compounds of the invention**

**1. Iso)thiazole Benzologues**

**EXAMPLE 1**

**2-(Pyridin-4-yl)benzothiazole**

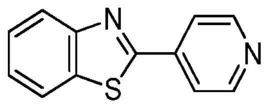

A solution of 2-aminobenzenethiol (7.01 g, 56.1 mmol) and pyridine-4-carboxaldehyde (6.00 g, 56.1 mmol) in EtOH (28 mL) was stirred under air for 120 h. The resulting mixture was filtered, and the residue washed with cooled MeOH (20 mL) and air dried to give the *title compound* (7.63 g, 64 %) as a cream powder. The liquors were reduced in volume and filtered to give a second crop (0.73 g, 6 %), $\delta_H$ ($CDCl_3$, 400 MHz) 7.46 (1H, dt, *J* = 1.2 and 8.2 Hz), 7.56 (1H, dt, *J* = 1.2 and 8.2 Hz), 7.93 - 7.99 (3H, m), 8.14 (1H, ddd, *J* = 8.2 Hz) and 8.78 (2H, dd, 1.6 and *J* = 4.5 Hz); $\delta_C$ ($CDCl_3$, 100 MHz) 121.21, 121.89, 123.93, 126.22, 126.84, 135.21, 140.47, 150.79, 153.97 and 165.11.

**4-(Benzothiazol-2-yl)-1-hexylpyridin-1-ium iodide**

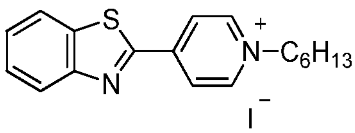

A solution of 2-(pyridin-4-yl)benzothiazole (0.54 g, 2.5 mmol) and 1-iodohexane (1.62 g, 7.6 mmol) in MeCN (40 mL) in the dark under $N_2$ was heated at reflux for 24 h, then cooled and diluted with $Et_2O$ (50 mL). The precipitate was filtered off, washed with $Et_2O$ (3 $\times$ 20 mL) and air dried to give the *title compound* (0.77 g, 71 %) as a yellow powder, $\delta_H$ (DMSO-$d_6$, 400 MHz) 0.85 (3H, t, *J* = 7.0 Hz), 1.21 - 1.37 (6H, m), 1.88 - 2.01 (2H, m), 4.65 (2H, t, *J* = 7.3 Hz), 7.62 - 7.72 (2H, m), 8.25 (1H, dd, *J* = 0.9 and 7.6 Hz), 8.35 (1H, dd, *J* = 1.1 and 7.6 Hz), 8.76 (2H, d, 6.8 Hz) and 9.23 (2H, d, *J* = 6.8 Hz); $\delta_C$ (DMSO-$d_6$, 100 MHz) 14.32, 22.34, 25.54, 31.06, 31.19, 61.11, 123.72, 124.82, 125.47, 128.23, 128.33, 136.64, 146.29, 146.73, 153.83 and 161.90.

**4-(Benzothiazol-2-yl)-1-hexylpyridin-1-ium tetrafluoroborate**

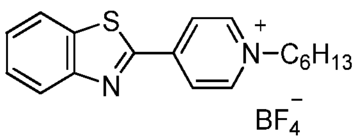

A solution of 4-(benzothiazol-2-yl)-1-hexylpyridin-1-ium iodide (0.77 g, 1.8 mol) in MeOH/$H_2O$ (40 mL, 1:1) was added dropwise to a solution of $NaBF_4$ (3.96 g, 36 mmol) in water (80 mL) with stirring. Stirring was continued for 0.5 h and the resulting precipitate filtered, washed with water (2 $\times$ 5 mL) and air dried to give the *title compound* (0.69 g, 98 %) as a pale yellow powder, $\delta_H$ (DMSO-$d_6$, 400 MHz) 0.88 (3H, t, *J* = 6.9 Hz), 1.25 - 1.39 (6H, m), 1.89 - 2.02 (2H, m), 4.67 (2H, t, *J* = 7.4 Hz), 7.64 - 7.75 (2H, m), 8.28 (1H, dd, *J* = 1 and 8.3 Hz), 8.37 (1H, dd, *J* = 1 and 7.6 Hz), 8.79(2H, d, 6.8 Hz) and 9.23 (2H, d, *J* = 6.8 Hz); $\delta_C$ (DMSO-*$d_6$*,100 MHz) 13.75, 21.78, 24.98, 30.50, 30.63, 60.57, 123.14, 124.26, 124.90, 127.67, 127.77, 136.09, 145.72, 146.19, 153.27 and 161.33.

**Compound 1: 2-(1-Hexylpyridin-1-ium-4-yl)-3-methylbenzothiazol-3-ium bis(tetrafluoroborate)**

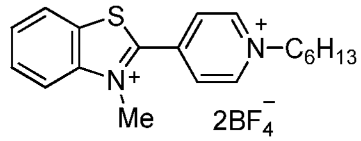

A mixture of 4-(benzothiazol-2-yl)-1-hexylpyridin-1-ium tetrafluoroborate (0.59 g, 1.5 mmol) in MeOTs (1.71 g) was heated at 180 °C for 2 h, cooled, triturated with $Et_2O$ (3 × 40 mL) and dried under $N_2$ to give 0.81 g of a light tan powder. The solid was redissolved in MeOTs (1.71 g), heated at 180°C for 1 h, cooled, triturated again with $Et_2O$ (4 × 30 mL) and air dried. The gummy solid was dissolved in MeOH/water (25 mL, 1:4) and added dropwise to a solution of $NaBF_4$ (3.38 g, 30.7 mmol) in water (50 mL) with stirring. Stirring was continued for 0.5 h and the resulting precipitate filtered, washed with water (2 × 5 mL), air dried and triturated with hot MeOH (8 mL). The residue was cooled, filtered and washed with cold MeOH (2 mL) to give the *title compound* (0.38 g, 51 %) as a cream powder, $\delta_H$ [$(CD_3)_2CO$, 400 MHz] 0.91 (3H, t, *J* = 7.0 Hz), 1.29 - 1.47 (4H, m), 1.49 - 1.61 (2H, m), 2.20 - 2.32 (2H, m), 4.58 (3H, s), 5.04 (2H, t, *J* = 7.7 Hz), 8.07 (1H, t, *J* = 7.9 Hz), 8.17 (1H, t, *J* = 7.8 Hz), 8.58 (1H, d, *J* = 8.4 Hz), 8.68 (1H, d, *J* = 8.4 Hz), 8.89 (2H, bd, *J* = 5.7 Hz) and 9.59 (2H, d, *J* = 6.5 Hz); $\delta_F$ [$(CD_3)_2CO$, 376 MHz] -151.40, -151.35; $\delta_C$ [$(CD_3)_2CO$, 100 MHz] 13.30, 22.14, 25.54, 30.98, 31.37, 38.42, 62.91, 117.97, 124.70, 129.92, 130.08, 131.05, 131.42, 141.05, 142.81, 146.47 and 168.29.

**EXAMPLE 2**

**4-(Benzothiazol-2-yl)-1-phenylpyridin-1-ium triflate**

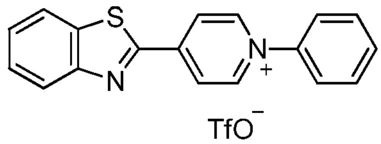

A mixture of 2-(pyridin-4-yl)benzothiazole (1.34 g, 6.3 mmol), diphenyliodonium triflate (3.47 g, 8.1 mmol), $Cu(OAc)_2.H_2O$ (0.11 g, 0.55 mmol, 10 mol%) in dry DMF (25 mL) was heated at 100°C for 16 h, cooled and the solvent removed under reduced pressure. The residue was triturated with $Et_2O$ (3 × 40 mL) and air dried to give the *title compound* (2.66 g, 96 %) as a yellow powder, $\delta_H$ [$(CD_3)_2CO$,400 MHz] 7.67 - 7.86 (5H, m), 8.01- 8.09 (2H, m), 8.27 - 8.37 (2H, m), 9.02 (2H, d, 6.8 Hz) and 9.56 (2H, d, *J* = 6.8 Hz); $\delta_F$ [$(CD_3)_2CO$, 376 MHz] -77.76; $\delta_C$ (DMSO-$d_6$, 100 MHz) 122.93, 124.60, 124.85, 125.25, 128.01, 128.18, 130.60, 131.78, 136.92, 142.95, 142.95, 145.92, 148.33, 154.18 and 160.76.

**Compound 2: 3-Methyl-2-(1-phenylpyridin-1-ium-4-yl)benzothiazol-3-ium bis(tetrafluoroborate)**

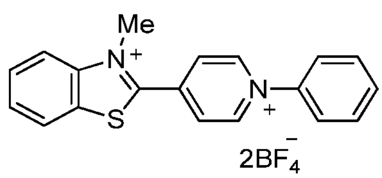

A mixture of 4-(benzothiazol-2-yl)-1-phenylpyridin-1-ium triflate (1.07 g, 2.4 mmol) MeOTs (3.62 g, 19 mmol) was heated at 180 °C for 3 h, cooled and triturated with $Et_2O$ (3 × 40 mL) and the residue dried under vacuum. The residue was dissolved in MeOH (5 mL) and added dropwise to a solution of $NaBF_4$ (5.37 g, 48.8 mmol) in water (40 mL) with stirring. Stirring was continued for 0.5 h and the resulting precipitate was filtered, washed with water (2 × 5 mL)

and air dried. The residue was triturated with hot MeOH (2 × 20 mL), filtered and air dried to give the *title compound* (0.72 g, 62 %) as a grey powder, $\delta_H$ (DMSO-*d6*, 400 MHz) 4.32 (3H, s), 7.80 - 7.90 (3H, m), 7.92 - 7.99 (2H, m), 8.04 (1H, t, *J* = 7.8 Hz), 8.14 (1H, t, *J* = 7.5 Hz), 8.55 (1H, d, *J* = 8.6 Hz), 8.69 (1H, d, *J* = 8.2), 8.79 (2H, d, *J* = 6.7 Hz) and 9.77 (2H, d, *J* = 6.7 Hz); $\delta_F$ (DMSO-$d_6$, 376 MHz) -148.23, -148.18; $\delta_C$ (DMSO-$d_6$, 100 MHz) 118.42, 125.21, 125.57, 129.92, 130.10, 130.99, 131.21, 131.61, 132.40, 141.77, 142.47, 142.92, 146.55 and 168.49.

## EXAMPLE 3

### (E)-3,4-Dihydronaphthalen-1(2H)-one O-acetyl oxime

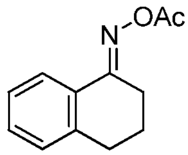

Solid hydroxylamine hydrochloride (7.14 g, 103 mmol) was added in one portion to a solution of 3,4-dihydronaphthalen-1(2*H*)-one (10.00 g, 68.5 mmol) in EtOH (30 mL). The resulting solution was heated at 60 °C for 1 h, poured into HCl (200 mL, 2 M) and extracted with EtOAc (3 × 50 mL). The combined organic extracts were washed with brine (50 mL), then water (50 mL) and dried ($Na_2SO_4$) and the solvent removed under reduced pressure. The residue was dissolved in pyridine (32 mL) to which $Ac_2O$ (13.97 g, 137 mmol) and DMAP (16 mg) were then added. The resulting solution was stirred at rt for 1 h, poured into HCl (300 mL, 2 M) and extracted with EtOAc (3 × 200 mL). After drying ($Na_2SO_4$) the solvent was removed under reduced pressure. The residue was twice crystallized from EtOAc/hexanes to give the *title compound* (10.51 g, 76 %) as colourless needles, $\delta_H$ ($CDCl_3$, 400 MHz) 1.87 - 1.93 (2H, m), 2.27 (3H, s), 2.77 - 2.82 (2H, br. t, *J* = 6.1 Hz), 2.86 - 2.90 (2H, m), 7.16 - 7.20 (1H, m), 7.22 - 7.26 (1H, m), 7.34 (1H, dt, *J* = 1.4 and 7.4 Hz) and 8.13 - 8.16 (1H, m); $\delta_C$ ($CDCl_3$, 100 MHz) 19.92, 21.33, 25.60, 29.55, 125.61, 126.59, 128.72, 128.95, 130.74, 140.93, 161.31 and 169.22.

### 2-(Pyridin-4-yl)naphtho[1,2-*d*]thiazole

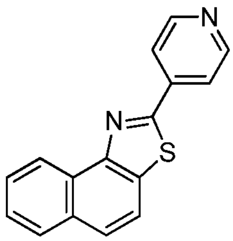

A mixture of (*E*)-3,4-dihydronaphthalen-1(2*H*)-one *O*-acetyl oxime (4.00 g, 19.7 mmol), pyridine-4-carboxaldehyde (1.40 g, 13.1 mmol) and sulfur (10.09 g, 39.4 mmol) in DMSO (60 mL) under $N_2$ was heated at 120 °C for 4 h, poured into water (200 mL) and extracted with EtOAc (3 × 100 mL). The combined extracts were washed with water (100 mL), dried ($Na_2SO_4$) and the solvent removed under reduced pressure. The residue was chromatographed on silica using EtOAc (25 - 30 % in hexanes gradient) as eluent. The fluorescent band was collected and the solvent removed under reduced pressure. The residue was crystallised from hot EtOAc/hexanes at 0 °C to give the *title compound* (1.14 g, 33 %) as brown needles, $\delta_H$ ($CDCl_3$, 600 MHz) 7.64 (1H, ddd, *J* = 1.3, 6.9 and 8.1 Hz), 7.74 (1H, ddd, *J* = 1.2, 6.9 and 8.2 Hz), 7.87 - 7.90 (1H, br. dm, *J* = 8.7 Hz), 7.95 (1H, d, *J* = 8.7 Hz), 7.98 - 8.01 (1H, br. m, *J* = 8.1 Hz), 8.06 (2H, d, *J* = 6.0 Hz), 7.80 (2H, d, *J* = 6.0 Hz) and 8.93 (1H, br. d, *J* = 8.2 Hz); $\delta_C$ ($CDCl_3$, 125 MHz) 118.90, 120.98, 123.96, 126.61, 127.17, 127.40, 128.22, 128.92, 132.16, 132.33, 140.77, 150.56, 150.78 and 163.88.

### 4-(Naphtho[1,2-*d*]thiazol-2-yl)-1-phenylpyridin-1-ium trifluoromethanesulfonate

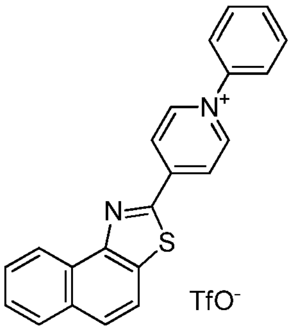

TfO⁻

A mixture of 2-(pyridin-4-yl)naphtho[1,2-*d*]thiazole (1.12 g, 4.3 mmol), diphenyliodonium trifluoromethanesulfonate (2.20 g, 5.1 mmol) and $Cu(OAc)_2.H_2O$ (86 mg, 10 mol %) in DMF (30 mL) under $N_2$ was heated at 100 °C for 16 h. The resulting solution was cooled and the solvent removed under reduced pressure. The residue was triturated with $Et_2O$ (3 × 30 mL) and air dried to give the *title compound* (2.09 g, 100 %) as a brown powder which was used without further purification in the next step, $\delta_H$ (DMSO-$d_6$, 400 MHz) 7.71 - 7.91 (5H, m), 7.93 - 8.01 (2H, m), 8.16 - 8.23 (2H, m), 8.40 (1H, d, *J* = 8.9 Hz), 8.92 (1H, d, *J* = 8.1 Hz), 8.96 (2H, d, *J* = 7.0 Hz) and 9.49 (2H, d, *J* = 7.0 Hz); $\delta_F$ (DMSO-$d_6$, 376 MHz) -77.75.

**Compound 3: 1-Methyl-2-(1-phenylpyridin-1-ium-4-yl)naphtho[1,2-*d*]thiazol-1-ium bis(tetrafluoroborate)**

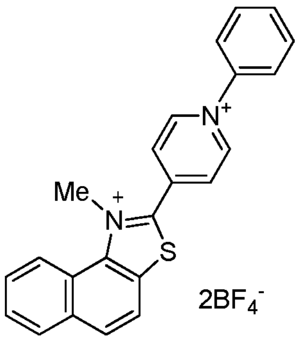

$2BF_4^-$

A solution of 4-(naphtho[1,2-*d*]thiazol-2-yl)-1-phenylpyridin-1-ium trifluoromethanesulfonate (2.09 g, 4.3 mmol) in methyl trifluoromethanesulfonate (9.03 g, 55.1 mmol) was heated at reflux. After 2 days the solution was diluted with $Et_2O$ (40 mL) and the residue collected by filtration. The filtrand was washed with $Et_2O$ (2 × 10 mL) and air dried. The solid was extracted with water (2 × 100 mL) and the solvent removed under reduced pressure. The residue was then dissolved in MeOH (5 mL) and added dropwise to a solution of $NaBF_4$ (5.05 g, 45.9 mmol) in water (40 mL) with stirring. Stirring was continued for 0.5 h and the resulting precipitate filtered, washed with water (3 × 5 mL) and triturated with hot MeOH (20 mL). After cooling, the residue was filtered off and air dried to give the *title compound* (0.23 g, 8 %) as a pale yellow solid. $\delta_H$ (DMSO-$d_6$, 400 MHz) 4.74 (3H, s), 7.76 - 8.12 (7H, m), 8.40 - 8.70 (3H, br. m), 8.82 (2H, br. d, *J* = 3.3 Hz), 9.11 (1H, d. dm, *J* = 9.0 Hz) and 9.79 (2H, br. d, *J* = 3.3 Hz); $\delta_F$ (DMSO-$d_6$, 376 MHz) -148.25 and -148.20.

**EXAMPLE 4**

**Quinoline-4-carbaldehyde**

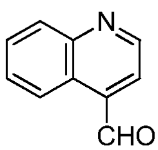

A mixture of 4-methylquinoline (6.00 g, 41.9 mmol), *p*-toluenesulfonic acid (7.22 g, 42 mmol) and iodine (4.26 g, 16.8 mmol, 40 mol%) in DMSO (300 mL) was heated at 130 °C for 16 h, cooled, poured into water (400 mL) and basified ($K_2CO_3$). The mixture was extracted with EtOAc (5 × 100 mL). The combined organic extracts were washed with aq. $Na_2S_2O_3$ (300 mL), then brine (200 mL) and dried ($Na_2SO_4$). The solvent was removed under reduced pressure to give the *title compound* (6.11 g, 93 %) as a beige solid, $\delta_H$ ($CDCl_3$, 400 MHz) 7.75 (1H, ddd, *J* = 1.4, 6.9 and 8.4 Hz), 7.80 (1H, d, *J* = 4.3 Hz), 7.83 (1H, d, *J* = 1.4, 6.9 and 8.4), 8.23 (1H, ddd, *J* = 0.7, 1.4 and 8.4 Hz), 9.11 (1H, ddd, *J* = 0.7, 1.4 and 8.4 Hz), 9.21 (1H, d, *J* = 4.3 Hz) and 10.53 (1H, s); $\delta_C$ ($CDCl_3$, 100 MHz) 123.89, 124.44, 125.87, 129.42, 130.06, 130.22, 136.76, 149.28, 150.48 and 192.92.

**2-(Quinolin-4-yl)-2,3-dihydrobenzothiazole**

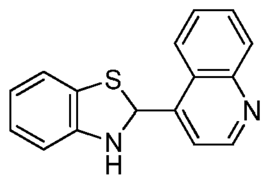

A solution of quinoline-4-carbaldehyde (3.00 g, 19.1 mmol) and 2-aminobenzenethiol (2.39 g, 19.1 mmol) in EtOH (40 mL) was stirred under air for 2 days. The solvent was decanted and the residue crystallised from hot EtOH (40 mL) and cooled to 0 °C, The product was collected by filtration, washed with EtOH (10 mL) and air dried to give the *title compound* (2.38 g, 47 %) as orange prisms. The filtrate liquors were reduced to give a second crop (1.19 g, 23 %) as a yellow powder, $\delta_H$ (DMSO-$d_6$, 400 MHz) 6.62 (1H, dt, *J* = 1.2 and 7.5 Hz), 6.78 (1H, br. dd, *J* = 1.1 and 7.8 Hz), 6.94 (1H, dt, *J* = 1,3 and 7.7 Hz), 6.99 (1H, dd, *J* = 1.2 and 7.4 Hz), 7.15 (2H, s), 7.62 (1H, d, *J* = 4.5 Hz), 7.67 (1H, ddd, *J* = 1.4, 6.9 and 8.4 Hz) 7.79 (1H, ddd, *J* = 1.4, 6.9, 8.4 Hz) 8.07 (2H, br. t, *J* = 8.8 Hz) and 8.88 (1H, d, *J* = 4.4 Hz); $\delta_C$ (DMSO-$d_6$, 100 MHz) 64.46, 109.88, 117.60, 119.59, 121.94, 123.84, 124.74, 125.14, 126.21, 127.39, 129.94, 130.26, 148.02, 148.16, 148.40 and 151.08.

**2-(Quinolin-4-yl)benzothiazole**

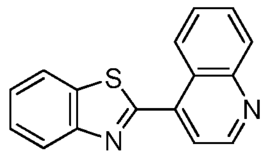

Solid DDQ (1.29 g, 5.7 mmol) was added to a solution of 2-(quinolin-4-yl)-2,3-dihydrobenzothiazole (1.50 g, 5.7 mmol) in DCM (1.2 L). The mixture was stirred at rt for 1 h, then filtered through silica using EtOAc (60 % in hexanes) as eluent. The solvent was removed under reduced pressure and the residue dissolved in EtOAc (200 mL) and washed sequentially with KOH (200 mL, 2 M), water (2 × 100 mL) and brine (100 mL). The extracts were dried ($Na_2SO_4$) and the solvent removed under reduced pressure. The residue was crystallised from hot EtOAc/hexanes. After cooling to -4 °C, the product was collected by vacuum filtration and washed with cold hexanes to give the *title compound* (1.19 g, 80 %) as pale lime needles, $\delta_H$ ($CDCl_3$, 400 MHz) 7.52 (1H, ddd, *J* = 1.3, 7.3 and 7.9 Hz), 7.60 (1H, ddd, *J* = 1.3, 7.2 and 8.2 Hz), 7.70 (1H, ddd, *J* = 1.3, 6.8 and 8.7 Hz), 7.80 (1H, d, *J* = 4.4 Hz), 7.82 (1H, ddd, *J* = 1.4, 6.9 and 8.4 Hz), 8.02 (1H, ddd, *J* = 0.7, 1.3 and 8.0 Hz), 8.22 (1H, ddd, *J* = 0.7, 1.4 and 8.4 Hz) 8.24 (1H, ddd, *J* = 0.6, 1.2 and 8.2 Hz), 9.00 (1H, ddd, *J* = 0.7, 1.4 and 8.5 Hz) and 9.05 (1H, d, *J* = 4.4 Hz); $\delta_C$ ($CDCl_3$, 100 MHz) 121.63, 122.19, 124.12, 124.98, 126.04, 126.17, 126.76, 128.19, 130.00 (2 × C), 135.34, 138.32, 149.20, 149.80, 154.18, 164.80.

**4-(Benzothiazol-2-yl)-1-phenylquinolin-1-ium tetrafluoroborate**

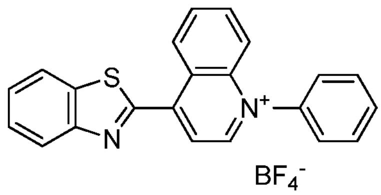

A mixture of 2-(quinolin-4-yl)benzothiazole (1.00 g, 3.8 mmol), diphenyliodonium trifluoromethanesulfonate (2.46 g, 5.7 mmol) and $Cu(OAc)_2.H_2O$ (76 mg, 10 mol %) in DMF (30 mL) under $N_2$ was heated at 100 °C for 16 h. The resulting solution was cooled and the solvent removed under reduced pressure, the residue was triturated with $Et_2O$ (3 × 40 mL) to give a mixture (62:38) of product and starting material. The residue and diphenyliodonium trifluoromethanesulfonate (1.64 g, 3.8 mmol) and $Cu(OAc)_2.H_2O$ (76 mg, 10 mol %) in DMF (30 ml) under $N_2$ was heated at 100 °C for 16 h. The resulting solution was cooled and the solvent removed under reduced pressure. The residue was triturated with $Et_2O$ (3 × 40 mL) to give a mixture of product and reactant (70:30). This solid was dissolved in warm MeOH (20 mL) and added dropwise to a solution of $NaBF_4$ (8.40 g, 76 mmol) in water (50 mL) with stirring. Stirring was continued for 0.5 h and the resulting precipitate collected by filtration and washed with water (5 mL). The solid was dissolved in hot MeOH (20 mL) and added dropwise to $NaBF_4$ (8.40 g, 76 mmol) in water (50 mL) with stirring. Stirring was continued for 0.5 h and the resulting precipitate filtered off, washed with water (5 mL) and air dried to give a yellow powder. The powder was triturated with hot EtOH (2 × 5 mL) to give the *title compound* (1.01 g, 54 %) as a yellow powder, $\delta_H$ ($CDCl_3$, 400 MHz) 7.67 - 7.79 (2H, m), 7.79 - 8.92 (6H, m), 8.12 - 8.24 (2H, m), 8.27 (1H, br. d, *J* = 7.6 Hz), 8.38 (1H, br. d, *J* = 7.9 Hz), 8.68 (1H, d, *J* = 6.2 Hz), 9.52 (1H, d, *J* = 6.2 Hz) and 9.69 (1H, br. dd, *J* = 1.5 and 8.3 Hz); $\delta_F$ ($CDCl_3$, 376 MHz) -154.67 and -154.62; $\delta_C$ ($CDCl_3$, 100 MHz) 120.31, 122.03, 122.56, 124.59, 126.34, 126.89, 127.61, 127.76, 128.91, 130.47, 131.13, 131.79, 135.76, 136.23, 140.35, 140.92, 148.20, 149.02, 154.37 and 161.44.

**Compound 4: 3-Methyl-2-(1-phenylquinolin-1-ium-4-yl)benzothiazol-3-ium tetrafluoroborate**

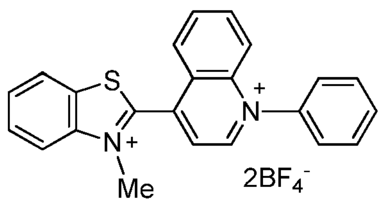

A mixture of 4-(benzothiazol-2-yl)-1-phenylquinolin-1-ium tetrafluoroborate (1.01 g, 2.4 mmol) in MeOTs (4.61 g, 24.8 mmol) was heated at 180 °C for 3 h, then cooled and triturated with $Et_2O$ (3 × 30 mL). The washings were discarded and the residue then dried under $N_2$. The residue was dissolved in hot MeOH (40 mL) and added dropwise to a solution of $NaBF_4$ (4.55 g, 41.4 mmol) in water (40 mL) with stirring. Stirring was continued for 0.5 h and the precipitate collected by filtration and washed with water (2 × 5 mL), and then with hot MeOH (5 mL) and air dried. The residue was triturated with AcMe (3 mL) to give the *title compound* (0.70 g, 56 %) as an off white powder, $\delta_H$ [$(CD_3)_2CO$, 600 MHz] 4.52 (3H, s), 7.88 - 8.02 (5H, m), 8.07 (1H, app. br. d, *J* = 9.0 Hz), 8.12 (1H, ddd, *J* = 0.7, 7.3 and 8.7 Hz), 8.19 - 8.24 (2H, m), 8.39 (1H, ddd, *J* = 1.3, 7.0 and 9.0 Hz), 8.57 (1H, ddd, *J* = 0.6, 1.3 and 8.5 Hz), 8.64 (1H, ddd, *J* = 0.7, 1.6, 7.9 Hz), 8.75 (1H, ddd, *J* = 0.6, 1.2 and 7.3 Hz), 9.00 (1H, d, *J* = 5.9 Hz) and 9.97 (1H, d, *J* = 5.9 Hz); $\delta_F$ [$(CD_3)_2CO$, 470 MHz] -151.44 and -151.39; $\delta_C$ [$(CD_3)_2CO$, 125 MHz] 38.76, 118.09, 121.40, 124.76, 125.84, 126.59, 127.07, 128.87, 130.00, 130.73, 131.06, 132.25, 132.32, 132.52, 137.16, 140.39, 140.69, 141.56, 142.98, 150.58 and 166.95.

**EXAMPLE 5**

**3-(Pyridin-4-yl)-1,2-benzisothiazole**

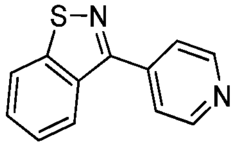

$^nBuLi$ (2.5 M in hexane) (32.2. mL, 80.5 mmol) was added dropwise to a stirred solution of thioanisole (2.00 g, 16.1 mmol) and *N,N,N',N'*-tetramethylethylenediamine (5.60 g, 7.24 mL, 48.3 mmol) in *tert*-butyl methyl ether (80 mL) under $N_2$. The reaction mixture was then stirred under $N_2$ at room temperature for 3 h. 4-Pyridinecarbonitrile (16.76 g, 161 mmol) was then added portion wise and the reaction mixture was then stirred under $N_2$ at room temperature for 24 h. The reaction mixture was then carefully quenched with sat. $NH_4Cl$ (100 mL) and extracted with DCM (3 × 200 mL). The organic layers were combined, dried ($Na_2SO_4$), filtered and the solvent was removed under reduced pressure. The resulting residue was chromatographed on silica gel [eluent = 1:9 graduated to 4:6 EtOAc:DCM]. The solvent of the resulting column fractions was removed under reduced pressure and the resulting residue was again chromatographed on silica gel [eluent = 1:9 graduated to 4:6 EtOAc:DCM]. The solvent of the resulting column fractions was removed under reduced pressure and the resulting residue was recrystallised from hot petroleum ether and filtered to give the *title compound* (0.33 g, 15 %) as colourless crystals. The filtrate from the recrystallisation was left to stand at -20°C for 16 h whereupon a second crop was obtained (0.15 g, 7%) as colourless crystals. Yield 0.48 g, 22 %. $\delta_H$ ($CDCl_3$, 400 MHz) 8.81 (2H, d, *J* = 5.3 Hz), 8.18 (1H, d , *J* = 8.2 Hz), 8.02 (1H, d, *J* = 8.2 Hz), 7.79 (2H, d, *J* = 5.9 Hz), 7.59 (1H, t, *J* = 7.3 Hz) and 7.50 (1H, t, *J* = 7.3 Hz); $\delta_C$ ($CDCl_3$, 100 MHz) 161.4, 153.9, 150.5, 142.2, 133.36, 127.9, 125.6, 124.1, 123.0 and 120.2.

**Compound 5: 4-(1,2-Benzisothiazol-3-yl)-1-phenylpyridin-1-ium tetrafluoroborate**

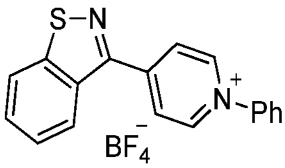

3-(Pyridin-4-yl)-1,2-benzisothiazole (0.36 g, 1.7 mmol), diphenyliodonium trifluoromethanesulfonate (1.12 g, 2.6 mmol) and $Cu(OAc)_2 \cdot H_2O$ (34 mg, 0.17 mmol) were dissolved in DMF (20 mL) under $N_2$ and the reaction mixture was stirred at 100°C for 16 h. The reaction mixture was cooled to room temperature and the solvent was removed under reduced pressure. The resulting pale yellow solid was triturated with diethyl ether (3 × 50 mL), filtered and dried under reduced pressure. The resulting pale yellow solid was dissolved in hot MeOH (150 mL) and added dropwise through a cotton wool plug to a stirred solution of $NaBF_4$ (15.0 g) in $H_2O$ (300 mL) whereupon a pale yellow precipitate formed. The suspension was stirred for 30 minutes and then filtered under reduced pressure. The resulting solid was washed with water (30 mL) and then dried under reduced pressure to give a pale yellow powder. The solid was then dissolved in hot acetone and added dropwise through a cotton wool plug back into the filtrate (which had been reduced by ca. 30% under vacuum) to give a pale yellow precipitate. The resulting suspension was filtered under reduced pressure and the solid so obtained, washed with water (30 mL) and then dried under reduced pressure to give the *title compound* as a pale yellow powder. Yield 0.38 g, 59 %. $\delta_H$ (DMSO-$d_6$, 400 MHz) 9.55 (2H, d, *J* = 6.9 Hz), 8.84 (2H, d, *J* = 6.9 Hz), 8.52 (2H, d, *J* = 8.9 Hz), 8.07 - 7.95 (2H, m) and 7.92 - 7.71 (5H, m); $\delta_C$ (DMSO-$d_6$, 100 MHz) 157.4, 154.0, 148.8, 145.6, 142.5, 132.8, 131.4, 130.3, 128.6, 126.8, 126.6, 124.8, 123.9 and 121.5; $\delta_F$ (DMSO-$d_6$, 376 MHz) -148.22 (4F, br. m).

**EXAMPLE 6**

**Compound 6: 2-Methyl-3-(1-phenylpyridin-1-ium-4-yl)-1,2-benzisothiazol-2-ium bis(hexafluorophosphate)**

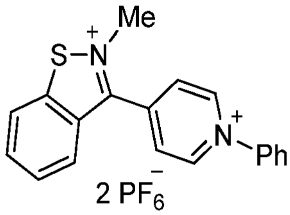

4-(1,2-Benzisothiazol-3-yl)-1-phenylpyridin-1-ium tetrafluoroborate (0.30 g, 0.8 mmol) was suspended in methyl trifluoromethanesulfonate (0.8 g, 5.4 mmol) and the reaction mixture was stirred under $N_2$ at 100°C for 80 minutes. The reaction mixture was then stirred under $N_2$ at 40°C for 4 h. After cooling to room temperature, diethyl ether (50 mL) was added and the resulting suspension was filtered to give a tan powder that was then triturated with ether (3 × 50 mL) and dried under reduced pressure. This tan powder was then dissolved in hot MeOH (50 mL) and added dropwise through a cotton wool plug to a stirred solution of $NH_4PF_6$ (10.0 g) in $H_2O$ (200 mL) whereupon a tan coloured precipitate formed. The precipitate was then washed with water (30 mL) and then dried under reduced pressure to give a pale tan powder. The pale tan powder was then recrystallised from hot MeOH to give the *title compound* as a pale tan powder Yield 0.18 g, 38 %. $\delta_H$ (DMSO-d$_6$, 400 MHz) 9.81 (2H, d, *J* = 6.9 Hz), 8.75 (2H, d, *J* = 6.9 Hz), 8.71 (1H, d, *J* = 6.6 Hz), 8.18 (1H, ddd, *J* = 8.5, 7.3, 0.9 Hz), 8.10 (1H, d, *J* = 6.6 Hz), 8.05 - 7.82 (6H, m) and 4.33 (3H, s); $\delta_C$ (DMSO-*d*$_6$, 100 MHz) 159.3, 147.8, 146.2, 142.5, 142.4, 134.5, 131.9, 130.9, 130.5, 129.3, 128.8, 127.0, 124.7, 122.9 and 39.5; $\delta_F$ (DMSO-*d*$_6$, 376 MHz) -70.14 (12F, d, *J* = 711.5 Hz).

## 2. (Benz)imidazoles and fused-ring derivatives

### EXAMPLE 7

**2-(Pyridin-4-yl)-1*H*-benzimidazole**

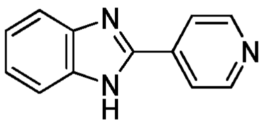

A solution of *o*-phenylenediamine (10.10 g, 93.5 mmol) and pyridine-4-carboxaldehyde (10.00 g, 93.5 mmol) in EtOH (500 mL) was stirred under air for 72 h and the solvent removed under reduced pressure. The residue was crystallised from EtOAc/hexanes then triturated with hot EtOAc three times to give the *title compound* (11.44 g, 63 %) as a tan powder, $\delta_H$ (DMSO-*d*$_6$, 400 MHz) 7.26 (2H, bs), 7.59 (1H, vbs), 7.71 (1H, vbs), 8.80 (2H, d, *J* = 6.1 Hz), 8.75 (2H, d, *J* = 6.1 Hz) and 13.26 (1H, bs).

**1-Methyl-2-(pyridin-4-yl)-1*H*-benzimidazole**

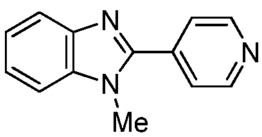

A mixture of 2-(pyridin-4-yl)-1*H*-benzimidazole (5.33g, 27.3 mmol), MeI (5.33 mL, 12.15 g, 85.6 mmol) and KOH (7.83 g, 140 mmol) in acetone (660 mL) was stirred at rt for 2 h and poured into PhMe (700 mL). The resulting solution was washed with water (1 L), brine (100 mL), dried ($Na_2SO_4$) and the solvent removed under reduced pressure. The residue was filtered through neutral alumina using EtOAc as eluent. The solvent was removed under reduced pressure and the residue crystallised from EtOAc/hexanes to give the *title compound* (2.80 g, 49 %) as yellow plates, $\delta_H$ ($CDCl_3$, 400 MHz) 3.83 (3H, s), 7.72 - 7.46 (3H, m), 7.72 (2H, d, *J* = 6.0 Hz), 7.83 - 7.87 (1H, m) and 8.06 (2H, d, *J* = 6.0 Hz); $\delta_C$ ($CDCl_3$, 100 MHz) 31.84, 109.88, 120.37, 123.03, 123.45, 123.74, 136.74, 137.86, 142.91, 150.38 and 150.76.

**1-Hexyl-4-(1-methyl-1*H*-benzimidazol-2-yl)pyridin-1-ium tetrafluoroborate**

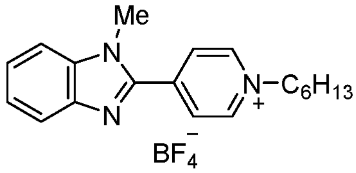

A solution of 1-methyl-2-(pyridin-4-yl)-1*H*-benzimidazole (1.47 g, 7 mmol) and 1-iodohexane (4.47 g, 21.1 mmol) in MeCN (40 mL) was heated at reflux in the dark under $N_2$ with stirring. After 24 h, the resulting mixture was cooled, diluted with $Et_2O$ (60 mL), filtered, washed with $Et_2O$ (30 mL) and dried under vacuum. The hygroscopic orange powder was dissolved in MeOH (30 mL) and added dropwise to a solution of $NaBF_4$ (4.55 g, 41.4 mmol) in water (200 mL) with stirring. Stirring was continued for 0.5 h and the resulting precipitate was filtered, washed with water (3 × 10 mL) and air dried to give the *title compound* (1.91 g, 73 %) as a pale yellow powder, $\delta_H$ [$(CD_3)_2CO$, 400 MHz] 0.88 (3H, t, *J* = 7.3 Hz), 1.27 - 1.56 (6H, m), 2.15 - 2.27 (2H, m), 4.23 (3H, s), 4.93 (2H, t, *J* = 7.7 Hz), 7.39 (1H, ddd, *J* = 1.1, 7.1 and 8.2 Hz), 7.48 (1H, ddd, *J* = 1.2, 7.1 and 8.2 Hz), 7.70 - 7.74 (1H, m), 7.80 - 7.84 (1H, m), 8.80 (2H, d, *J* = 6.9 Hz) and 9.34 (2H, d, *J* = 6.9 Hz Hz); $\delta_F$ [$(CD_3)_2CO$, 376 MHz] -151.70 and -151.54; $\delta_C$ [$(CD_3)_2CO$, 100 MHz] 13.31, 22.17, 25.54, 31.00, 31.24, 32.08, 61.60, 111.15, 120.61, 123.44, 125.00, 127.04, 137.95, 143.21, 145.02, 145.87 and 147.05.

**<u>Compound 7:</u> 2-(1-Hexylpyridin-1-ium-4-yl)-1,3-dimethyl-1*H*-benzimidazol-3-ium bis(tetrafluoroborate)**

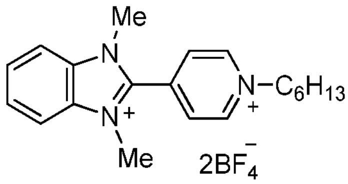

A mixture of 1-hexyl-4-(1-methyl-1*H*-benzimidazol-2-yl)pyridin-1-ium tetrafluoroborate (0.50 g, 1.3 mmol) in MeOTs (4.00 g, 21.5 mmol) was heated at 180 °C for 90 min, cooled and then triturated with $Et_2O$ (3 × 30 mL). The residue was dissolved in MeOH (20 mL) and added drop wise to a solution of $NaBF_4$ (20.97 g, 190 mmol) in water (100 mL) with stirring. Stirring was continued for 0.5 h and the resulting precipitate filtered, washed with water (2 × 3 mL) and air dried to give 2-(1-hexylpyridin-1-ium-4-yl)-1,3-dimethyl-1*H*-benzimidazol-3-ium bis(tetrafluoroborate) (0.45 g, 71 %) as a grey powder, $\delta_H$ ($CD_3OD$, 400 MHz) 0.91 - 0.99 (3H, m), 1.35 - 1.59 (6H, m), 2.10- 2.22 (2H, m), 4.07 (6H, s), 4.81 (2H, t, *J* = 7.6 Hz), 7.81 - 7.88 (2H, m), 8.05- 8.12 (2H, m), 8.61 (2H, d, *J* = 5.4 Hz) and 9.43 (2H, d, *J* = 5.4 Hz); $\delta_F$ (DMSO-$d_6$, 376 MHz) -148.25; $\delta_C$ (DMSO-$d_6$, 100 MHz) 14.34, 22.34, 25.60, 31.14, 31.37, 33.44, 62.15, 114.19, 128.06, 130.84, 132.49, 136.91, 145.57 and 146.60.

**EXAMPLE 8**

**<u>Compound 8:</u> 2-(1-hexylpyridin-1-ium-4-yl)-1,3-dimethyl-1*H*-benzimidazol-3-ium bis(hexafluorophosphate)**

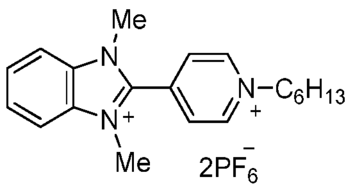

Solid ammonium hexafluorophosphate (1.16 g, 7.1 mmol) was added to the aqueous filtrate and washings obtained during the preparation of compound 3 above and the resulting precipitate filtered, washed with water (2 × 2

mL), triturated with hot MeOH (2 mL) and air dried to give 2-(1-hexylpyridin-1-ium-4-yl)-1,3-dimethyl-1*H*-benzimidazol-3-ium bis(hexafluorophosphate) (0.21 g, 27 %) as a cream powder; $\delta_H$ (DMSO-$d_6$, 400 MHz) 0.89 (3H, t, *J* = 6.8 Hz), 1.25 - 1.47 (6H, m), 1.94 - 2.08 (2H, m), 3.97 (6H, s), 4.74 (2H, m), 7.81 - 7.88 (2H, m), 8.15 - 8.23 (2H, m), 8.63 (2H, d, *J* = 6.4 Hz) and 9.48 (2H, d, *J* = 6.4 Hz); $\delta_F$ (DMSO-$d_6$, 376 MHz) -70.13 (d, *J* = 710 Hz) ; $\delta_C$ (DMSO-$d_6$, 100 MHz) 14.34, 22.35, 25.60, 31.14, 31.40, 33.47, 62.14, 114.20, 128.09, 130.87, 132.48, 136.90, 145.57 and 146.61.

**EXAMPLE 9**

**1-Hexyl-2-(pyridin-4-yl)-1*H*-benzimidazole**

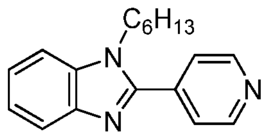

A mixture of 2-(pyridin-4-yl)-1*H*-benzimidazole (2.00 g, 10.3 mmol), powdered KOH (0.92 g, 16.4 mmol) and 1-iodohexane (2.39 g, 11.3 mmol) in anhydrous DMSO (30 mL) under $N_2$ was stirred at rt for 24 h then poured into water (100 ml) and extracted with DCM (3 × 100 mL). The combined extracts were washed with water (100 mL), dried ($Na_2SO_4$) and the solvent removed under reduced pressure. The residue was filtered through silica using EtOAc as eluent. The solvent was removed under reduced pressure and the residue triturated with hexanes (3 × 50 mL), filtered, washed with hexanes and air dried to give the *title compound* (2.53 g, 91 %) as a tan powder, $\delta_H$ ($CDCl_3$, 400 MHz) 0.77 - 0.87 (3H, m), 1.17 - 1.35 (6H, m), 1.83 - 1.96 (2H, m), 4.450 (2H, t, *J* = 7.6), 7.47 - 7.66 (3H, m), 7.90 (2H, d, *J* = 6 Hz), 7.94 (1H, d, *J* = 8 Hz) and 8.93 (2H, d, *J* = 6 Hz); $\delta_C$ ($CDCl_3$, 100 MHz) 13.85, 22.36, 26.26, 29.70, 30.99, 46.00, 111.72, 118.10, 124.02, 126.43, 126.58, 133.56, 134.98, 135.17, 147.07 and 149.00.

**Compound 9: 3-Hexyl-1-methyl-2-(1-methylpyridin-1-ium-4-yl)-1*H*-benzimidazol-3-ium bis(hexafluorophosphate)**

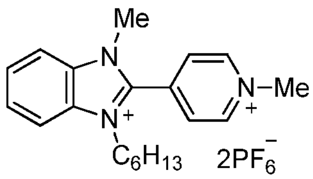

A mixture of 1-hexyl-2-(pyridin-4-yl)-1*H*-benzimidazole (0.51 g, 1.84 mmol) in MeOTs (1.71 g, 9.2 mmol) was heated at 180 °C for 3 h, cooled, triturated with $Et_2O$ (3 × 40 mL) and the residue air dried. The residue was dissolved in MeOH (8 mL) and added dropwise to a solution of $NaBF_4$ (8.10 g, 73.6 mmol) in water (100 mL) with stirring. Stirring was continued for 0.5 h. Ammonium hexafluorophosphate (10 g, 61 mmol) was added and stirring continued for 0.5 h. The resulting precipitate was filtered, washed with water (2 × 10 mL), triturated with hot MeOH (40 mL), cooled, filtered, washed with MeOH (5 mL) and air dried to give the *title compound* (0.96 g, 87 %) as a colourless powder, $\delta_H$ [$(CD_3)_2CO$, 400 MHz] 0.83 (3H, t, *J* = 6.7), 1.17 - 1.45 (6H, m), 1.93 - 2.02 (2H, m), 4.17 (3H, s), 4.63 (2H, t, *J* = 7.7 Hz), 4.84 (3H, s), 7.87 - 7.95 (2H, m), 8.17 - 8.33 (2H, m), 8.98 (2H, d, *J* = 6 Hz) and 9.61 (2H, d, *J* = 6 Hz); $\delta_F$ [$(CD_3)_2CO$, 376 MHz] -72.687 (d, *J* = 710 Hz); $\delta_C$ [$(CD_3)_2CO$, 100 MHz] 13.20, 22.19, 25.80, 29.44, 31.07, 32.82, 47.06, 49.33, 99.81, 113.72, 113.86, 128.01, 130.43, 131.81, 132.86, 137.48, 145.90 and 147.99.

**EXAMPLE 10**

**4-(1-Methyl-1*H*-benzimidazol-2-yl)-1-phenylpyridin-1-ium triflate**

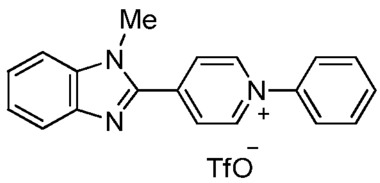

A mixture of 1-methyl-2-(pyridin-4-yl)-1*H*-benzimidazole (1.00 g, 4.8 mmol), diphenyliodonium triflate (3.08 g, 7.2 mmol), $Cu(OAc)_2.H_2O$ (9.6 mg, 0.48 mmol, 10 mol%) in dry DMF (50 mL) was heated at 100 °C for 16 h, cooled and the solvent removed under reduced pressure. The residue was triturated with $Et_2O$ (100 mL), air dried and then crystallised from hot MeOH (10 mL). The product was isolated by filtration, washed with MeOH and air dried to give the *title compound* (1.33 g, 64 %) as a yellow powder, $\delta_H$ [$(CD_3)_2CO$, 400 MHz] 4.294 (3H, s), 7.38 - 7.45 (1H, m), 7.47 - 7.53 (1H, m), 7.71 - 7.90 (5H, m), 8.01 - 8.11 (2H, m), 8.95 (2H, d, 7 Hz) and 9.51 (2H, d, *J* = 7 Hz); $\delta_F$ [$(CD_3)_2CO$, 376 MHz] -78.86; $\delta_C$ [$(CD_3)_2CO$, 100 MHz] 32.21, 111.26, 120.77, 123.65, 124.62, 125.32, 127.03, 130.60, 131.68, 138.12, 142.98, 143.39, 144.99, 146.72 and 146.88.

**Compound 10: 1,3-Dimethyl-2-(1-phenylpyridin-1-ium-4-yl)-1*H*-benzimidazol-3-ium bis(tetrafluoroborate)**

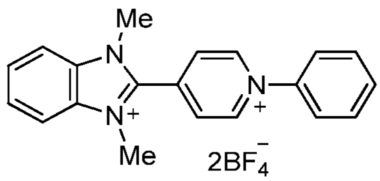

A mixture of 4-(1-methyl-1*H*-benzimidazol-2-yl)-1-phenylpyridin-1-ium triflate (0.54 g, 1.2 mmol) in MeOTs (4.62 g, 24.8 mmol) was heated at 180 °C for 3 h, cooled, triturated with $Et_2O$ (50 mL), the residue was filtered off, washed with $Et_2O$ (2 × 10 mL) and air dried. The residue was dissolved in MeOH (10 mL) and added dropwise to a solution of $NaBF_4$ (2.73 g, 24.8 mmol) in water (50 mL) with stirring. Stirring was continued for 0.5 h and the resulting precipitate filtered, washed with water (2 × 5 mL) and air dried. The residue was triturated with hot MeOH (5 mL), filtered and air dried to give the *title compound* (0.49 g, 83 %) as a pale tan powder, $\delta_H$ (DMSO-$d_6$, 400 MHz) 4.04 (6H, s), 7.80 - 8.01 (7H, m), 8.19 - 8.29 (2H, m), 8.81 (2H, d, *J* = 6.5 Hz) and 9.82 (2H, d, *J* = 6.5 Hz); $\delta_F$ (DMSO-$d_6$, 376 MHz) -148.26, -148.20; $\delta_C$ (DMSO-$d_6$, 100 MHz) 33.49, 114.25, 125.24, 128.15, 130.84, 130.99, 132.41, 132.53, 137.83, 142.96, 145.50 and 146.81.

**EXAMPLE 11**

**Compound 11: 1-Hexyl-3-methyl-2-(1-phenylpyridin-1-ium-4-yl)-1*H*-benzimidazol-3-ium bis(hexafluorophosphate)**

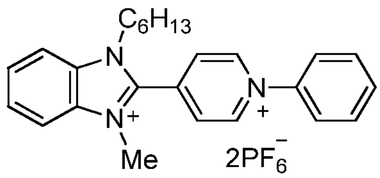

A mixture of 1-hexyl-2-(pyridin-4-yl)-1*H*-benzimidazole (0.8.0 g, 2.9 mmol), diphenyliodonium triflate (1.85 g, 4.3 mmol), $Cu(OAc)_2.H_2O$ (58 mg, 0.29 mmol, 10 mol%) in dry DMF (30 mL) was heated at 100 °C for 16 h, cooled and the solvent removed under reduced pressure. The residue was triturated with $Et_2O$ (3 × 30 mL) and dried under vacuum. The residue was heated in MeOTs (4.26 g, 22.4 mmol) at 180 °C for 2 h, cooled and then diluted with $Et_2O$ (80 mL). The solvent was decanted and the residue triturated with $Et_2O$ (3 × 30 mL) and dried under vacuum. The residue was dissolved in MeOH (20 mL) and added drop wise to a solution of ammonium hexafluorophosphate (4.16 g, 28.7 mmol) in water (100 mL). The precipitate was filtered and washed with water (2 × 10 mL) and air dried to give the *title compound* (0.68 g, 38 %) as a cream powder, $\delta_H$ [$(CD_3)_2CO$, 400 MHz] 0.82 (3H, t, *J* = 6.9 Hz), 1.17 - 1.45 (6H, m), 1.96 - 2.04

(2H, m), 4.19 (3H, s), 4.66 (2H, t, *J* = 7.7 Hz), 7.81 - 7.95 (5H, m), 7.99 - 8.10 (2H, m), 8.17 - 8.32 (2H, m), 9.13 (2H, bd, *J* = 5.2 Hz) and 9.89 (2H, bd, *J* = 5.2 Hz); $\delta_F$ [$(CD_3)_2CO$, 376 MHz] -72.47 (d, *J* = 710 Hz); $\delta_C$ [$(CD_3)_2CO$, 100 MHz] 13.31, 22.18, 25.82, 29.49, 31.06, 32.92, 47.14, 113.76, 113.94, 124.76, 128.08, 130.75, 130.95, 131.89, 132.35, 132.96, 138.59, 143.02, 144.71 and 147.04.

**3. Imidazo[1,2-a]pyridines**

**EXAMPLE 12**

**7-Bromo-2-phenylimidazo[1,2-*a*]pyridine A**

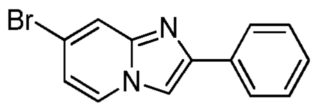

A mixture of phenacyl bromide (4.00 g, 20.1 mmol), 4-bromopyridin-2-amine (2.90 g, 16.8 mmol) and $NaHCO_3$ (1.69 g, 20.1 mmol) in MeOH (80 mL) was heated at reflux for 5 h, cooled and the solvent reduced. Water (200 mL) was added and the resulting mixture extracted with DCM (3 × 100 mL), dried ($Na_2SO_4$) and the solvent removed under reduced pressure. The residue was chromatographed on silica using MeOH (5 % in DCM) as eluent. The solvent was removed under reduced pressure and the residue crystallised from DCM/hexanes to give the *title compound* (2.07 g, 39 %) as a cream powder, $\delta_H$ ($CDCl_3$, 400 MHz) 6.91 (1H, dd, *J* = 1.9 and 7.1 Hz), 7.34 - 7.40 (1H, m), 7.43 - 7.49 (2H, m), 7.83 - 7.87 (2H, m), 7.92 - 7.98 (2H, m) and 8.00 (2H, dd, *J* = 0.5 and 7.1 Hz); $\delta_C$ ($CDCl_3$, 100 MHz) 108.27, 116.34, 118.21, 119.80, 125.72, 126.12, 128.30, 128.80, 133.27, 145.83 and 146.71.

**2-Phenyl-7-(pyridin-4-yl)imidazo[1,2-*a*]pyridine C**

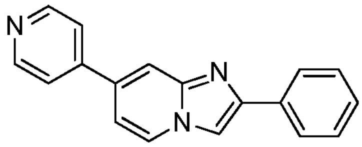

A mixture of 7-bromo-2-phenylimidazo[1,2-*a*]pyridine **A** (1.18 g, 4.3 mmol), and 4-(4,4,5,5-tetramethyl-1,3,2-dioxaborolan-2-yl)pyridine (1.32 g, 6.4 mmol), $K_2CO_3$ (0.90 g, 6.5 mmol) and $Pd(PPh_3)_4$ (0.20 g, 4 mol%) in degassed PhMe (40 mL) and EtOH (40 mL) under $N_2$ was heated at reflux for 16 h, cooled, poured into water (50 ml) and extracted with DCM (3 × 40 mL). The combined extracts were dried ($Na_2SO_4$) and the solvent removed under reduced pressure. The residue was chromatographed on silica using MeOH (5 % in DCM) as eluent. The band with Rf = 0.5 was collected, the solvent removed and the residue triturated with hot PhMe (15 mL). After cooling the product was collected and air dried to give the *title compound* (1.02 g, 87 %) as a cream powder, $\delta_H$ ($CDCl_3$, 400 MHz) 7.12 (1H, dd, *J* = 1.6 and 7 Hz), 7.35 - 7.42 (1H, m), 7.45 - 7.52 (2H, m), 7.59 (2H, dd, *J* = 1.5 and 4.6 Hz), 7.94 - 8.03 (4H, m), 8.25 (1H, dd, *J* = 0.9 and 7 Hz) and 8.74 (2H, dd, *J* = 1.4 and 4.7 Hz); $\delta_C$ ($CDCl_3$, 100 MHz) 108.43, 111.29, 115.22, 120.96, 125.90, 126.12, 128.34, 128.83, 133.42, 134.26, 145.67, 145.87, 147.38 and 150.66.

**1-Hexyl-4-(2-phenylimidazo[1,2-*a*]pyridin-7-yl)pyridin-1-ium iodide F**

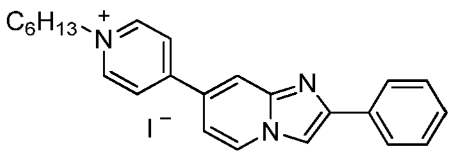

A mixture of 2-phenyl-7-(pyridin-4-yl)imidazo[1,2-*a*]pyridine C (1.02 g, 3.8 mmol) and 1-iodohexane (2.39 g, 11.3 mmol) in MeCN (40 mL) was heated at reflux for 16 h, cooled, the solvent reduced, and the mixture diluted with $Et_2O$ (50 mL). The precipitate was filtered, washed with $Et_2O$ (2 × 30 mL) and air dried to give the *title compound* (1.81 g, 99 %) as an orange powder, $\delta_H$ ($CD_3OD$, 400 MHz) 0.94 (3H, t, *J* = 6.9 Hz), 1.32 - 1.51 (6H, m), 2.00 - 2.14 (2H, m), 4.63 (2H, t, *J* = 7.4 Hz), 7.37 - 7.57 (4H, m), 7.99 (2H, d, *J* = 7.9 Hz), 8.29 (1H, s), 8.43 (1H, s), 8.53 (2H, d, *J* = 6.3 Hz), 8.68 (1H, d, *J* = 7.1 Hz) and 9.01 (2H, d, *J* = 6.3 Hz); $\delta_C$ ($CD_3OD$, 100 MHz)12.85, 22.08, 25.50, 30.91, 30.98, 60.92, 110.62, 110.81, 116.23, 124.51, 125.89, 127.48, 128.49, 128.61, 130.34, 132.67, 144.53 and 153.97

**1-Hexyl-4-(2-phenylimidazo[1,2-*a*]pyridin-7-yl)pyridin-1-ium tetrafluoroborate**

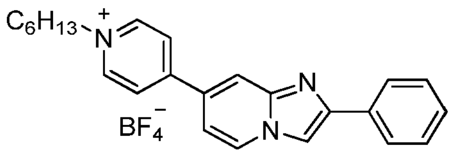

A solution of 1-hexyl-4-(2-phenylimidazo[1,2-*a*]pyridin-7-yl)pyridin-1-ium iodide **F** (1.81 g, 3.7 mmol) in MeOH (20 mL) was added dropwise to a solution of $NaBF_4$ (2.47 g, 22.4 mmol) in water (100 mL) with stirring. Stirring was continued for 0.5 h and the resulting precipitate filtered, washed with water (5 mL) and air dried to give the *title compound* (1.66 g, 100 %) as a golden yellow powder which was used without further purification in the next step.

**Compound 12: 7-(1-Hexylpyridin-1-ium-4-yl)-1-methyl-2-phenylimidazo[1,2-*a*]pyridin-1-ium bis(tetrafluoroborate)**

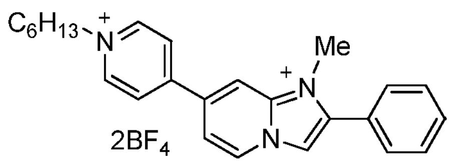

A mixture of 1-hexyl-4-(2-phenylimidazo[1,2-*a*]pyridin-7-yl)pyridin-1-ium tetrafluoroborate (1.66 g, 3.7 mmol) and MeOTs (2.79 g, 15 mmol) was heated at 180 °C for 2 h, cooled, triturated with $Et_2O$ (3 × 50 mL) and air dried. The residue was dissolved in MeOH (25 mL) and added dropwise to a solution of $NaBF_4$ (16.48 g, 150 mmol) with stirring. The resulting precipitate was filtered, washed with water (5 mL), then dissolved in warm MeOH (50 mL) and added dropwise to a solution of $NaBF_4$ (16.48 g, 150 mmol) in water (250 mL) with stirring. Stirring was continued for 0.5 h and the resulting precipitate filtered. The residue was triturated with EtOH (3 ml), filtered and air dried to give the *title compound* (0.38 g, 19 %) as a pale green powder. The ethanolic liquors were added dropwise to a solution of $NaBF_4$ (16.48 g, 150 mmol) in water (50 mL) with stirring. The resulting precipitate was filtered, washed with water (10 mL) and air dried to give a second crop (0.45 g, 22 %) as a khaki powder; $\delta_H$ [$(CD_3)_2CO$, 400 MHz] 0.88 (3H, t, *J* = 6.8 Hz), 1.26 - 1.55 (6H, m), 2.12 - 2.27 (2H, m), 4.26 (3H, s), 4.91 (2H, t, *J* = 7.2 Hz), 7.70 (3H, bs), 7.82 (2H, bs), 8.26 (1H, bs, *J* = 6.6 Hz), 8.69 (1H, bs), 8.86 (2H, bd, *J* = 5.8 Hz), 9.05 (1H, bs), 9.21 (1H, bd, *J* = 6.6 Hz) and 9.34 (2H, bd, *J* = 5.8 Hz); $\delta_F$ ($CDCl_3$, 376 MHz) -151.02, -150.97; $\delta_C$ ($CDCl_3$, 100 MHz) 13.31, 22.16, 25.54, 30.98, 31.26, 32.42, 61.73, 111.65, 113.97, 116.12, 125.24, 126.39, 129.44, 130.01, 130.29, 131.13, 138.32, 140.23, 140.38, 145.62 and 151.68.

**EXAMPLE 13**

**7-Bromo-2-(pyridin-4-yl)imidazo[1,2-*a*]pyridine B**

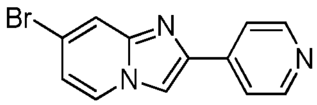

A mixture of 4-(bromoacetyl)pyridine hydrobromide (8.00 g, 28.5 mmol), 4-bromopyridin-2-amine (4.1 g, 23.7 mmol) and $NaHCO_3$ (4.78 g, 56.9 mmol) in MeOH (80 mL) was heated at reflux for 5 h, cooled and the solvent reduced in volume. Water (200 mL) was added and the resulting mixture extracted with DCM (3 × 150 mL), dried ($Na_2SO_4$) and the solvent removed under reduced pressure. The residue was chromatographed on silica using MeOH (5 % in DCM) as eluent. The fluorescent band with Rf = 0.6 (5 % MeOH in DCM) was collected. The solvent was removed under reduced pressure and the residue crystallised from MeOH (5 mL) at 3 °C, filtered and air dried to give the *title compound* (0.68 g, 10 %) as a light tan powder, $\delta_H$ ($CDCl_3$, 400 MHz) 6.96 (1H, dd, *J* = 1.7 and 7.2 Hz), 7.82 (2H, d, *J* = 6 Hz), 7.86 (1H, bs), 8.00 (1H, bs), 8.04 (1H, d, *J* = 7.2 Hz) and 8.69 (2H, d, *J* = 6 Hz); $\delta_C$ ($CDCl_3$, 100 MHz) 110.02, 117.09, 119.21, 120.19, 120.33, 125.95, 140.69, 143.88, 146.07 and 150.40.

**7-Phenyl-2-(pyridin-4-yl)imidazo[1,2-*a*]pyridine D**

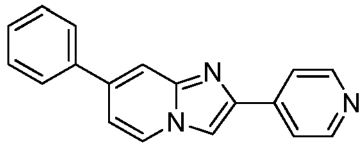

A mixture of 7-bromo-2-(pyridin-4-yl)imidazo[1,2-*a*]pyridine **B** (1.17 g, 4.3 mmol) and phenylboronic acid (0.78 g, 6.4 mmol), $K_2CO_3$ (0.88g, 6.4 mmol) and $Pd(PPh_3)_4$ (0.25 g, 5 mol%) in degassed PhMe (30 mL) and EtOH (30 mL) under $N_2$ was heated at reflux for 24 h, cooled, poured into water (100 mL) and extracted with DCM (3 × 80 mL). The extracts were dried ($Na_2SO_4$) and the solvent removed under reduced pressure. The residue was chromatographed on silica using MeOH (5 % in DCM) as eluent. The solvent was removed under reduced pressure and the residue triturated with hexanes containing a few drops of DCM. The residue was filtered off, washed with hexanes and air dried to give the *title compound* (1.02 g, 88 %) as a light tan powder, $\delta_H$ ($CDCl_3$, 400 MHz) 7.16 (1H, dd, *J* = 1.6 and 7 Hz), 7.41 - 7.57 (2H, m), 7.66 - 7.72 (2H, m), 7.83 - 7.92 (3H, m), 8.03 (1H, s), 8.21 (1H, d, *J* = 7 Hz) and 8.70 (2H, d, *J* = 6 Hz); $\delta_C$ ($CDCl_3$, 100 MHz) 109.55, 113.14, 114.54, 120.29, 125.66, 126.74, 128.53, 129.19, 138.40, 138.57, 141.16, 143.81, 146.49 and 150.37.

**1-Hexyl-4-(7-phenylimidazo[1,2-*a*]pyridin-2-yl)pyridin-1-ium iodide G**

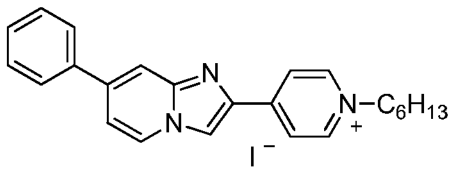

A mixture of 7-phenyl-2-(pyridin-4-yl)imidazo[1,2-*a*]pyridine **D** (1.00 g, 3.7 mmol) and 1-iodohexane (2.62 g, 12.4 mmol) in MeCN (40 mL) was heated at reflux for 16 h, cooled, diluted with $Et_2O$ (100 ml) and stirred for 0.5 h. The resulting precipitate was filtered, washed with $Et_2O$ (3 × 40 mL) and air dried to give the *title compound* (1.73 g, 97 %) as a dull yellow powder, $\delta_H$ ($CD_3OD$, 400 MHz) 0.95 (3H, t, *J* = 6.8 Hz), 1.32 - 1.54 (6H, m), 2.00 - 2.13 (2H, m), 4.61 (2H, t, *J* = 7.3 Hz), 7.40 - 7.59 (4H, m), 7.81 (2H, d, *J* = 7.6 Hz), 7.87 (1H, s), 8.56 (2H, d, *J* = 6 Hz), 8.61 (1H, d, *J* = 7.2 Hz), 8.87 (1H, s) and 8.96 (2H, d, *J* = 6 Hz); $\delta_C$ ($CD_3OD$, 100 MHz) 12.85, 22.07, 25.49, 30.90, 60.82, 112.96, 114.05, 115.63, 123.02, 126.49, 127.25, 128.74, 128.94, 137.62, 138.98, 140.88, 144.41, 147.30 and 149.56.

**Compound 13: 2-(1-Hexylpyridin-1-ium-4-yl)-1-methyl-7-phenylimidazo[1,2-*a*]pyridin-1-ium bis(tetrafluoroborate)**

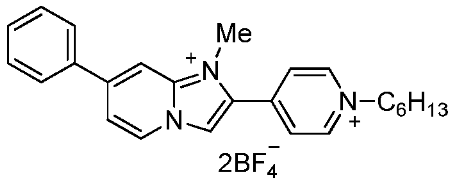

A solution of 1-hexyl-4-(7-phenylimidazo[1,2-*a*]pyridin-2-yl)pyridin-1-ium iodide **G** (1.73 g, 3.8 mmol) in MeOH (50 mL) was added dropwise by filtration through a cotton wool plug to $NaBF_4$ (10.00 g, 90 mmol) water (100 mL) with stirring. Stirring was continued for 0.5 h and the resulting precipitate was collected by filtration, washed with water (2 × 5 mL) and air dried. The resulting solid and MeOTs (2.87 g, 15.4 mmol) was heated at 180 °C with stirring for 2 h. The resulting oil was cooled, triturated with $Et_2O$ (5 × 50 mL) and air dried. The resulting gummy solid was dissolved in MeOH (30 mL) and added dropwise to a solution of $NaBF_4$ (7.54 g, 68.5 mmol) in water (100 mL) with stirring. Stirring was continued for 0.5 h and the resulting precipitate collected, dissolved in hot MeOH (60 mL) and added dropwise to $NaBF_4$ (7.54 g, 68.5 mmol) in water (100 mL) with stirring. Stirring was continued for 0.5 h and the resulting precipitate filtered, washed with water (2 × 10 mL) and air dried. The solid was crystallised from hot MeOH (30 mL), filtered and air dried to give the *title compound* (1.37 g, 74 %) as a tan powder, $\delta_H$ [$(CD_3)_2CO$, 400 MHz] 0.90 (3H, t, *J* = 7 Hz), 1.29 - 1.59 (6H, m), 2.16 - 2.28 (2H, m), 4.41 (3H, s), 4.96 (2H, t, *J* = 7.5 Hz), 7.59 -7.69 (3H, m), 8.02 - 8.16 (3H, m), 8.65 (1H, bs), 8.70 (2H, d, *J* = 6.2 Hz), 9.02 (1H, bs), 9.12 (1H, d, *J* = 7 Hz) and 9.41 (2H, d, *J* = 6.2 Hz); $\delta_F$ [$(CD_3)_2CO$, 376 MHz] -151.24, -151.18; $\delta_C$ [$(CD_3)_2CO$, 100 MHz] 13.29, 22.14, 25.54, 30.98, 31.25, 32.80, 62.15, 107.70, 116.81, 117.43, 127.72, 128.36, 129.55, 130.05, 130.73, 133.24, 135.84, 142.18, 142.50, 145.77 and 147.56.

**EXAMPLE 14**

**2,7-Di(pyridin-4-yl)imidazo[1,2-*a*]pyridine E**

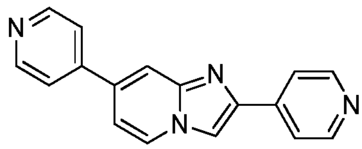

A mixture of 7-bromo-2-(pyridin-4-yl)imidazo[1,2-*a*]pyridine **B** (1.25 g, 4.6 mmol) and 4-(4,4,5,5-tetramethyl-1,3,2-dioxaborolan-2-yl)pyridine (1.22 g, 5.9 mmol), $K_2CO_3$ (0.82 g, 5.9 mmol) and $Pd(PPh_3)_4$ (0.26 g, 5 mol%) in degassed PhMe (30 mL) and EtOH (30 mL) under $N_2$ was heated at reflux for 16 h, cooled and the solvent removed under reduced pressure. The residue was washed with water (3 × 50 mL) and air dried. The residue was chromatographed on silica (pretreated with $Et_3N$ 10 % in DCM) using $Et_3N$ (10 % in DCM) to $Et_3N$/MeOH/DCM (12/3/85) gradient as eluent. The fractions were evaporated under reduced pressure and the residue triturated with hexanes. The resulting solid was dissolved in MeOH (10 mL) and water (100 mL) added. The resulting precipitate was filtered, washed with water and air dried to give a yellow powder which was used without further purification in the next step, $\delta_H$ ($CD_3OD$, 400 MHz) 7.38 (1H, dd, *J* = 1.7 and 7.2 Hz), 7.84 (2H, d, *J* = 6.3 Hz), 7.96 (2H, d, *J* = 6.2 Hz), 7.98 (1H, bs), 8.51 (1H, bs), 8.54 - 8.62 (2H, m) and 8.65 (2H, d, *J* = 6.2 Hz); $\delta_C$ ($CD_3OD$, 100 MHz) 111.88, 111.99, 114.05, 120.46, 121.38, 127.36, 135.64, 141.74, 142.94, 145.89, 146.28, 149.28 and 149.58.

**4,4'-(Imidazo[1,2-*a*]pyridine-2,7-diyl)bis(1-hexylpyridin-1-ium) bis(tetrafluoroborate) H**

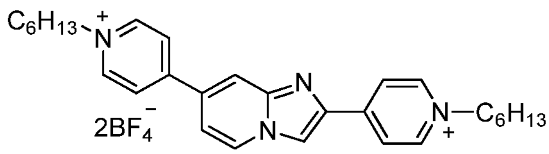

A solution of 2,7-di(pyridin-4-yl)imidazo[1,2-*a*]pyridine **E** (0.80 g, 2.9 mmol) and 1-iodohexane (3.74 g, 17.6 mmol) in MeCN (50 mL) was heated at reflux for 16 h, cooled, the solvent reduced in volume (~20 mL) and $Et_2O$ (60

mL) added. The resulting precipitate was filtered, washed with $Et_2O$ (2 × 30 mL) and air dried to give 1.69 g. The yellow powder dissolved in MeOH (60 mL) was added dropwise with via filtration through a cotton wool plug to $NaBF_4$ (6.47 g, 58.8 mmol) in water (300 mL) with stirring. The resulting precipitate was filtered, washed with ice cold water (2 × 20 mL) and air dried to give the *title compound* (1.32 g, 72 %) as a green fluorescent powder, $\delta_H$ (DMSO-$d_6$, 400 MHz) 0.83 - 0.94 (6H, m), 1.24 - 1.39 (12H, m), 1.90 - 2.03 (4H, m), 4.54 - 4.65 (4H, m), 7.75 (1H, dd, *J* = 1.4 and 7.3 Hz), 8.62 - 8.76 (5H, m), 8.93 (2H, d, *J* = 7.2 Hz), 9.10 (2H, d, *J* = 6.7 Hz), 9.16 (1H, bs) and 9.20 (2H, d, *J* = 6.7 Hz); $\delta_F$ (DMSO-$d_6$, 376 MHz) -148.21, -148.16; $\delta_C$ (DMSO-$d_6$,100 MHz) 14.31, 14.32, 22.34, 22.35, 25.57, 25.59, 31.02, 31.07, 31.10, 60.57, 60.61, 112.43, 117.54, 118.58, 123.59, 125.09, 129.20, 132.08, 141.17, 145.40, 145.51, 145.89, 148.60 and 152.42.

**Compound 14: 4,4'-(1-Methylimidazo[1,2-*a*]pyridine-1-ium-2,7-diyl)bis(1-hexylpyridin-1-ium) tris(tetrafluoroborate)**

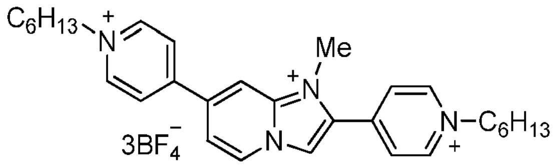

A mixture of 4,4'-(imidazo[1,2-*a*]pyridine-2,7-diyl)bis(1-hexylpyridin-1-ium) bis(tetrafluoroborate) (1.17 g, 1.9 mmol) and MeOTs (2.83 g, 15.2 mmol) was heated at 180 °C for 2 h, cooled and triturated with $Et_2O$ (50 mL). The residue was filtered and dried under vacuum to give 1.85 g. The solid was dissolved in MeOH (30 mL), added dropwise to a solution of $NaBF_4$ (30 g, 270 mmol) in water (250 mL) with stirring. Stirring was continued for 0.5 h and the resulting precipitate filtered, washed with water (2 × 5 mL) and air dried. The residue was dissolved in MeOH/$H_2O$ (30 mL, 1:1) and added dropwise to $NaBF_4$ (20 g, 182 mmol) in water (150 ml) with stirring. Stirring was continued for 0.5 h and the resulting precipitate filtered, washed with water (2 × 5 mL) and air dried to give the *title compound* (0.99 g, 73 %) as a grey powder, $\delta_H$ [$(CD_3)_2CO$, 400 MHz] 0.84 - 0.96 (6H, m), 1.27 - 1.59 (12H, m), 2.16 - 2.28 (4H, m), 4.42 (3H, s), 4.91 - 5.04 (4H, m), 8.33 (1H, bd, *J* = 6.7 Hz), 8.73 (2H, bd, *J* = 6 Hz), 8.90 (2H, bd, *J* = 6 Hz), 8.12 (2H, app. bs), 9.29 (1H, bd, *J* = 7 Hz), 8.40 (2H, bd, *J* = 6.4 Hz) and 9.45 (2H, bd, *J* = 6 Hz); $\delta_F$ [$(CD_3)_2CO$, 376 MHz] -151.17, - 151.12; $\delta_C$ [$(CD_3)_2CO$, 100 MHz] 13.29, 22.14, 25.54, 30.98, 31.25, 31.28, 33.13, 61.85, 62.27, 111.97, 116.76, 117.63, 126.64, 128.75, 130.89, 134.01, 140.01, 141.69, 141.84, 145.70, 145.85 and 151.48.

**4. Phenanthroimidazoles**

**EXAMPLE 15**

**6,9-Dibromo-1,2-bis[4-(*tert*-butyl)phenyl]-1*H*-phenanthro[9,10-*d*]imidazole**

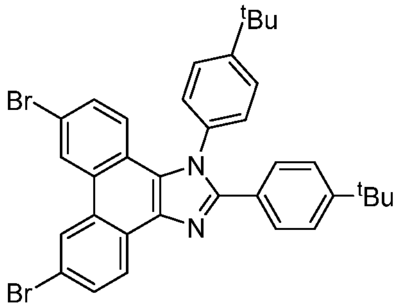

To a stirred mixture of 4-*tert*-butylaniline (2.20 g, 14.6 mmol, 2.35 mL, 1.5 equiv.) and 4-*tert*-butylbenzaldehyde (1.58 g, 9.75 mmol, 1.53 mL) was added acetic acid (100 mL), followed by 3,6-dibromophenanthrene-9,10-dione (3.58 g, 9.75 mmol) and ammonium acetate (9.38 g, 122 mmol, 12.5 equiv.), and the mixture heated at reflux under argon for 2 days. Methanol (20 mL) was added carefully, followed by water until the solution became cloudy. After cooling, the

precipitate was collected by vacuum filtration and washed thoroughly with 1:1 water:methanol to give the *title compound* (6.20 g, 99 %) a green powder, $\delta_H$ ($CDCl_3$, 400 MHz) 1.29 (9H, s), 1.45 (9H, s), 6.96 (1H, d, *J* = 8.9 Hz), 7.30 (2H, app d, *J* = 8.5 Hz), 7.37 (1H, dd, *J* = 1.9 and 8.9 Hz), 7.40 (2H, app. d, *J* = 8.5 Hz), 7.51 (2H, app. d, *J* = 8.5 Hz), 7.61 (2H, app. d, *J* = 8.5 Hz), 7.83 (1H, dd, *J* = 1.7 and 8.5 Hz) and 8.69 - 8.76 (3H, m); $\delta_C$ ($CDCl_3$, 100 MHz) 31.16, 31.41, 34.70, 35.07, 119.07, 119.84, 122.01, 122.35, 124.59, 125.25, 125.95, 126.17, 126.84, 127.18, 127.22, 128.00, 128.41, 128.63, 128.88, 129.53, 129.89, 130.87, 135.61, 137.06, 151.67, 152.23 and 153.56.

**1,2-Bis[4-(*tert*-butyl)phenyl]-6,9-di(pyridin-4-yl)-1*H*-phenanthro[9,10-*d*]imidazole**

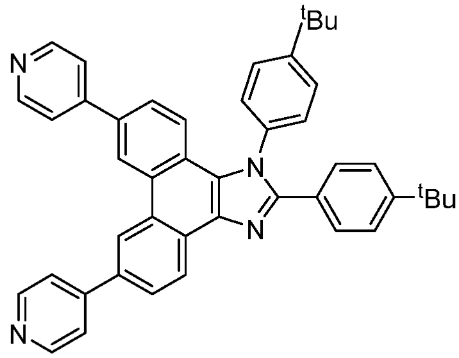

From 6,9-dibromo-1,2-bis-[4-(*tert*-butyl)phenyl]-1*H*-phenanthro[9,10-*d*]imidazole (5.25 g, 8.20 mmol), 4-(4,4,5,5-tetramethyl-1,3,2-dioxaborolan-2-yl)pyridine(3.53 g, 17.22 mmol, 2.1 equiv.), $Pd(PPh_3)_4$ (0.34 g, 0.287 mmol, 3.5 mol %), and $K_2CO_3$ (3.74 g, 17.22 mmol, 2.1 equiv.) in degassed PhMe/EtOH (1:1, 120 mL) for 5 days. After extraction and subsequent removal of the solvent the residue was slurried with DCM and passed through silica, followed by MeOH. The solvent was removed in vacuo, the residue triturated with $Et_2O$, and the resulting solid collected by vacuum filtration and air dried to give the *title compound* (4.28 g, 82 %) as a dark powder. $\delta_H$ ($CDCl_3$, 400 MHz) 1.31 (9H, s), 1.48 (9H, s), 7.27 (1H, d, *J* = 8.5 Hz), 7.32 (2H, d, *J* = 8.5 Hz), 7.47 (2H, d, *J* = 8.4 Hz), 7.55 - 7.58 (3H, m), 7.64 - 7.67 (4H, m), 7.75 (2H, d, *J* = 6 Hz), 8.01 (1H, dd, *J* = 1.2 and 8.3 Hz), 8.72 (2H, d, *J* = 6 Hz), 7.76 (2H, d, *J* = 6 Hz), 8.95 - 9.03 (3H, m); $\delta_C$ ($CDCl_3$, 100 MHz) 31.18, 31.45, 34.73, 35.11, 121.74, 121.78, 121.81, 122.02, 122.48, 123.70, 123.95, 125.29, 125.33, 126.32, 127.20, 127.36, 127.92, 128.39, 128.50, 128.57, 128.92, 129.28, 134.30, 135.29, 135.80, 137.70, 148.25, 148.84, 150.41, 150.42, 151.97, 152.27 and 153.55

**<u>Compound 15:</u> 4,4'-([1,2-bis(4-(*tert*-Butyl)phenyl]-3-methyl-1*H*-phenanthro[9,10-*d*]imidazole-3-ium-6,9-diyl)bis(1-methylpyridin-1-ium) tris(tetrafluoroborate)**

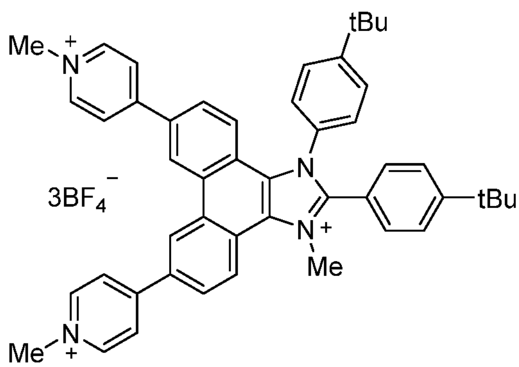

A mixture of 1,2-bis[4-(*tert*-butyl)phenyl]-6,9-di(pyridin-4-yl)-1*H*-phenanthro[9,10-*d*]imidazole (1.27 g, 2.0 mmol) and MeOTs (3.36 g, 18 mmol, 9.0 equiv.) was heated to 180 °C whilst stirring under argon for 8 hours, cooled, triturated with $Et_2O$, and the solid collected by vacuum filtration to give a grey powder (2.75 g). A mixture of the crude product (1.17 g) and MeOTs (3.6 g) was heated to 180 °C whilst stirring under argon for 32 hours, cooled, triturated with $Et_2O$,

and the solid collected by vacuum filtration. A filtered solution of the solid in hot 1:1 MeOH:water was added dropwise whilst stirring to a solution of $NaBF_4$ (2.64 g, 24 mmol, 12.0 equiv) in water (25 mL) to give the *title compound* (0.71 g, 36 %) as a green powder, $\delta_H$ (DMSO-$d_6$, 400 MHz) 1.28 (9H, s), 1.35 (9H, s), 4.39 (3H, s), 4.45 (6H, bs), 7.19 (1H, d, *J* = 8.8 Hz), 7.59 - 7.77 (9H, m), 8.26 (1H, dd, *J* = 1.5 and 8.9 Hz), 8.65 (1H, dd, *J* = 1.5 and 8.9 Hz), 8.85 (2H, app. d, *J* = 7 Hz), 8.95 (2H, app. d, *J* = 6.7 Hz), 9.10 - 9.22 (5H, m), 9.88 (1H, d, *J* = 1.5 Hz) and 9.93 (1H, d, *J* = 1.6 Hz). $\delta_F$ (DMSO-$d_6$, 376 MHz) -148.26 and -148.21; $\delta_C$ (DMSO-$d_6$, 100 MHz) 31.17, 31.39, 35.40, 38.42, 47.76, 47.83, 118.83, 122.65, 122.76, 123.48, 124.70, 125.48, 125.64, 125.78, 126.23, 126.36, 127.19, 127.65, 127.90, 128.27, 128.42, 128.53, 130.25, 130.36, 131.57, 132.61, 133.41, 133.51, 138.02, 146.12, 146.25, 151.71, 153.58, 153.72, 155.16 and 155.90.

**EXAMPLE 16**

**5,10-Dibromo-1,2-bis[4-(*tert*-butyl)phenyl]-1*H*-phenanthro[9,10-*d*]imidazole**

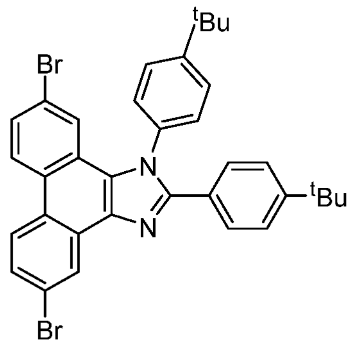

To a stirred mixture of 4-tert-butylaniline (2.20 g, 14.6 mmol, 2.35 mL, 1.5 equiv.) and 4-*tert*-butylbenzaldehyde (1.58 g, 9.75 mmol, 1.53 mL) was added acetic acid (100 mL), followed by 2,7-dibromophenanthrene-9,10-dione (3.58 g, 9.75 mmol) and ammonium acetate (9.38 g, 122 mmol, 12.5 equiv.), and the mixted heated under reflux under argon for 2 days. Methanol (20 mL) was added carefully, followed by water until the solution became cloudy. After cooling, the precipitate was collected by vacuum filtration and washed with 1:1 water:methanol to give a tan powder. The crude material was purified by column chromatography (neat DCM) to give the *title compound* (2.74 g, 44 %) an off-white powder, $\delta_H$ ($CDCl_3$, 400 MHz) 1.30 (9H, s), 1.47 (9H, s), 6.91 (1H, d, *J* = 1.9 Hz), 7.33 (2H, d, *J* = 8.5 Hz), 7.41 (2H, d, *J* = 8.3 Hz), 7.52 (1H, dd, *J* = 1.9 and 8.7 Hz), 7.62 (2H, d, *J* = 8.5 Hz), 7.66 (2H, d, *J* = 8.3 Hz), 7.69 (1H, dd, *J* = 2 and 9 Hz), 8.42 (1H, d, *J* = 8.7 Hz), 8.47 (1H, d, *J* = 9 Hz) and 9.00 (1H, d, *J* = 2 Hz); $\delta_C$ ($CDCl_3$, 100 MHz) 31.17, 31.43, 34.73, 35.12, 120.83, 122.00, 123.76, 124.26, 124.76, 125.36, 125.41, 125.35, 127.05, 127.16, 127.22, 127.34, 127.76, 127.92, 128.38, 128.58, 128.79, 128.84, 135.41, 137.02, 151.40, 152.31 and 153.90.

**1,2-Bis[4-(*tert*-butyl)phenyl]-5,10-di(pyridin-4-yl)-1*H*-phenanthro[9,10-*d*]imidazole**

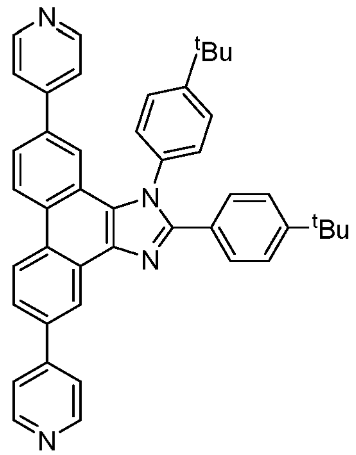

From 5,10-dibromo-1,2-bis[4-(*tert*-butyl)phenyl]-1H-phenanthro[9,10-*d*]imidazole (2.63 g, 4.10 mmol), 4-(4,4,5,5-tetramethyl-1,3,2-dioxaborolan-2-yl)pyridine(1.77 g, 8.61 mmol, 2.1 equiv.), $Pd(PPh_3)_4$ (0.17 g, 0.14 mmol, 3.5 mol %), and $K_2CO_3$ (1.87 g, 8.61 mmol, 2.1 equiv.) in degassed PhMe/EtOH (1:1, 80 mL) for 5 days. After extraction and subsequent removal of solvent, the solid was crystallised from hot PhMe to give the *title compound* (1.33 g, 51 %) as a green powder, $\delta_H$ ($CDCl_3$, 400 MHz) 1.31 (9H, s), 1.49 (9H, s), 7.22 (2H, dd, *J* = 1.5, 4.6 Hz), 7.35 (2H, app. d, *J* = 8.5 Hz), 7.50 - 7.56 (3H, m), 7.60 (2H, app. d, *J* = 8.5 Hz), 7.69 (2H, app. d, *J* = 8.8.5 Hz), 7.80 - 7.86 (3H, m), 7.95 (1H, dd, *J* = 2, 8.6 Hz), 8.57 (2H, dd, *J* = 1.4, 4.6 Hz), 8.76 (2H, dd, *J* = 1.4, 4.6 Hz), 8.81 (1H, d, *J* = 8.8 Hz), 8.86 (1H, d, *J* = 8.8 Hz), 9.21 (1H, d, *J* = 1.9 Hz); $\delta_C$ ($CDCl_3$, 100 MHz) 31.17, 31.51,34.74,35.13,119.32,121.08,121.22,121.94, 123.00, 123.78, 124.08, 124.33, 125.17, 125.38, 127.27, 127.32, 128.00, 128.10, 128.24, 128.60, 128.94, 129.05, 135.25, 135.98, 136.95, 137.81, 147.45, 147.93, 150.27, 150.38, 151.60, 152.30 and 153.66.

**Compound 16: 4,4'-([1,2-Bis(4-(*tert*-butyl)phenyl]-3-methyl-1*H*-phenanthro[9,10-*d*]imidazole-3-ium-5,10-diyl)bis(1-methylpyridin-1-ium) tris(tetrafluoroborate)**

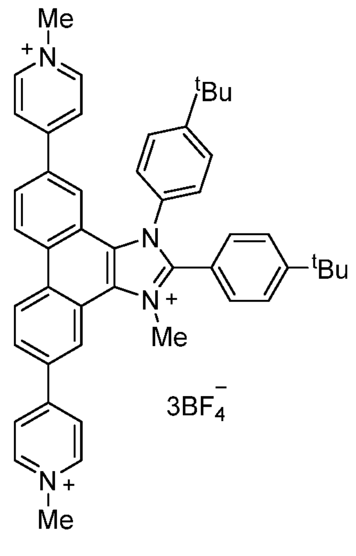

A mixture of 1,2-bis[4-(*tert*-butyl)phenyl]-5,10-di(pyridin-4-yl)-1*H*-phenanthro[9,10-*d*]imidazole (0.50 g, 0.79 mmol) and MeOTs (2.21 g, 11.9 mmol, 15 equiv.) was heated to 180 °C whilst stirring under argon for 24 hours, cooled, triturated with $Et_2O$, and the solid collected by vacuum filtration. A filtered solution of the solid in hot 4:1 MeOH:$H_2O$ was slowly added to a stirred solution of $NaBF_4$ (0.78 g, 7.11 mmol, 9.0 equiv.) in water (15 mL) and stirred for 30 minutes. The precipitate was collected by vacuum filtration and washed with water to give the *title compound* as a green powder (0.65 g, 86 %) as a green powder, $\delta_H$ (DMSO-$d_6$, 400 MHz) 1.27 (9H, s), 1.32 (9H, s), 4.35 (3H, s), 4.43 (3H, s), 4.57 (3H, s), 7.56 (1H, d, *J* = 1.7 Hz), 7.63 (2H, d, *J* = 8.5 Hz), 8.69 - 7.82 (6H, m), 7.98 (2H, d, *J* = 6.8 Hz), 8.44 (1H, dd, *J* = 1.7 and 8.8 Hz), 8.60 (1H, dd, *J* = 1.3 and 8.8 Hz), 8.88 (2H, d, *J* = 6.8 Hz), 8.96 (2H, d, *J* = 6.8 Hz), 9.17 (2H, d, *J* =

6.8 Hz), 9.28 (1H, d, *J* = 1.2 Hz), 9.47 - 9.57 (2H, m); $\delta_F$ (DMSO-$d_6$, 376 MHz) -148.25 and -148.2; $\delta_C$ (DMSO-$d_6$, 100 MHz) 31.16, 31.52, 35.40, 35.41, 38.06, 47.83, 47.89, 118.85, 121.13, 121.70, 122.46, 123.15, 124.81, 125.76, 125.90, 126.38, 126.92, 127.30, 127.65, 127.80, 127.87, 127.95, 128.38, 128.67, 131.03, 131.32, 131.61, 132.63, 134.18, 134.99, 146.26, 151.19, 153.50, 153.72, 155.16 and 155.89.

**EXAMPLE 17**

**6,9-Dibromo-1-[4-(*tert*-butyl)phenyl]-2-(pyridin-4-yl)-1*H*-phenanthro[9,10-*d*]imidazole**

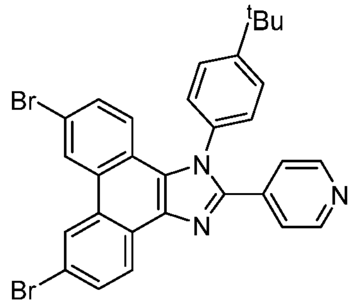

To a stirred mixture of 4-pyridinecarboxaldehyde (1.04 g, 9.75 mmol, 0.91 mL) and 4-tert-butylaniline (2.20 g, 14.6 mmol, 2.35 mL, 1.5 equiv.) was added acetic acid (100 mL), followed by 3,6-dibromophenanthrene-9,10-dione (3.58 g, 9.75 mmol) and ammonium acetate (9.38 g, 122 mmol, 12.5 equiv.), and the mixture heated under reflux under argon for 3 days. Methanol (20 mL) was added carefully, followed by water until the solution became cloudy. After cooling, the precipitate was collected by vacuum filtration and washed with 1 M $K_2CO_3$ solution, and air dried to give the *title compound* (4.76 g, 83 %) as a green powder, $\delta_H$ ($CDCl_3$, 400 MHz) 1.47 (9H, s), 6.99 (1H, d, *J* = 8.9 Hz), 7.37-7.56 (5H, m), 7.66 (2H, d, *J* = 8.5 Hz), 7.84 (1H, dd, *J* = 1.8 and 8.5 Hz), 8.54 (2H, d, *J* = 6.2 Hz) and 8.65 - 8.75 (3H, m); $\delta_C$ ($CDCl_3$, 100 MHz) 154.42, 149.94, 148.27, 137.89, 137.36, 134.98, 131.13, 130.17, 129.05, 128.82, 128.24, 128.10, 127.59, 126.99, 126.06, 125.31, 124.51, 122.74, 122.47, 121.71, 120.39, 119.91, 35.19 and 31.40.

**1-[4-(*tert*-Butyl)phenyl]-2,6,9-tri(pyridin-4-yl)-1*H*-phenanthro[9,10-*d*]imidazole**

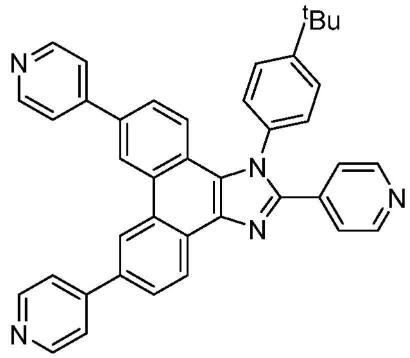

From 6,9-dibromo-1-(4-(*tert*-butyl)phenyl)-2-(pyridin-4-yl)-1*H*-phenanthro[9,10-*d*]imidazole (2.93 g, 5.00 mmol), 4-(4,4,5,5-tetramethyl-1,3,2-dioxaborolan-2-yl)pyridine(2.15 g, 10.5 mmol, 2.1 equiv.), $Pd(PPh_3)_4$ (0.20 g, 0.18 mmol, 3.5 mol %), and $K_2CO_3$ (1.45 g, 10.5 mmol, 2.1 equiv.) in degassed PhMe/EtOH (1:1, 120 mL) for 6 days. After extraction and subsequent removal of solvent, the residue was triturated with water (250 mL), then triturated with $Et_2O$ (50 mL). The resulting solid was dissolved in hot 10:1 PhMe:EtOH, cooled to -20 °C overnight, then concentrated in vacuo. The resulting precipitate was collected by vacuum filtration and washed with $Et_2O$ to give the *title compound* (1.50 g, 52 %) as a tan powder, $\delta_H$ ($CDCl_3$, 400 MHz) 1.50 (9H, s), 7.31 (1H, d, *J* = 8.7 Hz), 7.48 - 7.51 (4H, m), 7.56 - 7.77 (7H, m), 8.04 (1H, dd, *J* = 1.2 and 8.4 Hz), 8.56 (2H, d, *J* = 6.1 Hz), 8.73 (2H, d, 6 Hz), 8.76 (2H, d, *J* = 6 Hz), 8.94 - 9.05 (3H, m); $\delta_C$ ($CDCl_3$, 100 MHz) 31.43, 35.22, 121.81, 121.85, 122.01, 122.03, 122.60, 122.82, 123.38, 123.91,

125.62, 126.62, 127.61, 127.67, 128.19, 128.64, 129.29, 129.82, 135.12, 135.90, 137.63, 137.92, 148.05, 148.61, 149.95, 150.44, 150.47 and 154.42.

**Compound 17: 4,4',4"-{[1-(4-(*tert*-Butyl)phenyl]-3-methyl-1*H*-phenanthro[9,10-*d*]imidazole-3-ium-2,6,9-triyl} tris(1-methylpyridin-1-ium) tetrakis(tetrafluoroborate)**

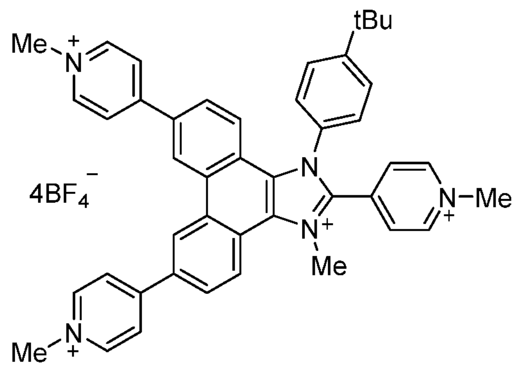

A mixture of 1-[4-(*tert*-butyl)phenyl]-2,6,9-tri(pyridin-4-yl)-1*H*-phenanthro[9,10-*d*]imidazole (1.17 g, 2.0 mmol) and MeOTs (2.60 g, 14 mmol, 7.0 equiv.) was heated to 180 °C whilst stirring under argon for 16 hours, cooled, triturated with $Et_2O$, and the solid collected by vacuum filtration. The solid was ground with a pestle and mortar along with a small amount of $Et_2O$, triturated with $Et_2O$, and air drive to give a green-yellow powder. A mixture of the powder (1.55 g) and MeOTs (1.0 g, 5.4 mmol) was heated to 180 °C whilst stirring under argon for 16 hours, cooled, triturated with $Et_2O$, and the solid collected by vacuum filtration. A filtered solution of the solid in hot MeOH:water (1:1) was added dropwise to a stirred solution of $NaBF_4$ (2.64 g, 24.0 mmol, 12.0 equiv.) in water (25 mL), stirred for 30 minutes, and the precipitate collected by vacuum filtration to give the *title compound* (0.71 g, 36 %) as a green powder, $\delta_H$ (DMSO-$d_6$, 400 MHz) 1.38 (9H, s), 4.39 (3H, s), 4.43 (3H, s), 4.45 (3H, s), 4.51 (3H, s), 7.02 (1H, d, *J* = 8.8 Hz), 8.26 (1H, dd, *J* = 1.8 and 8.8 Hz), 8.50 (2H, d, *J* = 6.8 Hz), 8.65 (1H, dd, *J* = 1.8 and 8.8 Hz), 8.84 (2H, d, *J* = 7.1 Hz), 8.95 (2H, d, *J* = 7 Hz), 9.12 - 9.22 (5H, m), 9.27 (2H, d, *J* = 7 Hz), 9.87 (1H, d, *J* = 1.8 Hz) and 9.93 (1H, d, *J* = 1.8 Hz); $\delta_F$ (DMSO-$d_6$, 376 MHz) -148.27, -148.22; $\delta_C$ (DMSO-$d_6$, 400 MHz) 31.41, 35.53, 38.62, 47.81, 47.88, 49.35, 122.24, 122.59, 122.95, 124.97, 125.58, 125.77, 126.37, 127.91, 128.22, 128.36, 128.55, 128.75, 130.62, 130.71, 131.28, 134.02, 134.15, 137.00, 145.64, 146.16, 146.28, 147.59, 153.41, 153.58 and 156.09.

**EXAMPLE 18**

**6,9-Dibromo-1-[4-(*tert*-butyl)phenyl]-2-(pyridin-4-yl)-1*H*-phenanthro[9,10-*d*]imidazole**

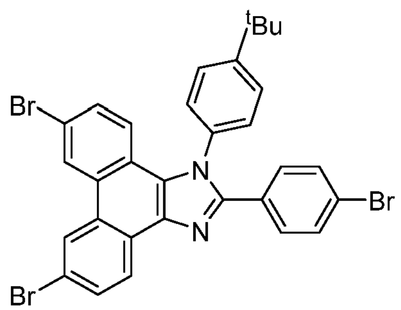

To a stirred mixture of 4-*tert*-butylaniline (2.20 g, 14.6 mmol, 2.35 mL, 1.5 equiv.) and 4-bromobenzaldehyde (1.80 g, 9.75 mmol) was added acetic acid (100 mL), followed by 3,6-dibromophenanthrene-9,10-dione (3.58 g, 9.75 mmol) and ammonium acetate (9.38 g, 122 mmol, 12.5 equiv.), and the mixture heated under reflux under argon for 2 days. Methanol (20 mL) was added carefully, followed by water until the solution became cloudy. After cooling, the precipitate was collected by vacuum filtration and washed with 1:1 water:methanol to give the *title compound* (6.10 g,

94 %) as a green powder, $\delta_H$ ($CDCl_3$, 400 MHz) 1.45 (9H, s), 6.97 (1H, d, *J* = 8.9 Hz), 7.34 - 7.44 (7H, m), 7.61 (2H, d, *J* = 8.4 Hz), 7.82 (1H, dd, *J* = 1.5 and 8.5 Hz) and 8.64 - 8.74 (3H, m); $\delta_C$ ($CDCl_3$, 100 MHz) 31.40, 35.12, 119.40, 120.05, 121.83, 122.34, 123.61, 124.48, 126.00, 126.02, 126.90, 127.37, 128.18, 128.25, 128.68, 129.12, 129.66, 130.01, 130.67, 130.96, 131.49, 135.25, 137.11, 150.33 and 153.93.

**1-[4-(*tert*-Butyl)phenyl]-6,9-di(pyridin-4-yl)-2-[4-(pyridin-4-yl)phenyl]-1*H*-phenanthro[9,10-*d*]imidazole**

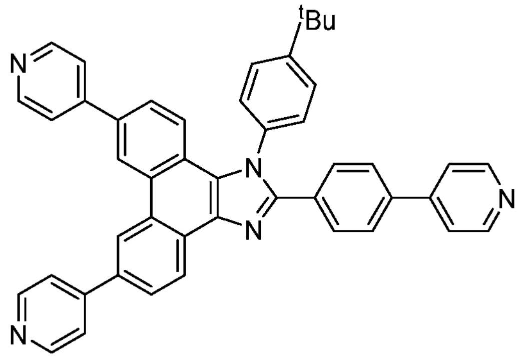

From 6,9-dibromo-1-[4-(*tert*-butyl)phenyl]-2-(4-bromophenyl)-1*H*-phenanthro[9,10-*d*]imidazole (5.44 g, 8.20 mmol), 4-(4,4,5,5-tetramethyl-1,3,2-dioxaborolan-2-yl)pyridine(5.30 g, 25.8 mmol, 3.15 equiv.), $Pd(PPh_3)_4$ (0.34 g, 0.38 mmol, 3.5 mol %), and $K_2CO_3$ (5.61 g, 25.8 mmol, 3.15 equiv.) in degassed PhMe/EtOH (1:1, 120 mL) for 7 days. After extraction and subsequent removal of solvent, the residue was dissolved in hot 1:1 DCM:isopropanol, cooled to -20 °C overnight, then concentrated in vacuo. The resulting precipitate was collected by vacuum filtration and washed with $Et_2O$ to give the *title compound* (3.73 g, 59 %)as a green powder, $\delta_H$ ($CDCl_3$, 400 MHz) 1.49 (9H, s), 7.32 (1H, d, *J* = 8.6 Hz), 7.48 - 7.53 (4H, m), 7.57 - 7.62 (3H, m), 7.64 - 7.71 (4H, m), 7.73 - 7.79 (4H, m), 8.06 (1H, dd, *J* = 1.4, 8.3 Hz), 8.67 (2H, dd, *J* = 1.5, 4.6 Hz), 8.73 (2H, dd, *J* = 1.5, 4.6 Hz), 8.77 (2H, dd, *J* = 1.5, 4.6 Hz), 8.98 - 9.07 (3H, m); $\delta_C$ ($CDCl_3$, 100 MHz) 31.45, 35.17, 121.46, 121.81, 121.91, 122.05, 122.58, 123.57, 123.93, 125.50, 126.50, 126.84, 127.40, 127.81, 128.23, 128.42, 128.53, 128.87, 129.53, 129.90, 130.98, 134.71, 135.58, 135.62, 137.86, 138.44, 147.30, 148.18, 148.77, 150.38, 150.44, 150.92 and 153.92.

**Compound 18: 4,4'-{1-[4-(*tert*-Butyl)phenyl]-3-methyl-2-(4-(1-methylpyridin-1-ium-4-yl)phenyl)-1*H*-phenanthro[9,10-*d*]imidazole-3-ium-6,9-diyl}bis(1-methylpyridin-1-ium) tetrakis(tetrafluoroborate)**

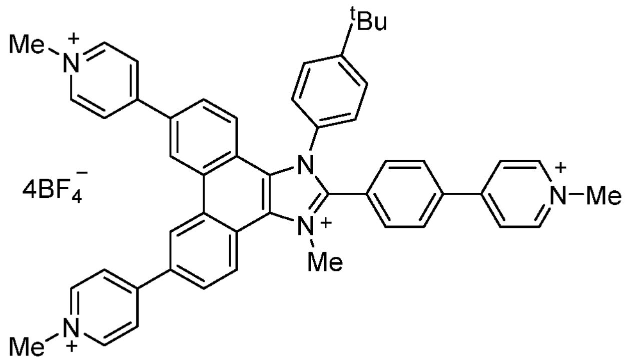

A mixture of 1-[4-(*tert*-butyl)phenyl]-6,9-di(pyridin-4-yl)-2-[4-(pyridin-4-yl)phenyl]-1*H*-phenanthro[9,10-*d*]imidazole (3.29 g, 5.0 mmol) and MeOTs (5.59 g, 30 mmol, 6.0 equiv.) was heated to 180 °C whilst stirring under argon for 16 hours, cooled, triturated with $Et_2O$, and the solid collected by vacuum filtration. The solid was ground with a pestle and mortar along with a small amount of $Et_2O$, triturated with $Et_2O$, and air drive to give a green powder. A mixture of the powder (4.91 g) and MeOTs (4.00 g, 21.5 mmol) was heated to 180 °C whilst stirring under argon for 16 hours, cooled, triturated with $Et_2O$, and the solid collected by vacuum filtration. A filtered solution of the solid in hot 1:1: Me-

OH:water was added dropwise to a stirred solution of $NaBF_4$ (6.59 g, 50 mmol, 12.0 equiv.) in water (65 mL), stirred for 30 minutes, and the precipitate collected by vacuum filtration to give the *title compound* (4.17 g, 78 %) as a khaki-green powder, $\delta_H$ (DMSO-$d_6$, 400 MHz) 1.35 (9H, s), 4.36 (3H, s), 4.39 (3H, s), 4.44 (3H, s), 4.48 (3H, s), 7.09 (1H, d, *J* = 8.8 Hz), 7.75 (2H, d, 8.6 Hz), 7.80 (2H, d, *J* = 8.6 Hz), 8.08 (2H, d, *J* = 8.4 Hz), 8.25 (1H, dd, *J* = 1.7, 8.9 Hz), 8.32 (2H, d, *J* = 8.5 Hz), 8.56 (2H, d, *J* = 6.9 Hz), 8.65 (1H, dd, *J* = 1.7, 8.8 Hz), 8.85 (2H, d, *J* = 7.1 Hz), 8.95 (2H, d, *J* = 7.1, Ar-H), 9.05 - 9.27 (7H, m), 9.88 (1H, d, *J* = 1.7 Hz) and 9.95 (1H, d, *J* = 1.7); $\delta_F$ (DMSO-$d_6$, 376 MHz) -148.25 and -148.20; $\delta_C$ (DMSO-$d_6$, 100 MHz) 31.41, 35.45, 38.48, 47.78, 47.84, 122.63, 123.35, 124.77, 125.21, 125.51, 125.67, 125.78, 126.30, 127.41, 127.88, 128.17, 128.43, 128.60, 129.09, 130.35, 130.48, 132.33, 133.04, 133.57, 133.69, 137.67, 147.13, 146.27, 146.43, 150.26, 152.92, 153.51, 153.67 and 155.48.

## 5. Benzoselenazoles

### EXAMPLE 19

**4-(Benzoselenazol-2-yl)-1-hexylpyridin-1-ium iodide**

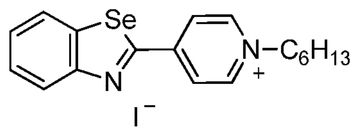

2-(Pyridin-4-yl)benzoselenazole (0.75 g, 2.89 mmol) and 1-iodohexane (1.23 mL, 8.67 mmol) were suspended in MeCN (40 mL) and then stirred at 80°C for 16 h. The reaction mixture was cooled to ambient temperature and the solvent was removed under reduced pressure. The resulting solid was triturated with acetone (20 mL), filtered, washed with acetone (20 mL) and dried under reduced pressure to give the desired product as an orange powder. Yield 1.10 g, 81 %. $\delta_H$ ($CD_3OD$, 300 MHz) 9.11 (d, *J* = 6.9 Hz, 2 H), 8.68 (d, *J* = 6.9 Hz, 2 H), 8.28 (d, *J* = 8.2 Hz, 1 H), 8.23 (d, *J* = 8.2 Hz, 1 H), 7.65 (dd, *J* = 7.3, 1.2 Hz, 1 H), 7.53 (dd, *J* = 7.3, 1.2 Hz, 1 H), 4.67 (t, *J* = 7.6, 2 H), 1.95 (quint., *J* = 7.4 Hz, 2 H), 1.55 - 1.23 (m, 6 H), 0.94 (t, *J* = 6.8 Hz, 3 H); $\delta_C$ (DMSO-$d_6$, 100 MHz) 166.72, 156.98, 151.53, 146.78, 142.04, 128.82, 128.80, 127.52, 126.90, 126.53, 62.87, 32.41, 32.32, 26.91, 23.48, 14.28.

**Compound 19: 4-(Benzoselenazol-2-yl)-1-hexylpyridin-1-ium tetrafluoroborate**

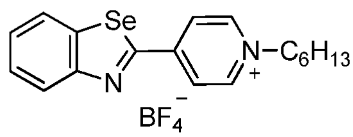

4-(Benzoselenazol-2-yl)-1-hexylpyridin-1-ium iodide (0.65 g, 1.38 mmol) was dissolved in hot MeOH (50 mL) and added dropwise through a cotton wool plug to a stirred solution of $NaBF_4$ (5.0 g) in $H_2O$ (200 mL) whereupon a pale yellow precipitate formed. The solution was stirred for 30 minutes and then filtered under reduced pressure. The resulting solid was washed with water (30 mL) and then dried under reduced pressure to give the desired product as a pale yellow powder. Yield 0.59 g, 76 %. $\delta_H$ (DMSO-$d_6$, 400 MHz) 9.20 (d, *J* = 6.7 Hz, 2 H), 8.74 (d, *J* = 6.7 Hz, 2 H), 8.39 (d, *J* = 8.0 Hz, 1 H), 8.28 (d, *J* = 8.0 Hz, 1 H), 7.66 (dd, *J* = 7.3, 0.9 Hz, 1 H), 7.55 (dd, *J* = 7.3, 0.9 Hz, 1 H), 4.64 (t, *J* = 7.4, 2 H), 1.95 (quint., *J* = 6.5 Hz, 2 H), 1.43 - 1.20 (m, 6 H), 0.87 (t, *J* = 6.7 Hz, 3 H); $\delta_C$ (DMSO-$d_6$, 100 MHz) 166.57, 154.99, 148.64, 145.71, 140.40, 127.61, 127.34, 126.49, 125.91, 125.21, 60.57, 30.68, 30.56, 25.05, 21.84, 13.81; $\delta$s (DMSO-$d_6$, 128 MHz) -1.3; $\delta_F$ (DMSO-$d_6$, 376 MHz) -148.27 (br. m, 4 F).

### EXAMPLE 20

**4-(Benzoselenazol-2-yl)-1-phenylpyridin-1-ium trifluoromethanesulfonate**

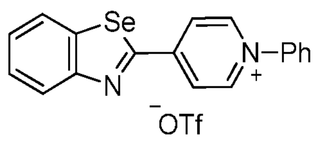

2-(Pyridin-4-yl)benzoselenazole (1.00 g, 3.86 mmol), diphenyliodonium triflate (2.49 g, 5.79 mmol) and $Cu(OAc)_2 \cdot H_2O$ (116 mg, 0.58 mmol) were dissolved in DMF (30 mL) under $N_2$ and the reaction mixture was stirred at 100°C for 16 h. The reaction mixture was cooled to room temperature and the solvent was removed under reduced pressure. The resulting yellow solid was triturated in hot MeOH, filtered and dried under reduced pressure to give the desired product as a yellow powder. Yield 1.70 g, 91 %. $\delta_H$ (DMSO-$d_6$, 400 MHz) 9.46 (d, *J* = 6.9 Hz, 2 H), 8.86 (d, *J* = 6.9 Hz, 2 H), 8.42 (d, *J* = 7.8 Hz, 1 H), 8.33 (d, *J* = 7.8 Hz, 1 H), 8.00 - 7.90 (m, 2 H), 7.84 - 7.74 (m, 3 H), 7.69 (dd, *J* = 7.4, 1.1 Hz, 1 H), 7.58 (dd, *J* = 7.4, 1.1 Hz, 1 H); $\delta_C$ (DMSO-$d_6$, 100 MHz) 166.28, 155.14, 149.35, 145.83, 142.39, 140.80, 131.40, 130.24, 127.75, 127.55, 126.60, 126.14, 124.97, 124.68, 120.65 (d, $J_{C-F}$ = 320.4 Hz); $\delta_F$ (DMSO-$d_6$, 376 MHz) -77.76 (s, 3 F).

**Compound 20: 4-(Benzoselenazol-2-yl)-1-phenylpyridin-1-ium tetrafluoroborate**

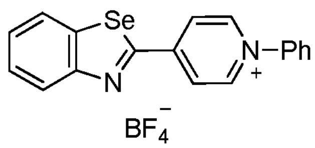

4-(Benzoselenazol-2-yl)-1-phenylpyridin-1-ium trifluoromethanesulfonate (1.00 g, 2.06 mmol) was dissolved in hot MeOH (50 mL) and added dropwise through a cotton wool plug to a stirred solution of $NaBF_4$ (5.0 g) in $H_2O$ (200 mL) whereupon a yellow precipitate formed. The solution was stirred for 30 minutes and then filtered under reduced pressure. The resulting solid was washed with water (30 mL) and then dried under reduced pressure to give the desired product as a yellow powder. Yield 0.61 g, 70 %. $\delta_H$ (DMSO-$d_6$, 400 MHz) 9.45 (d, *J* = 6.9 Hz, 2 H), 8.55 (d, *J* = 6.9 Hz, 2 H), 8.41 (d, *J* = 7.8 Hz, 1 H), 7.32 (d, *J* = 7.8 Hz, 1 H), 8.00 - 7.87 (m, 2 H), 7.83 - 7.72 (m, 3 H), 7.67 (dd, *J* = 7.4, 1.1 Hz, 1 H), 7.57 (dd, *J* = 7.4, 1.1 Hz, 1 H); $\delta_C$ (DMSO-$d_6$, 100 MHz) 166.30, 155.16, 149.36, 145.84, 142.40, 140.82, 131.43, 130.27, 127.77, 127.57, 126.61, 126.16, 124.99, 124.69; $\delta_B$ (DMSO-$d_6$, 128 MHz) -1.3; $\delta_F$ (DMSO-$d_6$, 376 MHz)-148.21 (br. m, 4 F).

**EXAMPLE 21**

**Compound 21: 4-(Benzoselenazol-2-yl)-1-phenylpyridin-1-ium hexafluorophosphate**

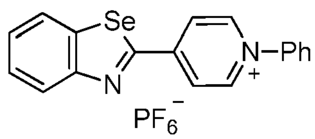

4-(Benzoselenazol-2-yl)-1-phenylpyridin-1-ium trifluoromethanesulfonate (0.20 g, 0.47 mmol) was dissolved in hot MeOH (20 mL) and added dropwise through a cotton wool plug to a stirred solution of $NH_4PF_6$ (0.50 g) in $H_2O$ (100 mL) whereupon a yellow precipitate formed. The solution was stirred for 30 minutes and then filtered under reduced pressure. The resulting solid was washed with water (30 mL) and then dried under reduced pressure to give the desired product as a yellow powder. Yield 0.13 g, 71 %. $\delta_H$ [$(CD_3)_2CO$, 400 MHz] 9.52 (d, *J* = 6.3 Hz, 2 H), 8.98 (d, *J* = 6.3 Hz, 2 H), 8.38 (d, *J* = 8.5 Hz, 1 H), 8.36 (d, *J* = 8.5 Hz, 1 H), 8.13 - 7.95 (m, 2 H), 7.92 - 7.78 (m, 3 H), 7.73 (t, *J* = 7.6 Hz, 1 H ), 7.61 (t, *J* = 7.6 Hz, 1 H); $\delta_C$ [$(CD_3)_2CO$, 100 MHz] 166.22, 156.65, 151.54, 146.67, 143.78, 141.91, 132.64, 131.45, 128.74, 128.71, 127.39, 126.99, 126.34, 125.43; $\delta_P$ [$(CD_3)_2CO$, 162 MHz] -144.27 (sept., *J* = 707.8 Hz, 1 P); $\delta_F$ [$(CD_3)_2CO$, 376 MHz] -72.51 (d, *J* = 707.8 Hz, 6 F).

## 6. Benzoxazoles and Benzoisoxazoles

**EXAMPLE 22**

**2-(Pyridin-4-yl)-2,3-dihydrobenzoxazole**

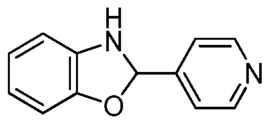

A solution of 2-aminophenol (8.00 g, 73.4 mmol) and pyridine-4-carboxaldehyde (7.85 g, 73.4 mmol) in EtOH (350 mL) was stirred under air for 5 days. The solvent was reduced in volume and the resulting solid filtered off, washed with EtOH (20 mL) and air dried to give the *title compound* (13.36 g, 92 %) as an orange powder, $\delta_H$ ($CDCl_3$, 400 MHz) 6.93 (1H, app. t, *J* = 7.7 Hz), 7.04 (1H, app. d, *J* = 8.1 Hz), 7.22 - 7.31 (2H, m), 7.35 (1H, app. d, *J* = 8.0 Hz), 7.76 (2H, bd, *J* = 4.5 Hz), 8.69 (1H, s) and 8.78 (2H, bs).

**2-(Pyridin-4-yl)benzoxazole**

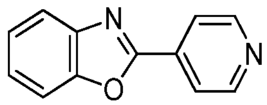

2,3-Dichloro-5,6-dicyano-1,4-benzoquinone (1.15g, 5 mmol) was added in one portion to a solution of 2-(pyridin-4-yl)-2,3-dihydrobenzoxazole (1.00 g, 5 mmol) in DCM (50 mL) with stirring. Stirring was continued for 1 h and $Et_3N$ (5 mL) was added. The resulting solution was filtered through silica using DCM (100 - 0 % in EtOAc) as eluent. The first band was collected to give the *title compound* (0.43 g, 43 %) as a pale yellow powder. The second band was collected to give unreacted starting material (0.28g, 28 %), $\delta_H$ ($CDCl_3$, 400 MHz) 7.39 - 7.47 (2H, m), 7.61 - 7.66 (1H, m), 7.81 - 7.85 (1H, m), 8.10 (2H, dd, *J* = 1.6 and 4.6 Hz) and 8.83 (2H, dd, *J* = 1.6 and 4.6 Hz).

**4-(Benzoxazol-2-yl)-1-hexylpyridin-1-ium iodide**

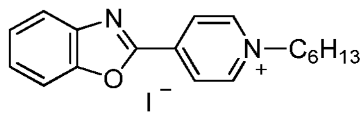

A solution of 2-(pyridin-4-yl)benzoxazole (1.00 g, 5.1 mmol) and 1-iodohexane (3.24 g, 15.3 mmol) in MeCN (30 mL) was heated at reflux in the dark under $N_2$ with stirring. After 16 h, the resulting mixture was cooled and diluted with $Et_2O$ (50 mL). The precipitate was filtered off, washed with $Et_2O$ (3 × 10 mL) and air dried to give the *title compound* (2.06 g, 99 %) as a yellow powder.

**Compound 22: 4-(Benzoxazol-2-yl)-1-hexylpyridin-1-ium tetrafluoroborate**

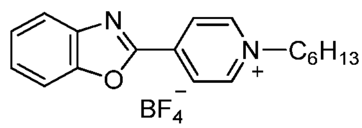

A solution of 4-(benzoxazol-2-yl)-1-hexylpyridin-1-ium iodide (2.01 g, 4.9 mmol) in warm MeOH/water (50 mL, 1:1) was added dropwise to a solution of $NaBF_4$ (5.42 g, 40 mmol) in water (100 mL) with stirring. Stirring was continued for 0.5 h and the resulting precipitate filtered off, washed with water (3 × 10 mL) and air dried to give the *title compound* (1.77. g, 98 %) as a colourless powder, $\delta_H$ ($CD_3OD$, 400 MHz) 0.89 - 0.97 (3H, bt, *J* = 5.7 Hz), 1.30 - 1.51 (6H, m), 2.00 - 2.14 (2H, m), 4.69 (2H, bt, *J* = 7.4 Hz), 7.50 - 7.65 (2H, bm), 7.83 (1H, bd, *J* = 8.2 Hz), 8.93 (1H, bd, *J* = 8 Hz), 8.78 (2H, bd, *J* = 5.5 Hz) and 9.16 (2H, bd, *J* = 5.5 Hz).

**EXAMPLE 23**

**4-(Benzoxazol-2-yl)-1-phenylpyridin-1-ium triflate**

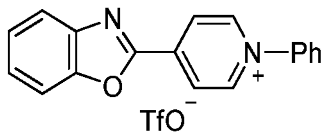

A mixture of 2-(pyridin-4-yl)benzoxazole (1 g, 5.1 mmol), diphenyliodonium triflate (3.29 g, 7.6 mmol), $Cu(OAc)_2.H_2O$ (100 mg, 10 mol%) in dry DMF (50 mL) was heated at 100 °C for 16 h, cooled and the solvent removed under reduced pressure. The residue was triturated with $Et_2O$ (50 mL), washed with $Et_2O$ (3 × 10 mL) and air dried. The resulting solid was triturated with hot MeOH (20 mL), cooled, filtered and air dried to give the *title compound* (1.86 g, 86 %) as a colourless powder, $\delta_H$ ($CD_3OD$, 400 MHz) 7.54 - 7.70 (2H, bm), 7.76 - 8.00 (7H, bm), 8.93 (2H, bd, *J* = 5.9 Hz) and 9.42 (2H, bd, *J* = 5.9 Hz).

**Compound 23: 4-(Benzoxazol-2-yl)-1-phenylpyridin-1-ium tetrafluoroborate**

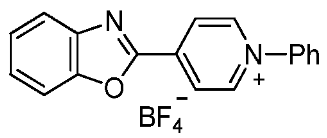

A solution of 4-(benzoxazol-2-yl)-1-phenylpyridin-1-ium triflate (0.25 g, 0.59 mmol) in warm MeOH (10 mL) was added dropwise to a solution of $NaBF_4$ (0.65 g, 5.9 mmol) in water (30 mL) with stirring. The resulting precipitate was filtered, washed with water (2 × 3 mL), dissolved in warm MeOH (10 mL) and added dropwise to a solution of $NaBF_4$ (0.65 g, 5.9 mmol) in water (30 mL) and stirred for 0.5 h. The resulting precipitate was filtered off, washed with water (2 × 3 mL) and air dried to give the *title compound* (0.20 g, 95 %) as a pale yellow powder, $\delta_H$ (DMSO-$d_6$, 400 MHz) 7.56 - 7.62 (1H, m), 7.64 - 7.70 (1H, m), 7.74 - 7.82 (3H, m), 7.91 - 8.01 (3H, m), 8.05 (1H, d, *J* = 7.9 Hz), 8.87 (2H, bd, *J* = 6.8 Hz) and 9.53 (2H, bd, *J* = 6.8 Hz).

**EXAMPLE 24:**

**4-(2-Methoxybenzoyl)pyridine**

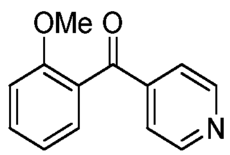

A freshly prepared solution of 2-methoxyphenylmagnesium bromide (40 mL, ca. 1.38 M, 55.4 mmol) was added to a stirred solution of 4-cyanopyridine (2.78 g, 26.7 mmol) in dry THF (20 mL) which had been cooled to 0°C under $N_2$.

After the addition was complete the reaction mixture was stirred and heated at 50°C under $N_2$ for 16 h. The solution was then cooled to 0°C and $H_2O$ (20 mL) was slowly added. The reaction mixture was then evaporated to dryness under reduced pressure and then 2M HCl (100 mL) was added, and the solution was heated at 80 °C for 8 h. The reaction mixture was then cooled to room temperature and the solution was washed with EtOAc (2 × 100 mL). The aqueous phase was basified with NaOH (5 M) and extracted with DCM (3 × 100 mL). The organic layers were combined, dried ($Na_2SO_4$), filtered and the solvent was removed under reduced pressure. The resulting residue was chromatographed on silica gel [eluent = 1:9 graduated to 1:1 EtOAc:Petroleum ether]. The solvent of the resulting column fractions was removed under reduced pressure to give the *title compound* as a yellow oil. Yield 4.60 g, 82 %. $\delta_H$ ($CDCl_3$, 400 MHz) 8.75 (2H, dd, *J* = 4.4, 1.6 Hz), 7.57 -7.51 (3H, m), 7.46 (1H, dd, *J* = 7.6, 1.8 Hz), 7.07 (1H, td, *J* = 7.55, 0.9 Hz), 6.99 (1H, d, *J* = 8.3 Hz) and 3.68 (3H, s); $\delta_C$ ($CDCl_3$, 100 MHz) 195.6, 158.0, 150.5, 144.8, 133.5, 130.5, 127.3, 122.4, 121.0, 111.7 and 55.6.

**3-(Pyridin-4-yl)-1,2-benzisoxazole**

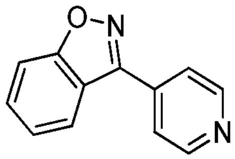

4-(2-Methoxybenzoyl)pyridine (3.60 g, 16.8 mmol) was dissolved in dry DCM (100 mL) and cooled to 0 °C under $N_2$. $BBr_3$ (8.42 g, 3.24 mL, 33.6 mmol) was added dropwise and the reaction mixture was stirred for 16 h. The reaction mixture was added to ice, neutralized with $NaHCO_3$ and stirred for 1 h. The reaction mixture was then extracted with DCM (2 × 200 mL) and the organic layers were combined, dried ($Na_2SO_4$), filtered, and the solvent removed under reduced pressure. The resulting residue was chromatographed on silica gel [eluent = 1:9 graduated to 4:6 EtOAc:Petroleum ether]. The solvent of the resulting column fractions was removed under reduced pressure to give impure 4-(2-hydroxybenzoyl)pyridine (2.60 g) as a yellow solid which was used in the subsequent reactions without further purification. The impure 4-(2-hydroxybenzoyl)pyridine (2.60 g) was dissolved in 7N ammonia in methanol (30 mL) and stirred at room for 48 h to give an orange coloured solution. The reaction mixture was then evaporated to dryness under reduced pressure and redissolved in dry THF (40 mL) under $N_2$. *N*-Chlorosuccinimide (2.60 g, 19.5 mmol) and $K_2CO_3$ (3.60g, 138 mmol) were then added to the reaction mixture which was then stirred for 16 h at room temperature under $N_2$. The reaction mixture was then diluted with diethyl ether (100 mL) and quenched with water (100 mL). The organic layer was separated and the aqueous layer was extracted with diethyl ether (2 × 100 mL). The organic layers were combined, dried ($Na_2SO_4$), filtered and the solvent removed under reduced pressure. The resulting residue was chromatographed on silica gel [eluent = 1:9 graduated to 3:7 EtOAc:petroleum ether]. The solvent of the resulting column fractions was removed under reduced pressure to give a pale yellow solid that was then triturated with pentane and dried under reduced pressure to give the *title compound* as an off-white solid. Yield 1.2 g, 36 %. $\delta_H$ ($CDCl_3$, 400 MHz) 8.84 (2H, dd, *J* = 4.5, 1.5 Hz), 7.95 (1H, dt, *J* = 8.0, 0.9 Hz), 7.89 (2H, dd, *J* = 4.4, 1.6 Hz) 7.70 (1H, dt, *J* = 8.4, 0.8 Hz), 7.67 (1H, ddd, *J* = 8.5, 7.0, 1.0 Hz) and 7.44 (1H, ddd, *J* = 8.5, 7.0, 1.0 Hz); $\delta_C$ ($CDCl_3$, 100 MHz) 164.3, 155.4, 150.9, 136.7, 130.4, 124.6, 122.3, 1217, 119.9, 110.6.

**Compound 24: 4-(1,2-Benzisoxazol-3-yl)-1-phenylpyridin-1-ium hexafluorophosphate**

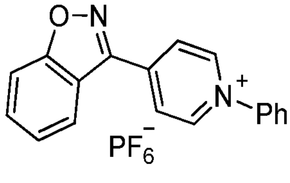

3-(Pyridin-4-yl)-1,2-benzisoxazole (1.00 g, 5.1 mmol) diphenyliodonium trifluoromethanesulfonate (3.29 g, 7.64 mmol) and $Cu(OAc)_2$ . $H_2O$ (0.1 g, 0.51 mmol) were dissolved in DMF (40 mL) under $N_2$ and the reaction mixture was stirred at 100°C for 16 h. The reaction mixture was cooled to room temperature and the solvent was removed under

reduced pressure. The resulting pale green solid was triturated with diethyl ether (3 $\times$ 50 mL), filtered and dried under reduced pressure. The resulting hydroscopic pale green solid was dissolved in hot MeOH:$H_2O$ (ca. 2:1, 150 mL) and added dropwise through a cotton wool plug to a stirred solution of $NH_4PF_6$ (17.5.0 g) in $H_2O$ (350 mL) whereupon a pale yellow precipitate formed. The suspension was stirred for 30 minutes and then filtered under reduced pressure. The resulting solid was washed with water (50 mL) and then dried under reduced pressure to give a pale green powder. The solid was then dissolved in hot acetone:$H_2O$ (ca. 2:1, 200 mL) and added dropwise through a cotton wool plug back into the filtrate (which had been reduced by ca. 20% under vacuum) to give a pale green precipitate. The resulting suspension was then filtered under reduced pressure and the solid was washed with water (50 mL) and then dried under reduced pressure to give a pale green powder. The green powder was triturated with MeOH (50 mL), filtered and dried under reduced pressure to give the *title compound* as a colourless powder. Yield 1.17 g, 55 %. $\delta_H$ (DMSO-$d_6$, 400 MHz) 9.60 (2H, d, $J$ = 7.0 Hz), 8.96 (2H, d, $J$ = 6.9 Hz), 8.43 (1H, d, $J$ = 8.1 Hz), 8.09 (1H, d, $J$ = 8.6 Hz) 8.05 - 7.98 (2H, m), 7.93 (1H, ddd, $J$ = 8.5, 7.1, 1.0 Hz), 7.89 - 7.79 (3H, m) and 7.72 (1H, ddd, $J$ = 8.5, 7.3, 0.5 Hz); $\delta_C$ (DMSO-$d_6$, 100 MHz) 164.1, 153.3, 146.0, 144.1, 142.5, 131.6, 131.5, 130.3, 126.2, 125.8, 124.8, 122.4, 118.6 and 110.7; $\delta_F$ (DMSO-$d_6$, 376 MHz) -170.13 (6F, d, $J$ = 711.0 Hz).

**Evaluation of oxido-reduction potentials and absorption spectra of the compounds of the invention**

Method for measuring oxido-reduction potentials

The oxido-reduction potentials of the compounds are measured by cyclic voltammetry with 3 electrodes.
The 3 electrodes used are:

- 1 Platinum working electrode
- 1 Platinum auxiliary or counter electrode
- 1 Platinum reference electrode which is immersed into a solution constituted of 0.01 M $AgNO_3$ + 0.1 M TBAP (tetrabutylammonium perchlorate) in acetonitrile.

The scan rate of the potential is fixed to 100mV/s.
$E_1^{red}$ corresponds to the first reduction peak of the analyzed compound.
$E_2^{red}$ corresponds to the second reduction peak of the analyzed compound.
$E_1^{\ 1/2}$ corresponds to the oxido-reduction potential of an oxidant/reductor system as calculated below:

$$E_1^{\ 1/2} = (E_1^{red} + E_1^{ox})/2$$

wherein $E_1^{ox}$ corresponds to the first oxidation peak of the analyzed compound.
$\Delta E^{red}$ corresponds to the difference between $E_1^{red}$ and $E_2^{red}$ as calculated below:

$$\Delta E^{red} = |E_2^{red}| - |E_1^{red}|.$$

The indicated potential values are the first reduction potentials for the compounds, with respect to the standard hydrogen reference electrode (SHE).
The analyzed solution comprises 0.005 M of the compound to be analyzed and 0.25 M of $TBABF_4$ salt in propylene carbonate as solvent.

Method for measuring absorption spectra

This solution is introduced into a quartz cell where.
This solution is introduced into a quartz cell where at least one working electrode in the form of a platinum grid is placed to colour the analysed compound on this electrode. The absorption spectrum of the analysed compound in the time domain is measured by uv-visible spectroscopy.
The results for each of the synthesized compounds are indicated in Table 1 below. $E_1^{red}$ corresponds to the first reduction potential. The colour indicated in Table 1 is the visual colour perceived by emmetropic eyes under day light conditions. It should be noted that the $\lambda_{max}$ value just gives an approximate indication of the colour of a particular compound. However, as a consequence of the broad nature of the absorption bands, the whole absorption spectrum has to be taken into account in order to understand the final perceived colour of any one compound.
In comparison, Table 2 shows the results obtained for 3 known compounds.

By comparing compound 2 (orange) and compounds 6-7 (red) of the invention with known compound COMP1 (green), it appears that replacing one phenyl pyridinium group by one substituted benzimidazolium or substituted benzothiazolium group reduces the maximum absorption wavelength in the visible range from 645 nm to 550 nm or below. At their activated state these molecules are red or orange rather than green.

By comparing compounds 1, 10, 16 (orange), 3, 4, 5 (purple), 8 (green), 9, 11 (yellow/green), 13-14 (yellow), with known compounds COMP2 (blue) and COMP3 (blue), it appears that including different groups like imidazolium, benzimidazolium, benzothiazolium, alone or included in more complex molecular structures have also this effect of shifting the maximum absorption wavelength to lower values. These groups can be either introduced in between two alkyl bi pyridinium groups or could replace the central pyridinium group of a ter pyridinium. The molecules thus obtained are yellow, orange, red, green or purple rather than blue.

The results show that the known compound COMP3, which contains a phenyl group in between two alkyl bi pyridinium groups, is also orange with an activation potential of - 1.21V. The compounds of the invention have a similar or lower activation potential than COMP3, except compounds 11 and 14.

Table 1

| Cpd | Structure | $E_1^{1/2}$ (V) | E1 1/2 (V) vs SHE | reversi bility | color | λ 1 max (nm) | λ 2 max (nm) | λ cut (nm) clear | $E_1^{red}$ peak potential (V) | E1 peak potential (V) vs SHE | $E_2^{red}$ peak potenti al (V) | E2 peak potential (V) vs SHE | $\Delta E^{red}$ |
|---|---|---|---|---|---|---|---|---|---|---|---|---|---|
| 1 | 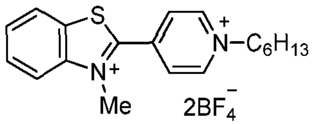 | -0.62 | -0.078 | Y | orange | 421 | 530 | 399 | -0.65 | -0.108 | -0.92 | -0.378 | -0.27 |
| 2 | 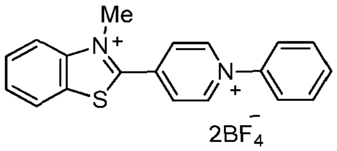 | -0.53 | 0.012 | Y | orange | 446 | 550 | 435 | -0.57 | -0.028 | -0.78 | -0.238 | -0.21 |
| 3 | 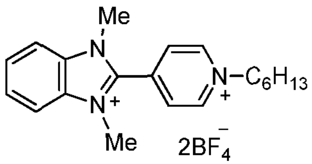 | - | NA | N | purple | 390 | wide absor ption | 380 | -1 | -0,458 | - | NA | NA |
| 4 | 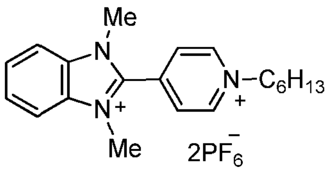 | -0.98 | -0.438 | Y | purple | 390 | 525 | 382 | -1 | -0.458 | -1.35 | -0.808 | -0.35 |
| 5 | 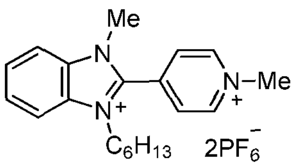 | - | NA | N | purple | 402 | 530 | 373 | -0.48 | 0.062 | -0.86 | -0.318 | -0.38 |
| 6 | 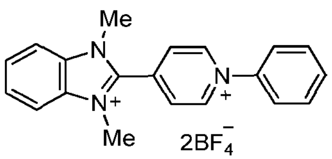 | -0.8 | -0.258 | Y | red | 416 | 530 | 397 | -0.87 | -0.328 | -1.22 | -0.678 | -0.35 |

(continued)

| Cpd | Structure | $E_1^{1/2}$ (V) | E1 1/2 (V) vs SHE | reversi bility | color | λ 1 max (nm) | λ 2 max (nm) | λ cut (nm) clear | $E_1^{red}$ peak potential (V) | E1 peak potential (V) vs SHE | $E_2^{red}$ peak potenti al (V) | E2 peak potential (V) vs SHE | $\Delta E^{red}$ |
|---|---|---|---|---|---|---|---|---|---|---|---|---|---|
| 7 | 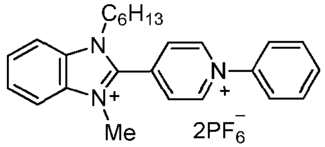 | -0.82 | -0.278 | Y | red | 415 | 530 | 393 | -0.9 | -0.358 | -1.24 | -0.698 | -0.34 |
| 8 | 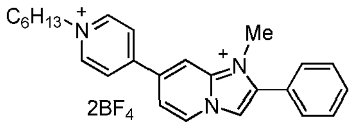 | -1.07 | -0.528 | Y | green | 437 | 650 | 473 | -1.1 | -0.558 | -1.5 | -0.958 | -0.4 |
| 9 | 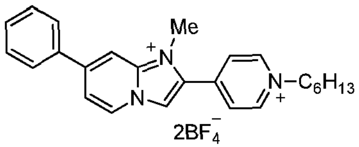 | -1.2 | -0.658 | Y | yellow | 412 | - | 412 | -1.24 | -0.698 | -1.65 | -1.108 | -0.41 |
| 10 | 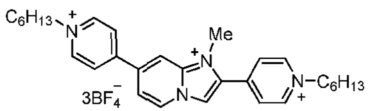 | -0.96 | -0.418 | Y | orange | 470 | 580 | | | 0.542 | | 0.542 | 0 |
| 11 | 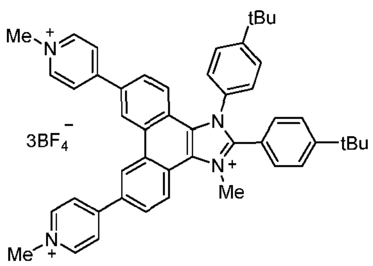 | -1.39 | -0.848 | Y | green | 410 | 640 | 450 | -1.45 | -0.908 | - | NA | NA |

(continued)

| Cpd | Structure | $E_1^{1/2}$ (V) | E1 1/2 (V) vs SHE | reversi bility | color | λ1 max (nm) | λ2 max (nm) | λ cut (nm) clear | $E_1^{red}$ peak potential (V) | E1 peak potential (V) vs SHE | $E_2^{red}$ peak potenti al (V) | E2 peak potential (V) vs SHE | $\Delta E^{red}$ |
|---|---|---|---|---|---|---|---|---|---|---|---|---|---|
| 12 | 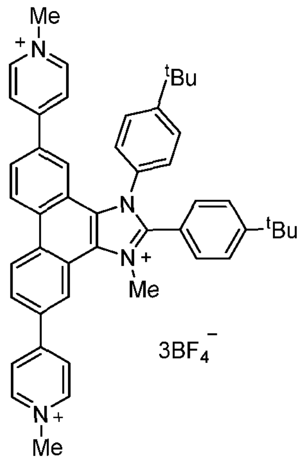 | -1.27 | -0.728 | Y | orange | 486 | 750 | 439 | -1.32 | -0.778 | - | NA | NA |
| 13 | 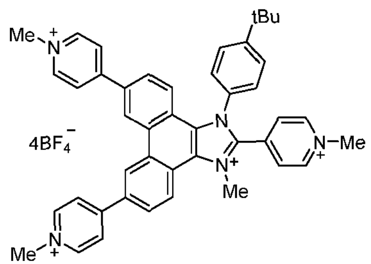 | | 0.542 | N | yellow | 443 | 650 | 456 | -0.98 | -0.438 | -1.48 | -0.938 | -0.5 |
| 14 | 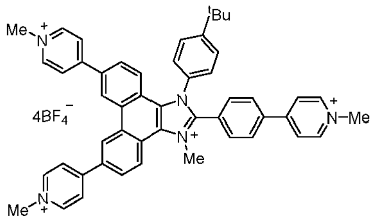 | -1.4 | -0.858 | Y | yellow | 409 | 750 | 426 | -1.53 | -0.988 | - | NA | NA |

(continued)

| Cpd | Structure | $E_1^{1/2}$ (V) | E1 1/2 (V) vs SHE | reversibility | color | λ 1 max (nm) | λ 2 max (nm) | λ cut (nm) clear | $E_1^{red}$ peak potential (V) | E1 peak potential (V) vs SHE | $E_2^{red}$ peak potenti al (V) | E2 peak potential (V) vs SHE | $\Delta E^{red}$ |
|---|---|---|---|---|---|---|---|---|---|---|---|---|---|
| 15 | 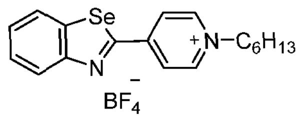 | -1,12 | -0,578 | Y | red | 533 | | 437 | -1,15 | -0,608 | - | NA | NA |
| 16 | 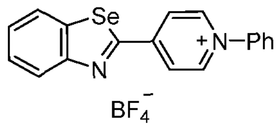 | -0,94 | -0,398 | Y | red | 541 | | 458 | -1,02 | -0,478 | - | NA | NA |
| 17 | 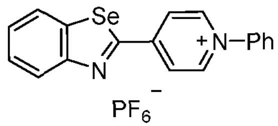 | -1 | -0,458 | Y | red | 545 | | 463 | -1 | -0,458 | - | NA | NA |
| 18 | 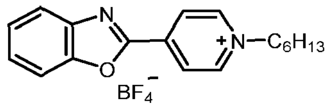 | -1,14 | -0,598 | Y | orange | 405 | 496 | 407 | -1,17 | -0,628 | - | NA | NA |
| 19 | 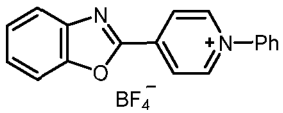 | -0,97 | -0,428 | Y | orange | 405 | 495 | 425 | -1 | -0,458 | - | NA | NA |

Table 2

| Ref | Structure | $E_1^{1/2}$ (V) | E11/2 (V) vs N SHE | reversi bility | color | λ 1 max (nm) | λ 2 max (nm) | λ cut (nm) clear | $E_1^{red}$ peak potential (V) | E1 peak potential (V) vs SHE | $E_2^{red}$ peak potenti al (V) | E2 peak potential (V) vs SHE | $\Delta E^{red}$ |
|---|---|---|---|---|---|---|---|---|---|---|---|---|---|
| COMP 1 | 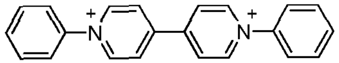 $2BF_4^-$ | -0,62 | -0,078 | Y | green | 442 | 645 | 395 | -0,64 | -0,098 | -0,93 | -0,388 | -0,29 |
| COMP 2 | 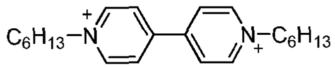 $2\ BF_4^-$ | -0,79 | -0,248 | Y | blue | 399 | 608 | 316 | -0,84 | -0,298 | -1,2 | -0,658 | -0,36 |
| COMP 3 | 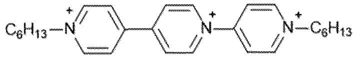 $3BF_4^-$ | -0,41 | 0,132 | Y | blue | 400 | 620 | 410 | -0,47 | 0,072 | -0,69 | -0,148 | -0,22 |

**Claims**

1. An electrochromic compound represented by formula (I):

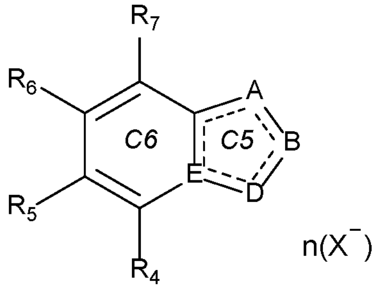

(I)

wherein:

A is N, $^+N$, $N-R_1$, $^+N-R_1$ or $C-R_1$;
B is $C-R_2$, S, Se, O, N, $N-R_2$ or $^+N-R_2$;
D is $C-R_3$, N, S, O, Se, $N-R_3$ or $^+N-R_3$;
E is C, N or $^+N$;
$\mathbf{R_1}$ is H, Ci-Cis alkyl, aryl or Z;
$\mathbf{R_2}$ is H, Ci-Cis alkyl, aryl, **Z** or aryl substituted by **Z**;
$\mathbf{R_3}$ is H or Ci-Cis alkyl, aryl or **Z**;
$\mathbf{R_4}$ is H, Ci-Cis alkyl, aryl or **Z**;
$\mathbf{R_5}$ is H, Ci-Cis alkyl, aryl or **Z**;
$\mathbf{R_6}$ is H, Ci-Cis alkyl, aryl or **Z**;
$\mathbf{R_7}$ is H, Ci-Cis alkyl, aryl or **Z**;
$\mathbf{R_7}$ and $\mathbf{R_6}$ and/or $\mathbf{R_6}$ and $\mathbf{R_5}$ and/or $\mathbf{R_5}$ and $\mathbf{R_4}$ may form together an aromatic ring or heteroaromatic ring fused to the six-membered(hetero)cyclic core (Cycle C6) they are attached to, optionally substituted by **Z**,
wherein **Z** is

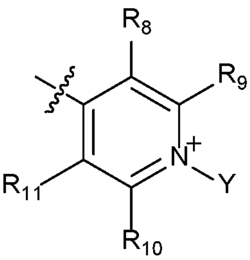

;

with **Y** being $C_1$-$C_6$ alkyl or aryl;
$\mathbf{R_8}$, $\mathbf{R_9}$, $\mathbf{R_{10}}$ and $\mathbf{R_{11}}$ are independently selected from H and Ci-Cis alkyl;
$\mathbf{R_8}$ and $\mathbf{R_9}$ or $\mathbf{R_{10}}$ and $\mathbf{R_{11}}$ may form an aromatic ring fused to the pyridinium group they are attached to,
when **B** = C-Z, and when **A**=$^+N$, $\mathbf{R_8}$ or $\mathbf{R_{11}}$ may form with **A** a ring, aromatic or not, fused with the five-membered heterocyclic ring (Cycle C5) **A** is attached to,
**n** is selected to counterbalance the number of positive charges;
**X** is a counterion;
- - - - - -is a single bond or a double bond;
**with the 3 following provisos**:

1) cycle C5 is a five membered heterocyclic ring with 2 of **A**, **B**, **D** and **E** being independently selected from: N, $N-R_1$, $^+N-R_1$, $N-R_2$, $^+N-R_2$, $N-R_3$, $^+N-R_3$, S, Se and O;
2) cycle C5 and Cycle C6 form a conjugated system; and
3) at least one of $\mathbf{R_1}$, $\mathbf{R_2}$, $\mathbf{R_3}$, $\mathbf{R_4}$, $\mathbf{R_5}$, $\mathbf{R_6}$ or $\mathbf{R_7}$ is **Z** or at least $\mathbf{R_7}$ and $\mathbf{R_6}$ form together an aromatic ring substituted by **Z** or at least $\mathbf{R_5}$ and $\mathbf{R_6}$ form together an aromatic ring substituted by **Z** or at least $\mathbf{R_5}$ and $\mathbf{R_4}$ form together an aromatic ring substituted by **Z;**

and wherein said compound of formula (I) is selected from those wherein:

- **A** is $^+$N-$R_1$; **B** is C-$R_2$; **D** is S or N-$R_3$; **E** is C; or
- **A** is $^+$N-$R_1$; **B** is C-$R_2$; **D** is C-$R_3$; **E** is N; or
- **A** is N; **B** is C-$R_2$; **D** is Se or O; **E** is C; or
- **A** is C-$R_1$; **B** is N or $^+$N-$R_2$; **D** is S; **E** is C; or
- **A** is C-$R_1$; **B** is N, **D** is O; **E** is C; or
- **A** is C-$R_1$; **D** is N, $^+$N-$R_3$, S, Se or O; **E** is C; or
- **B** is N or $^+$N-$R_2$; **A** is C-$R_1$; **D** is C-$R_3$; **E** is N; or
- **D** is $^+$N-$R_3$ or N; **A** is C-$R_1$; **B** is C-$R_2$; **E** is N; or
- **E** is $^+$N; **A** is O, S, or Se; **B** is C-$R_2$; **D** is C-$R_3$;

and wherein said electrochromic compound of formula (I) **is not** one of the following compounds:
a compound of formula $D_1$:

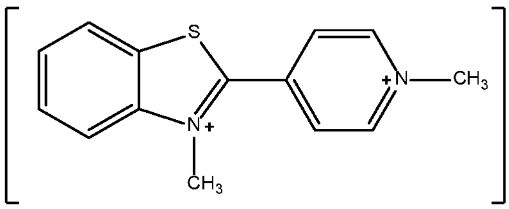

$2H_3CSO_4^-$

($D_1$)

or a compound of formula D2:

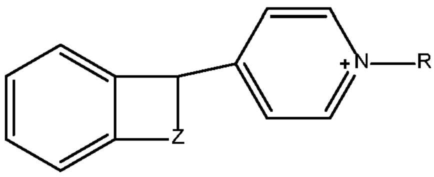

$X^-$

($D_2$)

wherein R is lower alkyl having one to four carbon atoms selected from the group consisting of methyl, ethyl, propyl, isopropyl, butyl and isobutyl; Z is a trivalent radical selected from the group consisting of:

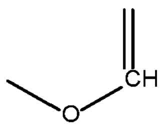

, and

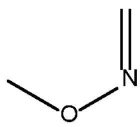

;

and $X^-$ is a monovalent pharmaceutically acceptable anion selected from the group consisting of chloride, bromide, iodide and fluoride;

or one compound selected from the group consisting of:

1-methyl-4-(1,2-benzisoxazol-3-yl)pyridinium iodide,
1-ethyl-4-(1,2-benzisoxazol-3-yl)pyridinium chloride,
1-methyl-4-(3-benzofuranyl)-pyridinium iodide,
1-methyl-4-(3-benzofuranyl)-pyridinium chloride, and
1-butyl-4-(3-benzofuranyl)pyridinium bromide.

**2.** The compound of formula (I) according to claim 1, wherein said compound is represented by formulae (II), (III), (IV), (V), (VI), (VII), (VIII), (IX) or (X):

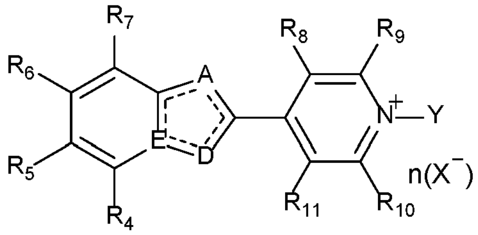

(II)

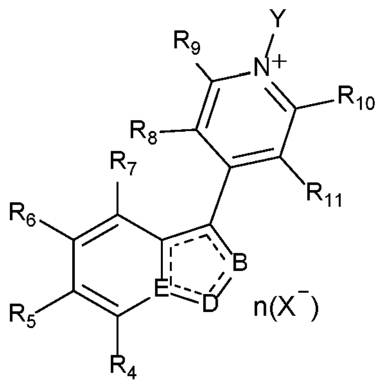

(III)

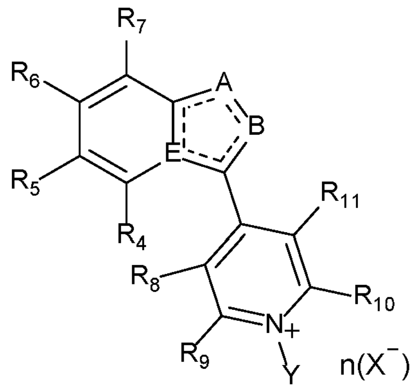

(IV)

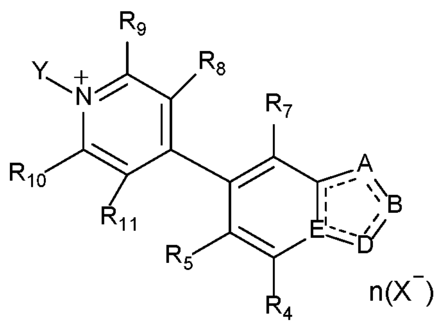

(V)

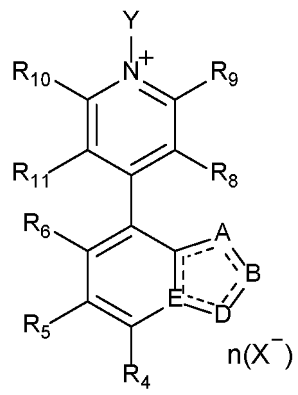

(VI)

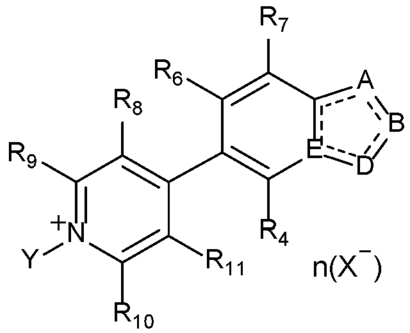

(VII)

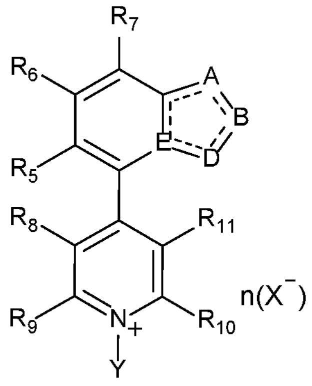

(VIII)

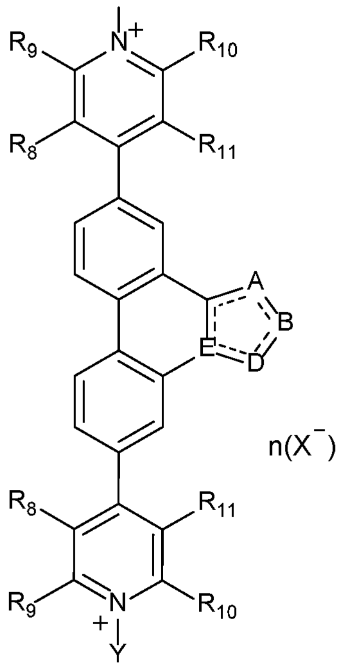

(IX)

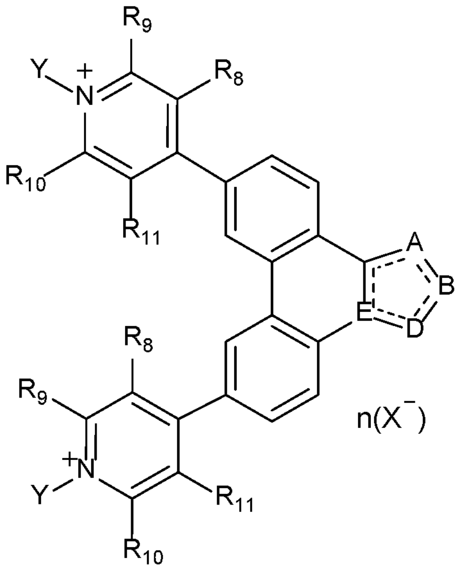

(X)

wherein **A**, **B**, **D**, **E**, $\mathbf{R_1}$, $\mathbf{R_2}$, $\mathbf{R_3}$, $\mathbf{R_4}$, $\mathbf{R_5}$, $\mathbf{R_6}$, $\mathbf{R_7}$, $\mathbf{R_8}$, $\mathbf{R_9}$, $\mathbf{R_{10}}$ and $\mathbf{R_{11}}$, when present, are as defined in claim 1; and wherein **Z**, **Y**, **n** and **X** are also as defined in claim 1.

**3.** The compound of formula (II) according to claim 1 wherein

**A** is $^+N\text{-}R_1$;
**D** is S;
**E** is C;
and $\mathbf{R_1}$, $\mathbf{R_4}$, $\mathbf{R_5}$, $\mathbf{R_6}$, $\mathbf{R_7}$, $\mathbf{R_8}$, $\mathbf{R_9}$, $\mathbf{R_{10}}$, $\mathbf{R_{11}}$, **Z**, **Y**, **n** and **X** are as defined in claim 1.

**4.** The compound of formula (II) according to claim 3 wherein

$\mathbf{R_7}$ and $\mathbf{R_6}$ and/or $\mathbf{R_6}$ and $\mathbf{R_5}$ and/or $\mathbf{R_5}$ and $\mathbf{R_4}$ form together an aromatic ring or heteroaromatic ring fused to the (hetero)cyclic core (Cycle C6) they are attached to, optionally substituted by **Z**
and $R_1$, $R_8$, $R_9$, $R_{10}$, $R_{11}$, Z, Y, n and X are as defined in claim 1.

**5.** The compound of formula (II) according to claim 3 wherein $\mathbf{R_8}$ and $\mathbf{R_9}$ or $\mathbf{R_{10}}$ and $\mathbf{R_{11}}$ form an aromatic ring fused to the pyridium group they are attached to
And $\mathbf{R_1}$, $\mathbf{R_4}$, $\mathbf{R_5}$, $\mathbf{R_6}$, $\mathbf{R_7}$, **Z**, **Y**, **n** and **X** are as defined in claim 1.

**6.** The compound of formula (I) according claim 1, wherein said compound is selected from:

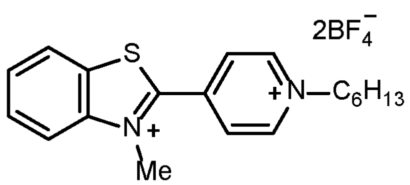

,

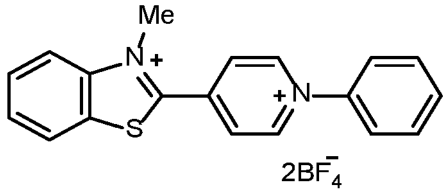

,

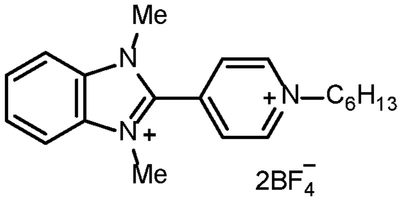

,

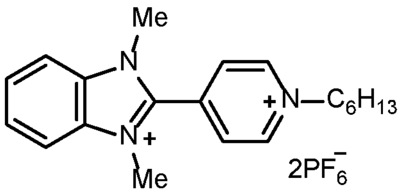

,

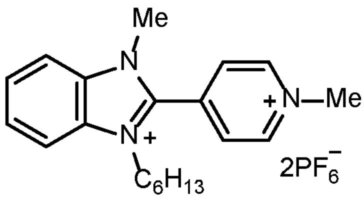

,

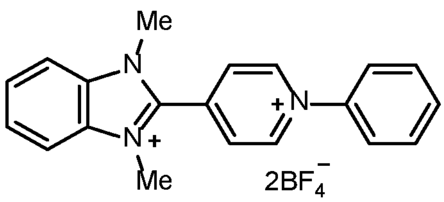

,

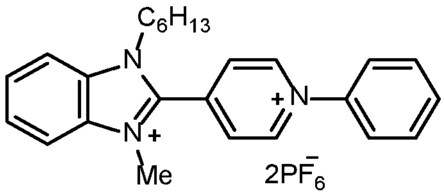

,

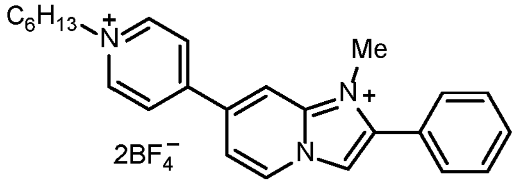

,

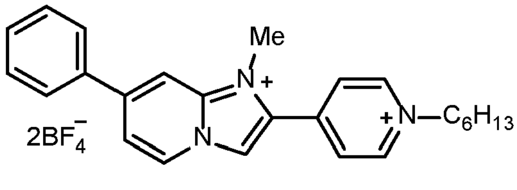

,

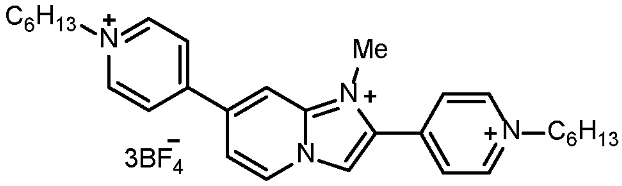

,

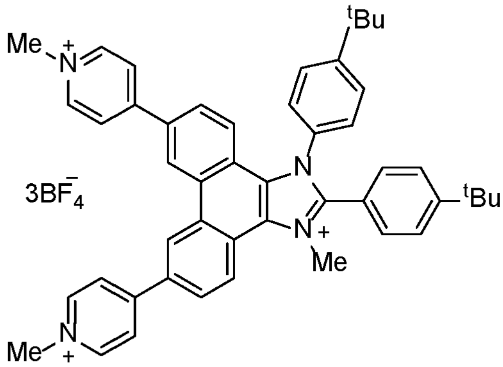

,

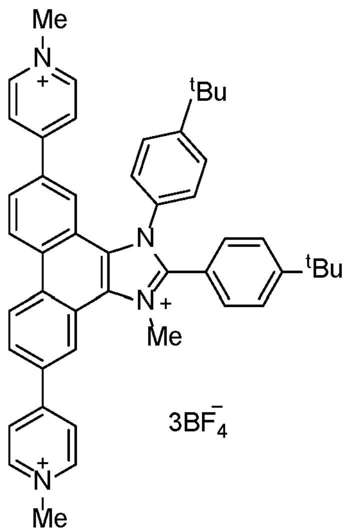

,

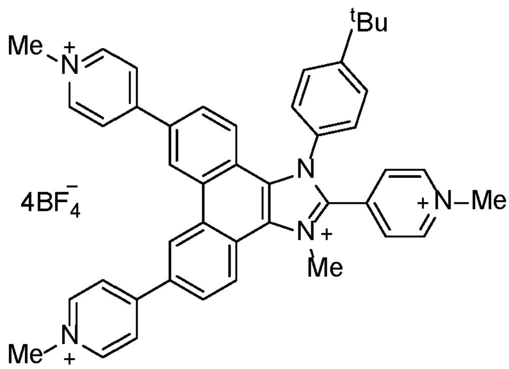

,

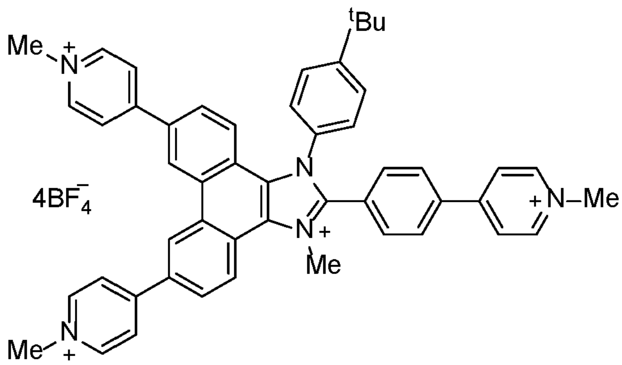

,

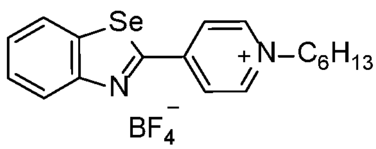

,

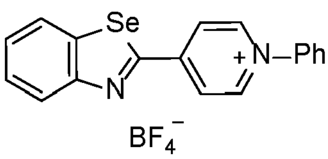

,

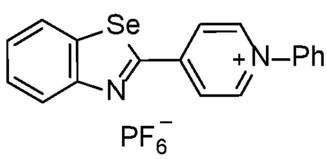

,

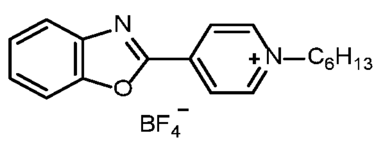

,

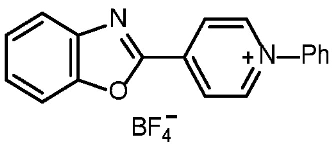

.,

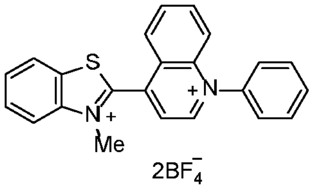

, ,

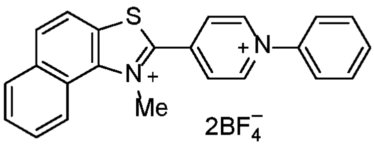

,

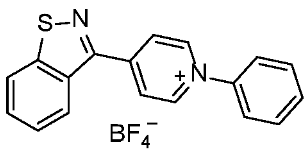

,

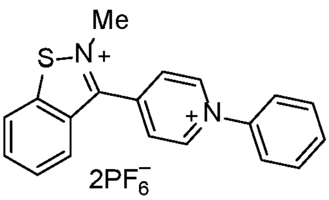

,

and

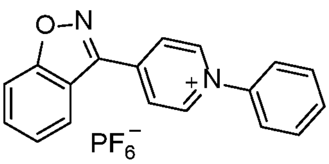

**7.** An electrochromic composition comprising at least one compound as defined in any one of claims 1 to 6.

**8.** The electrochromic composition according to claim 7, wherein said composition further comprises a host medium, preferably a host medium that is a fluid, a mesomorphous media or a gel.

**9.** An electrochromic device comprising a compound as defined in any one of claims 1 to 6, or an electrochromic composition as defined in claim 7 or 8.

**10.** The electrochromic device according to claim 9, wherein said electrochromic device is selected from an optical article, preferably an optical lens or an optical filter, a window, notably aircraft window, a visor, a mirror, a head mounted device and a display, more preferably an optical article selected from optical lenses, and most preferably an optical article selected from ophthalmic lenses.

**Patentansprüche**

1. Elektrochrome Verbindung, die durch Formel (I) wiedergegeben wird:

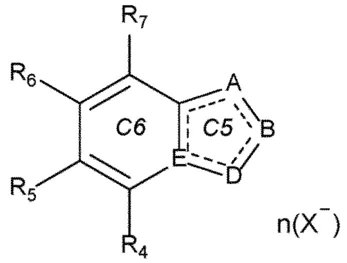

(I)

wobei:

**A** für N, $^+N$, N-$R_1$, $^+N$-$R_1$ oder C-$R_1$ steht;
**B** für C-$R_2$, S, Se, 0, N, N-$R_2$ oder $^+N$-$R_2$ steht;
**D** für C-$R_3$, N, S, 0, Se, N-$R_3$ oder $^+N$-$R_3$ steht;
**E** für C, N oder $^+N$ steht;
$\mathbf{R_1}$ für H, $C_1$-$C_{18}$-Alkyl, Aryl oder **Z** steht;
$\mathbf{R_2}$ für H, $C_1$-$C_{18}$-Alkyl, Aryl, **Z** oder Aryl, das durch **Z** substituiert ist, steht;
$\mathbf{R_3}$ für H oder $C_1$-$C_{18}$-Alkyl, Aryl oder **Z** steht;
$\mathbf{R_4}$ für H, $C_1$-$C_{18}$-Alkyl, Aryl oder **Z** steht;
$\mathbf{R_5}$ für H, $C_1$-$C_{18}$-Alkyl, Aryl oder **Z** steht;
$\mathbf{R_6}$ für H, $C_1$-$C_{18}$-Alkyl, Aryl oder **Z** steht;
$\mathbf{R_7}$ für H, $C_1$-$C_{18}$-Alkyl, Aryl oder **Z** steht;
$\mathbf{R_7}$ und $\mathbf{R_6}$ und/oder $\mathbf{R_6}$ und $\mathbf{R_5}$ und/oder $\mathbf{R_5}$ und $\mathbf{R_4}$ zusammen einen aromatischen oder heteroaromatischen Ring bilden können, der an den sechsgliedrigen (hetero)cyclischen Kern (Ring C6), an den sie gebunden sind, anelliert ist und gegebenenfalls durch **Z** substituiert ist,
wobei **Z** für

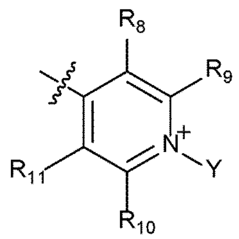

steht;
wobei **Y** für $C_1$-$C_6$-Alkyl oder Aryl steht;
$\mathbf{R_8}$, $\mathbf{R_9}$, $\mathbf{R_{10}}$ und $\mathbf{R_{11}}$ unabhängig aus H und $C_1$-$C_{18}$-Alkyl ausgewählt sind;
$\mathbf{R_8}$ und $\mathbf{R_9}$ oder $\mathbf{R_{10}}$ und $\mathbf{R_{11}}$ einen aromatischen Ring bilden können, der an die Pyridiniumgruppe, an die sie gebunden sind, anelliert ist,
dann, wenn **B** = C-Z und wenn **A**=$^+$N, $\mathbf{R_8}$ oder $\mathbf{R_{11}}$ mit **A** einen aromatischen oder nichtaromatischen Ring bilden können, der mit dem fünfgliedrigen heterocyclischen Ring (Ring C5), an den **A** gebunden ist, anelliert ist,
**n** so gewählt ist, dass die Zahl positiver Ladungen ausgeglichen wird;
**X** für ein Gegenion steht;
<u>- - - - - -</u> für eine Einfachbindung oder eine Doppelbindung steht;
**mit den 3 folgenden Maßgaben:**

1) Ring C5 ist ein fünfgliedriger heterocyclischer Ring, wobei 2 von **A, B, D** und **E** unabhängig aus N, N-$R_1$, $^+N$-$R_1$, N-$R_2$, $^+N$-$R_2$, N-$R_3$, $^+N$-$R_3$, S, Se und 0 ausgewählt sind;
2) Ring C5 und Ring C6 bilden ein konjugiertes System; und
3) mindestens eines von $\mathbf{R_1}$, $\mathbf{R_2}$, $\mathbf{R_3}$, $\mathbf{R_4}$, $\mathbf{R_8}$, $\mathbf{R_6}$ oder $\mathbf{R_7}$ steht für **Z** oder mindestens $\mathbf{R_7}$ und $\mathbf{R_6}$ bilden zusammen einen aromatischen Ring, der durch **Z** substituiert ist, oder mindestens $\mathbf{R_5}$ und $\mathbf{R_6}$ bilden zu-

sammen einen aromatischen Ring, der durch **Z** substituiert ist, oder mindestens $\mathbf{R_5}$ und $\mathbf{R_4}$ bilden zusammen einen aromatischen Ring, der durch **Z** substituiert ist;

und wobei die Verbindung der Formel (I) aus denjenigen ausgewählt ist, in denen:

- **A** für $^+N-R_1$ steht; **B** für $C-R_2$ steht; **D** für S oder $N-R_3$ steht; **E** für C steht; oder
- **A** für $^+N-R_1$ steht; **B** für $C-R_2$ steht; **D** für $C-R_3$ steht; **E** für N steht; oder
- **A** für N steht; **B** für $C-R_2$ steht; **D** für Se oder 0 steht; **E** für C steht; oder
- **A** für $C-R_1$ steht; **B** für N oder $^+N-R_2$ steht; **D** für S steht; **E** für C steht; oder
- **A** für $C-R_1$ steht; **B** für N steht; **D** für 0 steht; **E** für C steht; oder
- **A** für $C-R_1$ steht; **D** für N, $^+N-R_3$, S, Se oder 0 steht; **E** für C steht; oder
- **B** für N oder $^+N-R_2$ steht; **A** für $C-R_1$ steht; **D** für $C-R_3$ steht; **E** für N steht; oder
- **D** für $^+N-R_3$ oder N steht; **A** für $C-R_1$ steht; **B** für $C-R_2$ steht; **E** für N steht; oder
- **E** für $^+N$ steht; **A** für 0, S oder Se steht; **B** für $C-R_2$ steht; **D** für $C-R_3$ steht;

und wobei es sich bei der elektrochromen Verbindung der Formel (I) **nicht** um eine der folgenden Verbindungen handelt:

eine Verbindung der Formel $D_1$:

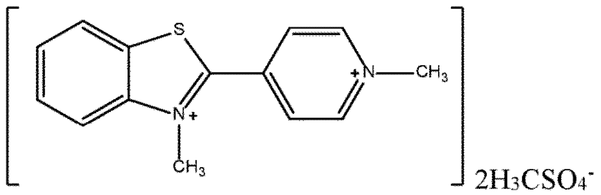

($D_1$)

oder eine Verbindung der Formel D2:

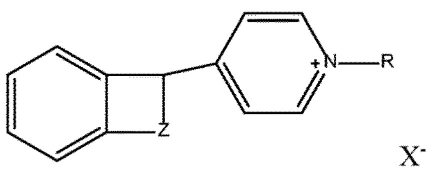

($D_2$)

wobei R für Niederalkyl mit einem bis vier Kohlenstoffatomen steht, das aus der Gruppe bestehend aus Methyl, Ethyl, Propyl, Isopropyl, Butyl und Isobutyl ausgewählt ist; Z für einen dreiwertigen Rest steht, der aus der Gruppe bestehend aus

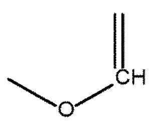 und 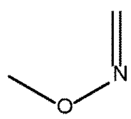

ausgewählt ist;
und $X^-$ für ein einwertiges pharmazeutisch unbedenkliches Anion steht, das aus der Gruppe bestehend aus Chlorid, Bromid, Iodid und Fluorid ausgewählt ist;

oder eine Verbindung aus der Gruppe bestehend aus:

1-Methyl-4-(1,2-benzisoxazol-3-yl)pyridiniumiodid, 1-Ethyl-4-(1,2-benzisoxazol-3-yl)pyridinium-chlorid,
1-Methyl-4-(3-benzofuranyl)pyridiniumiodid,

1-Methyl-4-(3-benzofuranyl)pyridiniumchlorid und
1-Butyl-4-(3-benzofuranyl)pyridiniumbromid.

2. Verbindung der Formel (I) nach Anspruch 1, wobei die Verbindung durch Formel (II), (III), (IV), (V), (VI), (VII), (VIII), (IX) oder (X) wiedergegeben wird:

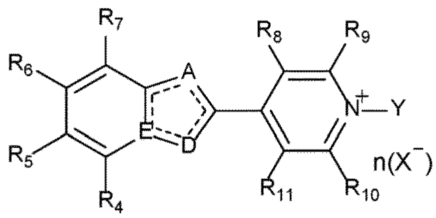

(II)

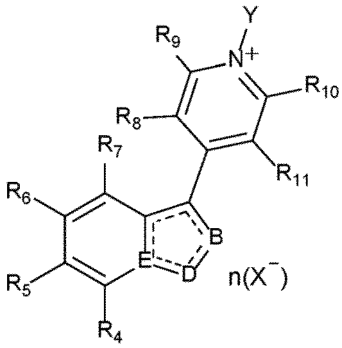

(III)

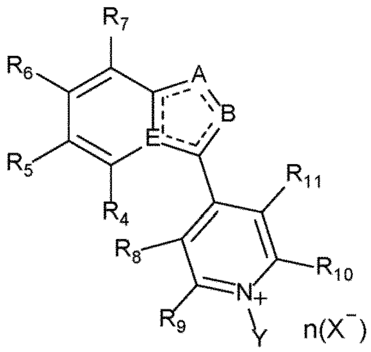

(IV)

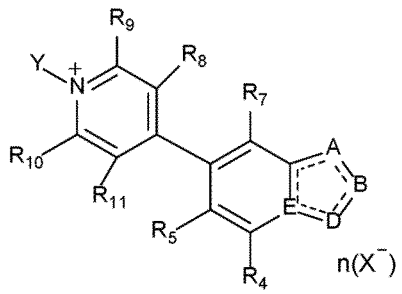

(V)

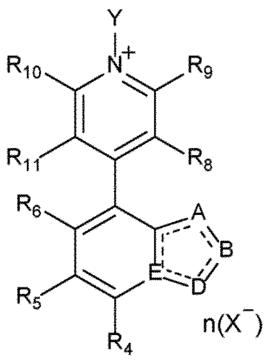

(VI)

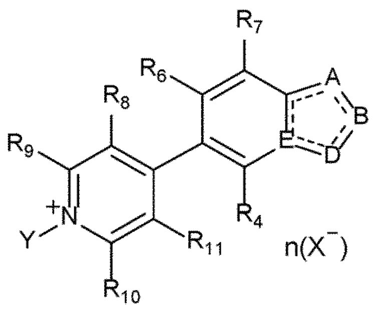

(VII)

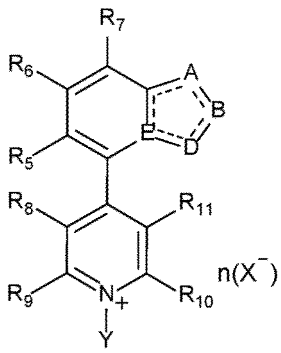

(VIII)

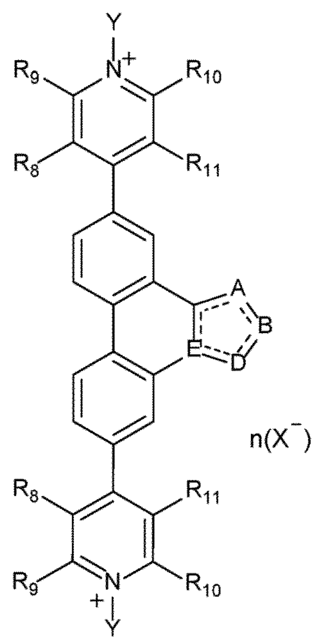

(IX)

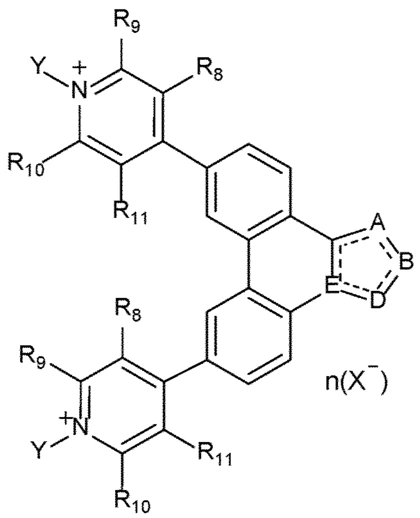

(X)

wobei **A, B, D, E, $R_1$, $R_2$, $R_3$, $R_4$, $R_5$, $R_6$, $R_7$, $R_8$, $R_9$, $R_{10}$** und **$R_{11}$,** sofern vorhanden, wie in Anspruch 1 definiert sind und wobei **Z, Y, n** und **X** ebenfalls wie in Anspruch 1 definiert sind.

**3.** Verbindung der Formel (II) nach Anspruch 1, wobei

**A** für $^+N$-$R_1$ steht;
**D** für S steht;
**E** für C steht
und **$R_1$, $R_4$, $R_8$, $R_6$, $R_7$, $R_8$, $R_9$, $R_{10}$, $R_{11}$, Z, Y, n** und **X** wie in Anspruch 1 definiert sind.

**4.** Verbindung der Formel (II) nach Anspruch 3, wobei

$\mathbf{R_7}$ und $\mathbf{R_6}$ und/oder $\mathbf{R_6}$ und $\mathbf{R_5}$ und/oder $\mathbf{R_5}$ und $\mathbf{R_4}$ zusammen einen aromatischen oder heteroaromatischen Ring bilden, der an den (hetero)cyclischen Kern (Ring C6), an den sie gebunden sind, anelliert ist und gegebenenfalls durch **Z** substituiert ist,
und $R_1$, $R_8$, $R_9$, $R_{10}$, $R_{11}$, Z, Y, n und X wie in Anspruch 1 definiert sind.

**5.** Verbindung der Formel (II) nach Anspruch 3, wobei

$\mathbf{R_8}$ und $\mathbf{R_9}$ oder $\mathbf{R_{10}}$ und $\mathbf{R_{11}}$ einen aromatischen Ring bilden, der an die Pyridiumgruppe, an die sie gebunden sind, anelliert ist,
und $\mathbf{R_1}$, $\mathbf{R_4}$, $\mathbf{R_8}$, $\mathbf{R_6}$, $\mathbf{R_7}$, **Z**, **Y**, **n** und **X** wie in Anspruch 1 definiert sind.

**6.** Verbindung der Formel (I) nach Anspruch 1, wobei die Verbindung ausgewählt ist aus:

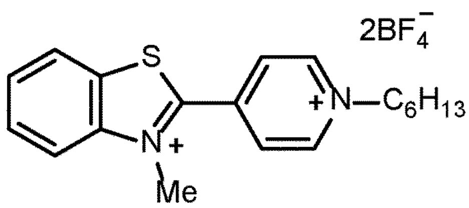

,

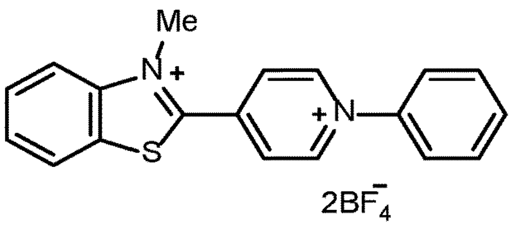

,

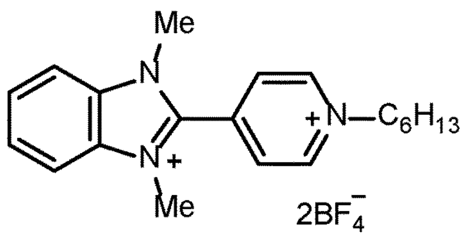

,

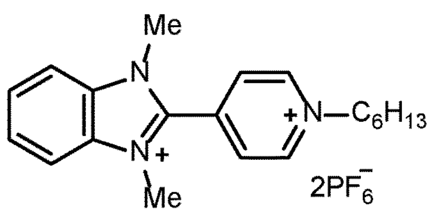

,

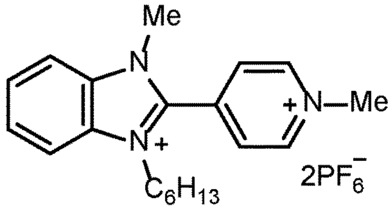

,

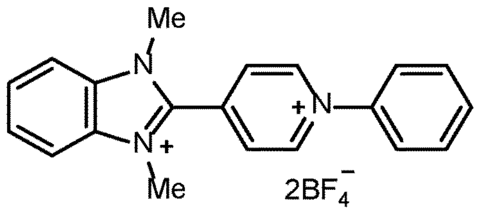

,

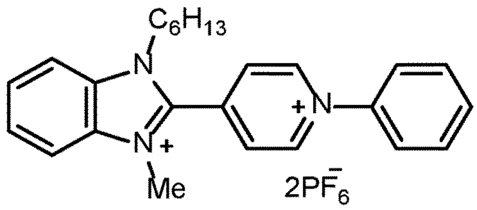

,

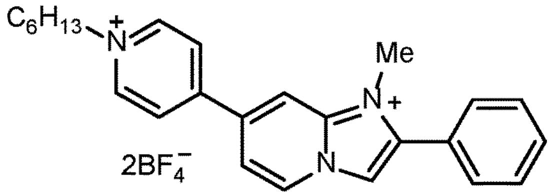

,

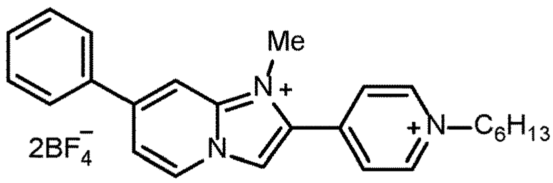

,

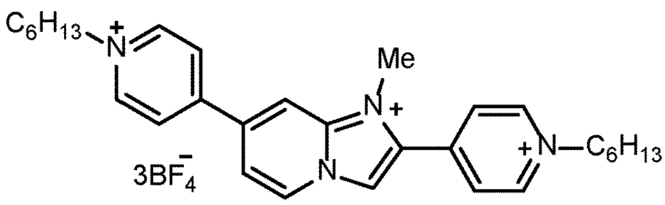

,

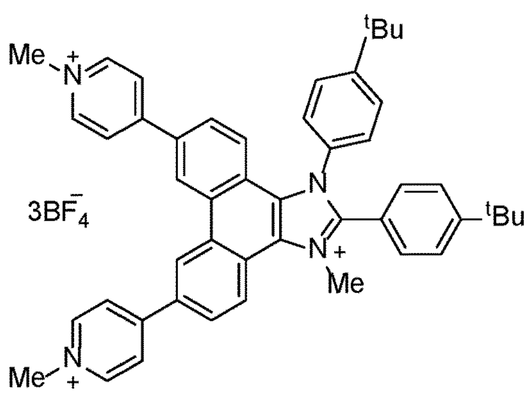

,

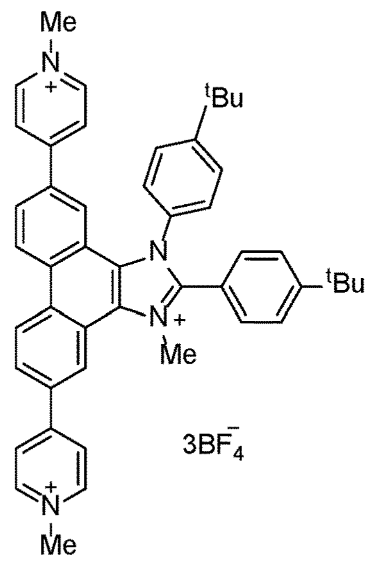

,

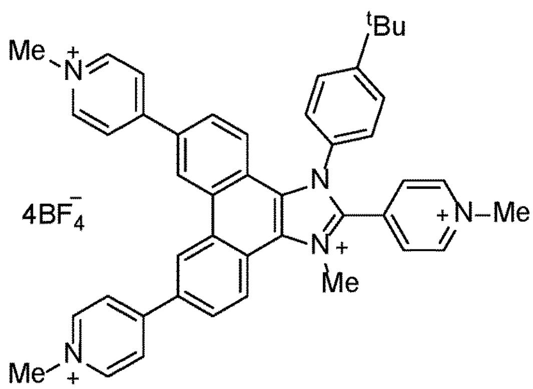

,

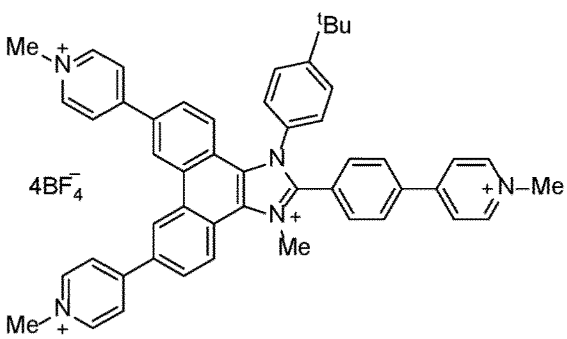

,

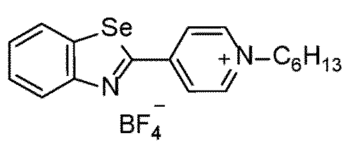

,

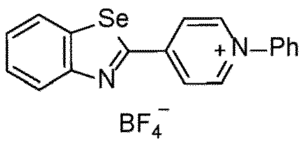

,

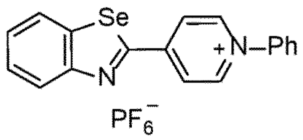

,

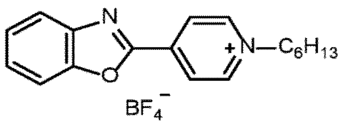

,

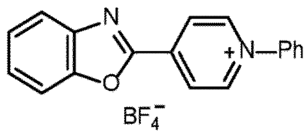

.,

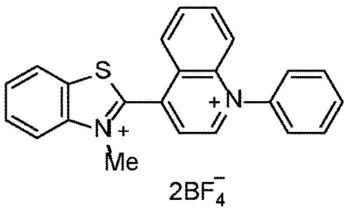

, ,

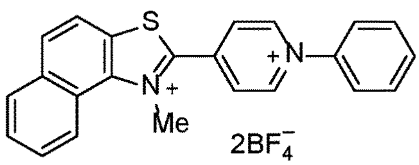

,

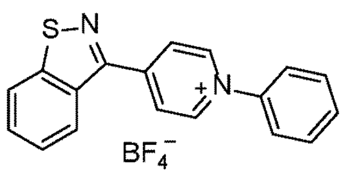

,

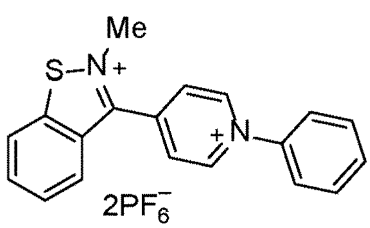

und

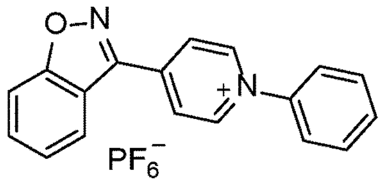

.

7. Elektrochrome Zusammensetzung, umfassend mindestens eine Verbindung gemäß einem der Ansprüche 1 bis 6.

8. Elektrochrome Zusammensetzung nach Anspruch 7, wobei die Zusammensetzung ferner ein Wirtsmedium umfasst, vorzugsweise ein Wirtsmedium, das ein Fluid, ein mesomorphes Medium oder ein Gel ist.

9. Elektrochrome Vorrichtung, umfassend eine Verbindung gemäß einem der Ansprüche 1 bis 6 oder eine elektrochrome Zusammensetzung gemäß Anspruch 7 oder 8.

10. Elektrochrome Vorrichtung nach Anspruch 9, wobei die elektrochrome Vorrichtung ausgewählt ist aus einem optischen Gegenstand, vorzugsweise einer optischen Linse oder einem optischen Filter, einem Fenster, insbesondere einem Luftfahrzeugfenster, einem Blendschutz, einem Spiegel, einer am Kopf befestigten Vorrichtung und einer Anzeige, weiter bevorzugt einem optischen Gegenstand, der aus optischen Linsen ausgewählt ist, und ganz besonders bevorzugt einem optischen Gegenstand, der aus ophthalmischen Linsen ausgewählt ist.

**Revendications**

1. Composé électrochromique représenté par la formule (I) :

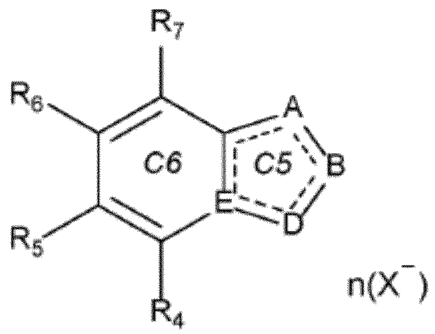

(I)

dans lequel

A est N, $^{+}N$, $N-R_1$, $^{+}N-R_2$ ou $C-R_1$ ;
B est $C-R_2$, S, Se, 0, N, $N-R_2$ ou $^{+}N-R_2$ ;
D est $C-R_3$, N, S, 0, Se, $N-R_3$ ou $^{+}N-R_3$ ;
E est C, N ou $^{+}N$ ;
$R_1$ est H, $C_1$-$C_{18}$ alkyle, aryle ou Z ;
$R_2$ est H, $C_1$-$C_{18}$ alkyle, aryle, Z ou aryle substitué par Z ;
$R_3$ est H ou $C_1$-$C_{18}$ alkyle, aryle ou Z ;
$R_4$ est H, $C_1$-$C_{18}$ alkyle, aryle ou Z ;
$R_5$ est H, $C_1$-$C_{18}$ alkyle, aryle ou Z ;
$R_6$ est H, $C_1$-$C_{18}$ alkyle, aryle ou Z ;
$R_7$ est H, $C_1$-$C_{18}$ alkyle, aryle ou Z ;
$R_7$ et $R_6$ et/ou $R_6$ et $R_5$ et/ou $R_5$ et $R_4$ peuvent former ensemble un cycle aromatique ou un cycle hétéroaromatique condensé au noyau (hétéro)cyclique à 6 chaînons (Cycle C6) auquel ils sont fixés, éventuellement substitué par Z,
dans lequel Z est

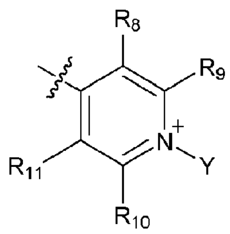

;

Y est $C_1$-$C_6$ alkyle ou aryle ;
$R_8$, $R_9$, $R_{10}$ et $R_{11}$ sont indépendamment choisis parmi H et $C_1$-$C_{18}$ alkyle ;
$R_8$ et $R_9$ ou $R_{10}$ et peuvent former un cycle aromatique condensé au groupe pyridinium auquel ils sont fixés, lorsque B = C-Z, et lorsque A=$^+$N, $R_8$ ou $R_{11}$ peut former un cycle avec A, aromatique ou non, condensé au cycle hétérocyclique à cinq chaînons (Cycle C5) auquel A est fixé,
n est choisi pour contrebalancer le nombre de charges positives ;
X est un contre-ion ;
- - - - - - est une simple liaison ou une double liaison ;
avec les 3 conditions suivantes :

1) Cycle C5 est un cycle hétérocyclique à cinq chaînons avec 2 parmi A, B, D et E qui sont indépendamment choisis parmi : N, N-$R_1$, $^+$N-$R_1$, N-$R_2$,$^+$N-$R_2$, N-$R_3$, $^+$N-$R_3$, S, Se et 0 ;
2) cycle C5 et Cycle C6 forment un système conjugué ; et
3) au moins l'un parmi $R_1$, $R_2$, $R_3$, $R_4$, $R_5$, $R_6$ ou $R_7$ est Z ou au moins $R_7$ et $R_6$ forment ensemble un cycle aromatique substitué par Z ou au moins $R_5$ et $R_6$ forment ensemble un cycle aromatique substitué par Z ou au moins $R_5$ et $R_4$ forment ensemble un cycle aromatique substitué par Z ;

et dans lequel ledit composé de formule (I) est choisi parmi ceux dans lesquels :

- A est $^+$N-$R_2$ ; B est C-$R_2$ ; D est S ou N-$R_3$ ; E est C ; ou
- A est $^+$N-$R_1$ ; B est C-$R_2$ ; D est C-$R_3$ ; E est N ; ou
- A est N ; B est C-$R_2$ ; D est Se ou O ; E est C ; ou
- A est C-$R_1$ ; B est N ou $^+$N-$R_2$ ; D est S ; E est C ; ou
- A est C-$R_1$ ; B est N, D est O ; E est C ; ou
- A est C-$R_1$ ; D est N, $^+$N-$R_3$, S, Se ou O ; E est C ; ou
- B est N ou $^+$N-$R_2$ ; A est C-$R_2$ ; D est C-$R_3$ ; E est N ; ou
- D est $^+$N-$R_3$ ou N ; A est C-$R_2$ ; B est C-$R_2$ ; E est N ; ou

E est $^+$N ; A est O, S, ou Se ; B est C-$R_2$ ; D est C-$R_3$ ;
et dans lequel ledit composé électrochromique de formule (I) n'est pas l'un des composés suivants :

un composé de formule $D_1$ :

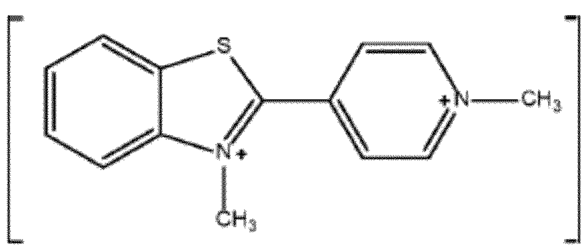

$2H_3CSO_4^-$

($D_1$)

ou un composé de formule D2 :

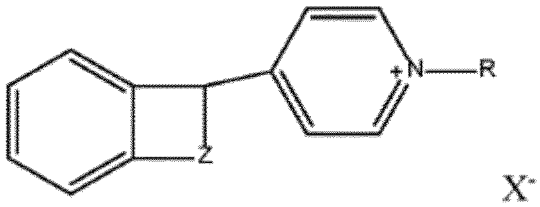

(D2)

dans lequel R est alkyle inférieur ayant un à quatre atomes de carbone choisi dans le groupe constitué par méthyle, éthyle, propyle, isopropyle, butyle et isobutyle ; Z est un radical trivalent choisi dans le groupe constitué par :

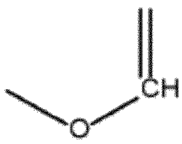

et

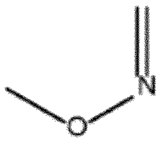

;

et $X^-$ est un anion pharmaceutiquement acceptable monovalent choisi dans le groupe constitué par chlorure, bromure, iodure et fluorure ;
ou un composé choisi dans le groupe constitué par :

iodure de 1-méthyl-4-(1,2-benzisoxazol-3-yl)pyridinium,
chlorure de 1-éthyl-4-(1,2-benzisoxazol-3-yl)pyridinium,
iodure de 1-méthyl-4-(3-benzofuranyl)pyridinium,
chlorure de 1-méthyl-4-(3-benzofuranyl)-pyridinium, et
bromure de 1-butyl-4-(3-benzofuranyl)pyridinium.

2. Composé de formule (I) selon la revendication 1, dans lequel ledit composé représenté par les formules (II), (III), (IV), (V), (VI), (VII), (VIII), (IX) ou (X) :

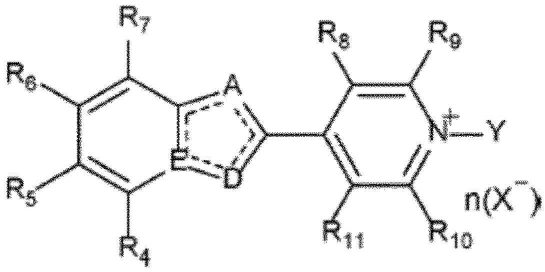

(II)

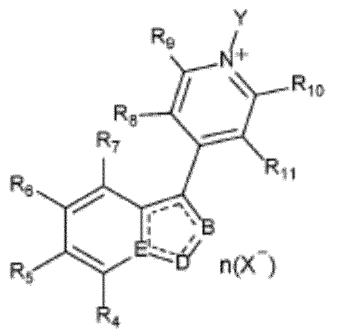

(III)

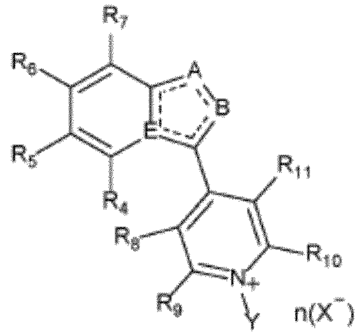

(IV)

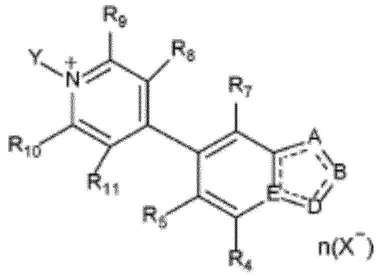

(V)

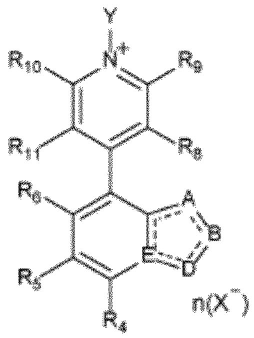

(VI)

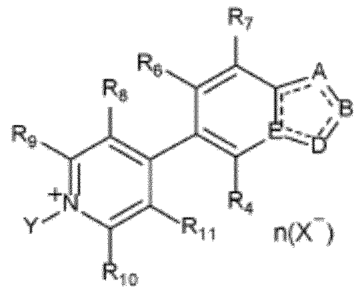

(VII)

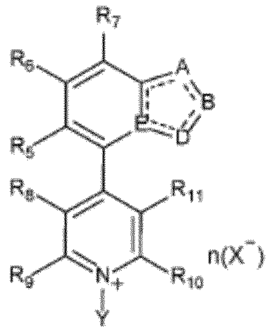

(VIII)

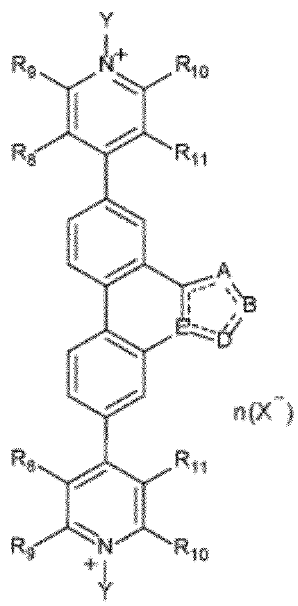

(IX)

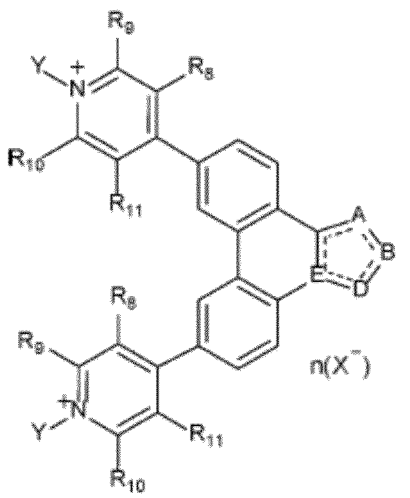

(X)

dans lequel A, B, D, E, $R_1$, $R_2$, $R_3$, $R_4$, $R_5$, $R_6$, $R_7$, $R_8$, $R_9$, $R_{10}$ et $R_{11}$, lorsqu'ils sont présents, sont tels que définis dans la revendication 1 ;
et dans lequel Z, Y, n et X sont également tels que définis dans la revendication 1.

**3.** Composé de formule (II) selon la revendication 1 dans lequel

A est $^+N-R_1$ ;
D est S ;
E est C ;
et $R_1$, $R_4$, $R_5$, $R_6$, $R_7$, $R_8$, $R_9$, $R_{10}$, $R_{11}$, Z, Y, n et X sont tels que définis dans la revendication 1.

**4.** Composé de formule (II) selon la revendication 3 dans lequel
$R_7$ et $R_6$ et/ou $R_6$ et $R_5$ et/ou $R_5$ et $R_4$ forment ensemble un cycle aromatique ou un cycle hétéroaromatique condensé au noyau (hétéro)cyclique (Cycle C6) auquel ils sont fixés, éventuellement substitué par Z et $R_1$, $R_8$, $R_9$, $R_{10}$, $R_{11}$, Z, Y, n et X sont tels que définis dans la revendication 1.

**5.** Composé de formule (II) selon la revendication 3 dans lequel

$R_8$ et $R_9$ ou $R_{10}$ et $R_{11}$ forment un cycle aromatique condensé au groupe pyridinium auquel ils sont fixés et $R_1$, $R_4$, $R_5$, $R_6$, $R_7$, Z, Y, n et X sont tels que définis dans la revendication 1.

**6.** Composé de formule (I) selon la revendication 1, dans laquelle ledit composé est choisi parmi :

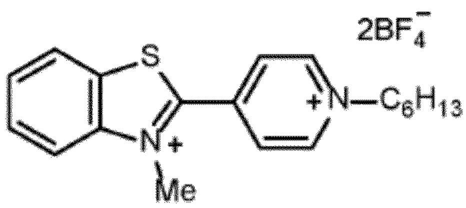

,

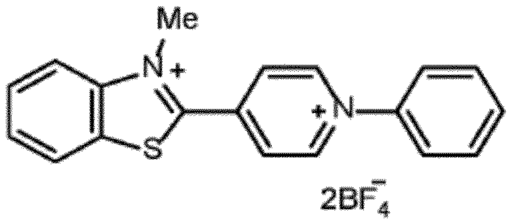

,

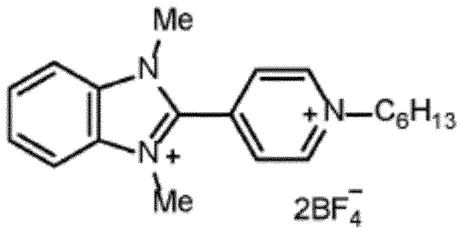

,

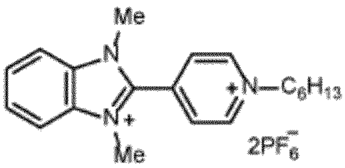

,

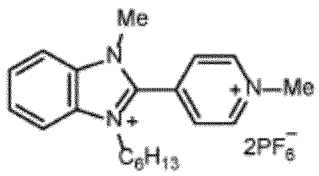

,

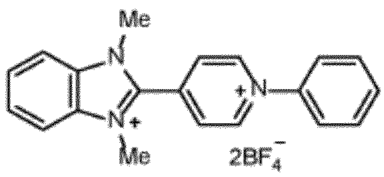

,

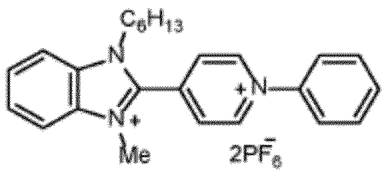

,

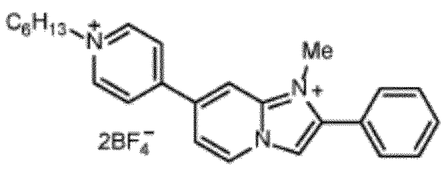

,

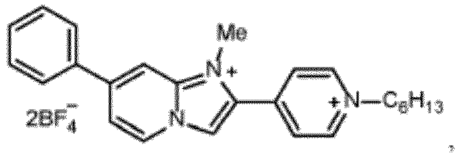

,

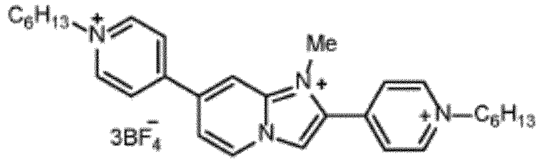

,

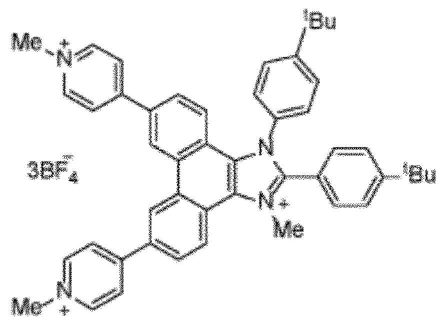

,

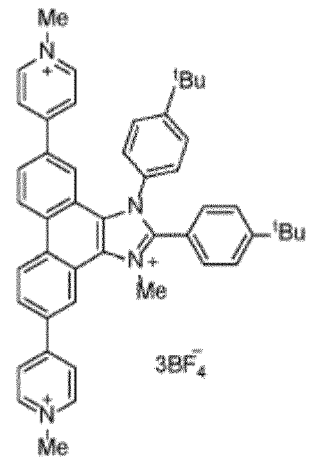

,

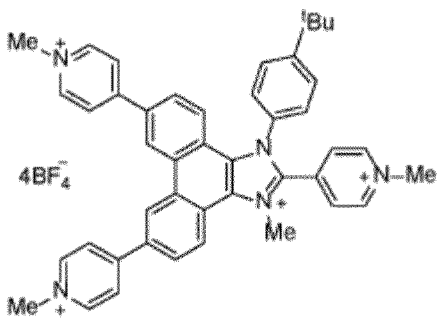

,

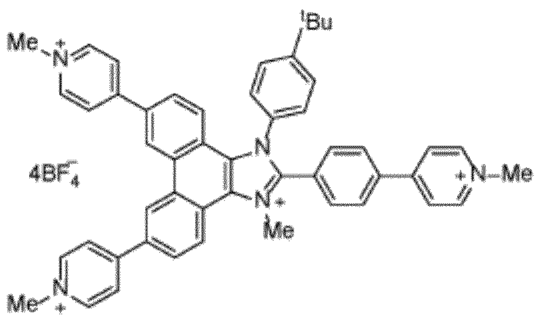

,

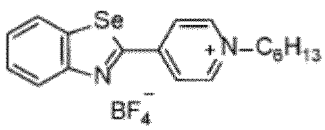

,

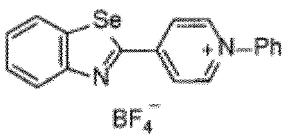

,

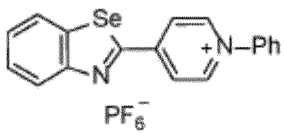

,

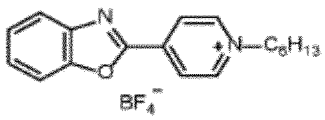

,

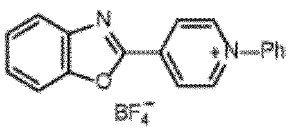

,

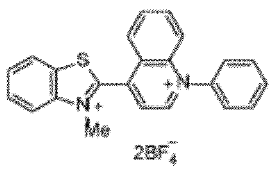

, ,

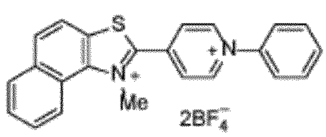

,

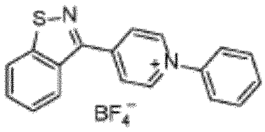

,

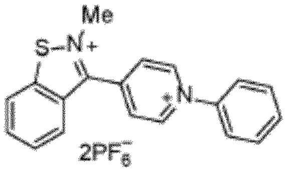

,

et

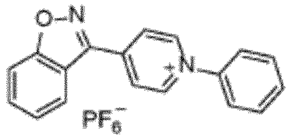

.

7. Composition électrochromique comprenant au moins un composé tel que défini dans l'une quelconque des revendications 1 à 6.

8. Composition électrochromique selon la revendication 7, dans laquelle ladite composition comprend en outre un milieu hôte, préférablement un milieu hôte qui est un fluide, un milieu mésomorphe ou un gel.

9. Dispositif électrochromique comprenant un composé tel que défini dans l'une quelconque des revendications 1 à 6, ou une composition électrochromique telle que définie dans la revendication 7 ou 8.

10. Dispositif électrochromique selon la revendication 9, ledit dispositif électrochromique étant choisi parmi un article optique, préférablement une lentille optique ou un filtre optique, une fenêtre, notamment une fenêtre d'aéronef, un viseur, un miroir, un dispositif monté sur la tête et un affichage, plus préférablement un article optique choisi parmi des lentilles optiques, et le plus préférablement un article optique choisi parmi des lentilles ophtalmiques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013245176 A
- US 3678062 A
- GB 1317442 A
- WO 2006101455 A, K. C. L. Lee and E. T. Sun,
- WO 2006013250 A
- FR 2937154
- FR 2950710


### Non-patent literature cited in the description


- **SALEHI NAEIMEH et al.** *Bioorganic Chemistry,* 2019, vol. 83, 559-568
- **YU et al.** *Journal of Photochemistry and Photobiology A: Chemistry,* 2006, vol. 178, 62-69
- **J. REVUELTA ; F. MACHETTI ; S. CICCHI.** Modern Heterocyclic Chemistry. Wiley-WCH, 2011, vol. 2, 809-923
- **P. E. MILLER ; G. L. OLIVER ; J. R. DANN ; J. W. GATES JR.** *J. Org. Chem.,* 1957, vol. 22, 664
- **Y. LIAO ; H. QI ; S. CHEN ; P. JIANG ; W. ZHOU ; G.-J. DENG.** *Org. Lett.,* 2012, vol. 14, 6004
- **K. HALMAN ; O. H. HANKOVSZKY.** *Acta Chim. Acad. Sci. Hung.,* 1965, vol. 43, 263
- **P. ZAVINS ; E. S. LAVINOVITCH ; A. ARENS.** *Khim. Geterotsikl. Soedin.,* 1973, vol. 104
- **G. N. DOROFEENKO ; A. V. KOBLIK ; B. A. TERTOV ; T. I. POLYAKOVA.** *Khim. Geterotsikl. Soedin.,* 1973, vol. 1016
- **M. BIELAWSKI ; B. OLOFSSON.** *Chem. Commun.,* 2007, vol. 2521
- **T. LV ; Z. WANG ; J. YOU ; J. LAN ; G. GAO.** *J. Org. Chem.,* 2013, vol. 73, 5723
- **C. REUS ; M. STOLAR ; J. VANDERKLEY ; J. NEUBAUER ; T. BAUMGARTNER.** *J. Am. Chem. Soc.,* 2015, vol. 137, 11710
- **W.-Z. WENG ; J.-S. GUO ; K.-X. LIU ; T.-Q. SHAO ; L.-Q. SONG ; Y.-P. ZHU ; Y.-Y. SUN ; Q.-G. MENG.** *Can. J. Chem.,* 2020, vol. 98, 179
- **S. HANEDA ; Z. GAN ; K. EDA ; M. HAYASHI.** *Organometallics,* 2007, vol. 26, 6551
- **H. L. BLEWITT.** Special Topics in Heterocyclic Chemistry. Wiley-Interscience, 1977, 117-178
- **F. COUTY ; G. EVANO.** Comprehensive Heterocyclic Chemistry III. Elsevier, 2008, vol. 11, 409-499
- **A. K. BAGDI ; S. SANTRA ; K. MONIR ; A. HAJRA.** *Chem. Commun.,* 2015, vol. 51, 1555
- **S. M. ROOPAN ; S. M. PATIL ; J. PALANIRAJA.** *Res. Chem. Intermed.,* 2016, vol. 42, 2740
- **M. P. HAY ; S. TURCETTE ; J. U. FLANNAGAN ; M. BONNET ; D. A. CHAN ; P. D. SUTPHIN ; P. NGUYEN ; A. J. GRACCIA ; W. A. DENNY.** *J. Med. Chem.,* 2010, vol. 53, 787
- **M. O. BANIKHALED ; J. D. MOTTISHAW ; H. SUN.** *Cryst. Growth Des.,* 2015, vol. 15, 2235
- **A. PATEL ; S. Y. SHARP ; K. HALL ; W. LEWIS ; M. F. G. STEVENS ; P. WORKMAN ; C. J. MOODY.** *Org. Biomol. Chem.,* 2016, vol. 14, 3889
- **T. H. VO ; M. SHEKHIREV ; D. A. KUNKEL ; F. ORANGE ; M. J.-F. GUINEL ; A. ENDERSBE ; A. SINITSKII.** *Chem. Commun.,* 2014, vol. 50, 4172
- **K. SKONIECZNY ; D. T. GRYKO.** *J. Org. Chem.,* 2015, vol. 80, 5753
- **M. M. HERAVIL ; M. DARAIEL ; V. ZADSIRJAN.** *Mol. Divers.,* 2015, vol. 19, 577
- **D. KUMAR ; K.R. J. THOMAS.** *J. Photochem. Photobiol. A: Chem,* 2011, vol. 218, 162
- **W-C. CHEN ; Y. YUAN ; Y. XIONG ; A. L. ROGACH ; Q-X. TONG ; C-S. LEE.** *ACS Appl. Mater. Interfaces,* 2017, vol. 9, 26268
- **M. JURICEK ; J. C. BARNES ; E. J. DALE ; W-G. LIU ; N. L. STRUTT ; C. J. BRUNS ; N. A. VERMEULEN ; K. C. GHOORAY ; A. A. SARJEANT ; C. L. STERN.** *J. Am. Chem. Soc.,* 2013, vol. 135, 12736
- **Y. NAKAMURA ; N. ARATANI ; A. OSUKA.** *Chem. Asian J.,* 2007, vol. 2, 860
- **V. GRAY ; K. BÖRJESSON ; D. DZEBO ; M. ABRAHAMSSON ; B. ALBINSSON ; K. MOTH-POULSEN.** *J. Phys. Chem. C,* 2016, vol. 120, 19018
- **J. F. S. CARVALHO ; J. LOUVEL ; M. L. J. DOORNBOS ; E. KLAASE ; Z. YU ; J. BRUSSEE ; A. P. IJZERMAN.** *J. Med. Chem.,* 2013, vol. 56, 2828
- **A. N. WODWARD ; J. M. KOLESAR ; S. R. HALL ; N-A. SALEH ; D. S. JONES ; M. G. WALTER.** *J. Am. Chem. Soc.,* 2017, vol. 139, 8467

- **L. PESCATORI ; A. ARDUINI ; A. POCHINI ; A. SECCHI ; C. MASSERA ; F. UGOZZOLI.** *Org. Biomol. Chem.,* 2009, vol. 7, 3698
- **M. KUROBOSHI ; T. YAMAMOTO ; H. TANAKA.** *Synlett,* 2013, vol. 24, 0197
- **J. A. ZOLTEWICZ ; M. P. CRUSKIE, JR.** *Tetrahedron,* 1995, vol. 51, 3103
- **T. SU ; S. XIE ; B. LI ; J. YAN ; L. HUANG ; X. LI.** *Synlett,* 2015, vol. 26, 215
- **Z. XU ; H. HUANG ; H. CHEN ; G.-J. DENG.** *Org. Chem. Front.,* 2019, vol. 6, 3060
- **R. ZHU ; Z. LIU ; J. CHEN ; X. XIONG ; Y. WANG ; L. HUANG ; J. BAI ; Y. DENG ; J. HUANG.** *Org. Lett.,* 2018, vol. 20, 3161
- Isoxazoles. **F. GUALTIERI ; M. GIANNELLA.** Chemistry of Heterocyclic Compounds. John Wiley & Sons Inc, 1999, vol. 48, 1
- **H. HEPBURN ; T. J. DONOHOE.** *Chem. Eur. J.,* 2020, vol. 26, 1963